# EUROPÄISCHE PATENTSCHRIFT

(11) **EP 1 409 697 B1**
(45) Veröffentlichungstag und Bekanntmachung des Hinweises auf die Patenterteilung: **30.04.2008**
(21) Anmeldenummer: 02784843.1
(22) Anmeldetag: 05.07.2002
(51) Int. Cl.: C12N 15/82, A01H 5/00

(54) **EXPRESSIONSKASSETTEN ZUR TRANSGENEN EXPRESSION VON NUKLEINSÄUREN**
EXPRESSION CASSETTES FOR THE TRANSGENIC EXPRESSION OF NUCLEIC ACIDS
CASSETTES D'EXPRESSION POUR L'EXPRESSION TRANSGENIQUE D'ACIDES NUCLEIQUES

(30) Priorität: 13.07.2001 DE 10133407; 04.12.2001 DE 10159455; 22.02.2002 DE 10207582
(43) Veröffentlichungstag der Anmeldung: 21.04.2004
(73) Patentinhaber: Sungene GmbH & Co. KGaA, 06466 Gatersleben (DE)
(72) Erfinder: HEIM, Ute, 06466 Gatersleben (DE); HILLEBRAND, Helke, 68159 Mannheim (DE); KUNZE, Irene, 06466 Gatersleben (DE); HERBERS, Karin, 06484 Quedlinburg (DE); SONNEWALD, Uwe, 06484 Quedlinburg (DE); GLICKMANN, Eric, 06484 Ditfurt (DE); LEIN, Wolfgang, 14471 Potsdam (DE); HELL, Rüdiger, 68159 Mannheim (DE); JOST, Ricarda, 69759 Arnsberg (DE)
(74) Vertreter: Bieberbach, Andreas
(86) Internationale Anmeldenummer: PCT/EP2002/007527
(87) Internationale Veröffentlichungsnummer: WO 2003/006660

(56) Entgegenhaltungen:
- WO-A-99/31258
- US-B1- 6 229 067
- DATABASE EMBL [Online] 4. Juli 1997 (1997-07-04) retrieved from EMBL Database accession no. Z97337 XP002214553
- DATABASE EMBL [Online] 10. März 1998 (1998-03-10) retrieved from EMBL Database accession no. AB011474 XP002214554
- DATABASE EMBL [Online] 2. September 1997 (1997-09-02) retrieved from EMBL Database accession no. AB006698 XP002214555
- ELLIOTT R C ET AL: "CIS-ACTING ELEMENTS FOR LIGHT REGULATION OF PEA FERREDOXIN I GENE EXPRESSION ARE LOCATED WITHIN TRANSCRIBED SEQUENCES" PLANT CELL, Bd. 1, Nr. 7, 1989, Seiten 691-698, XP002214681 ISSN: 1040-4651
- LUEBBERSTEDT THOMAS ET AL: "Promoters from genes for plastid proteins possess regions with different sensitivities toward red and blue light." PLANT PHYSIOLOGY (ROCKVILLE), Bd. 104, Nr. 3, 1994, Seiten 997-1006, XP002214550 ISSN: 0032-0889
- PETRACEK MARIE E ET AL: "Light-regulated changes in abundance and polyribosome association of ferredoxin mRNA are dependent on photosynthesis." PLANT CELL, Bd. 9, Nr. 12, Dezember 1997 (1997-12), Seiten 2291-2300, XP002214551 ISSN: 1040-4651
- GALLO-MEAGHER M ET AL: "THE PEA FERREDOXIN I GENE EXHIBITS DIFFERENT LIGHT RESPONSES IN PEA AND TOBACCO" PLANT CELL, Bd. 4, Nr. 4, 1992, Seiten 383-388, XP002214552 ISSN: 1040-4651
- HOLTORF S ET AL: "COMPARISON OF DIFFERENT CONSTITUTIVE AND INDUCIBLE PROMOTERS FOR THE OVEREXPRESSION OF TRANSGENES IN ARABIDOPSIS THALIANA" PLANT MOLECULAR BIOLOGY, NIJHOFF PUBLISHERS, DORDRECHT, NL, Bd. 29, 1. November 1995 (1995-11-01), Seiten 637-646, XP002036874 ISSN: 0167-4412

## Beschreibung

Die Erfindung betrifft Expressionskassetten und Vektoren, die pflanzliche konstitutive Promotoren enthalten, sowie die Verwendung dieser Expressionskassetten oder Vektoren zur transgenen Expression von Nukleinsäuresequenzen - bevorzugt Selektionsmarkern - in Organismen, bevorzugt in Pflanzen. Die Erfindung betrifft ferner mit diesen Expressionskassetten oder Vektoren transformierte transgene Pflanzen, davon abgeleitete Kulturen, Teile oder Vermehrungsgut, sowie die Verwendung derselben zur Herstellung von Nahrungs-, Futtermitteln, Saatgut, Pharmazeutika oder Feinchemikalien.

Ziel biotechnologischer Arbeiten an Pflanzen ist die Herstellung von Pflanzen mit verbesserten Eigenschaften zum Beispiel zur Steigerung der landwirtschaftlichen Produktivität. Die Herstellung transgener Pflanzen ist eine grundlegende Technik der Pflanzenbiotechnologie und damit eine unerlässliche Voraussetzung für die pflanzliche Grundlagenforschung, sowie für die Herstellung von Pflanzen mit verbesserten, neuen Eigenschaften für die Landwirtschaft, zur Qualitätssteigerung bei Nahrungsmitteln oder zur Produktion bestimmter Chemikalien oder Pharmazeutika (Dunwell JM, J Exp Bot. 2000;51 Spec No:487-96). Oft sind die natürlichen Abwehrmechanismen der Pflanze zum Beispiel gegen Pathogene unzureichend. Die Einführung fremder Gene aus Pflanzen, Tieren oder mikrobiellen Quellen kann die Abwehr verstärken. Beispiele sind der Schutz gegen Insektenfraßss in Tabak durch Expression des *Bacillus thuringiensis* Endotoxin unter Kontrolle des 35 S CaMV Promotors (Vaeck et al. (1987) Nature 328:33-37) oder der Schutz des Tabaks gegen Pilzbefall durch Expression einer Chitinase aus der Bohne unter Kontrolle des CaMV Promotors (Broglie et al. (1991) Science 254:1194-1197). Ferner kann durch die Einführung fremder Gene eine Herbizidresistenz erzielt werden, was die Anbaubedingungen optimiert und Ernteverluste verringert (Ott KH et al. (1996) J Mol Biol 263(2):359-368). Auch kann die Qualität der Erzeugnisse verbessert werden. So kann die Haltbarkeit und Lagerfähigkeit von Ernteerzeugnissen zum Beispiel durch Inaktivierung bestimmter Reifungsgene erhöht werden. Gezeigt wurde dies zum Beispiel an der Tomate durch Inaktivierung der Polygalacturonase (Hamilton AJ et al.(1995) Curr Top Microbiol Immunol 197:77-89).

Eine Grundvoraussetzung für die transgene Expression bestimmter Gene in Pflanzen ist die Bereitstellung pflanzenspezifischer Promotoren. Verschiedene pflanzliche Promotoren sind bekannt. Dabei kann zwischen konstitutiven Promotoren, die eine lokal und zeitlich nur wenig beschränkte Expression in verschiedenen Teilen einer Pflanze ermöglichen, und spezifischen Promotoren, die eine Expression nur in bestimmten Teilen oder Zellen einer Pflanze (z.B. Wurzel, Samen, Pollen, Blättern etc,) oder nur zu bestimmten Zeitpunkten der Entwicklung gestatten, unterschieden werden. Konstitutive Promotoren werden beispielsweise für die Expression sogenannter Selektionsmarker verwendet. Selektionsmarker (z.Bb. Antibiotika- oder Herbizidresistenzgene) erlauben es, das Transformationsereignis aus der Vielzahl der nicht transformierten, aber ansonsten identischen pflanzlichen Individuen herauszufiltern.

Konstitutive in Pflanzen aktive Promotoren sind bislang relativ selten geschrieben. Zu nennen sind der TR-Doppelpromotor aus Agrobacterium tumefaciens, die Promotoren der vakuolären ATPase Untereinheiten oder der Promotor eines prolinreichen Proteins aus Weizen (WO 91/13991) sowie der Ppc1 Promotor aus Mesembryanthemum cryctallinum (Cushman et al. (1993) Plant Mol Biol 21:561-566).

Die derzeit in Pflanzen überwiegend verwendeten konstitutiven Promotoren sind fast ausschließlich virale oder aus Agrobacterium isolierte Promotoren. Dabei handelt es sich im einzelnen um den Nopalinsynthase (nos) Promotor (Shaw et al. (1984) Nucleic Acids Res. 12(20):7831-7846), den Mannopinsynthase (*mas*) promotor (Comai et al. (1990) Plant Mol Biol 15 (3):373-381) und den Octopinsynthase (*ocs*) Promotor (Leisner and Gelvin (1988) Proc Natl Acad Sci USA 85(5):2553-2557) aus *Agrobacterium tumefaciens* oder der CaMV35S Promotor aus dem Blumenkohl-Mosaikvirus. Letzterer ist der am häufigsten verwendete Promotor in Expressionssystemen mit ubiquitärer und andauernder Expression (Odell et al.(1985) Nature 313:810-812; Battraw and Hall (1990) Plant Mol Biol 15:527-538; Benfey et al. (1990) EMBO J 9(69):1677-1684; US 5,622,472). Der häufig als konstitutiver Promoter angewandte CaMV 35S Promotor zeigt jedoch Variationen in der Aktivität in unterschiedlichen Pflanzen und in unterschiedlichen Geweben derselben Pflanze (Atanassova et al. (1998) Plant Mol Biol 37:275-85; Battraw and Hall (1990) Plant Mol Biol 15:527-538; Holtorf et al. (1995) Plant Mol Biol 29:637-646; Jefferson et al. (1987) EMBO J 6:3901-3907), Ein weiterer Nachteil des 35S Promotors ist, das bei einer Infektion mit dem Blumenkohlmosaikvirus und seinen typischen pathogenen Varianten die Expression des Transgens verändert wird. So sind Pflanzen, die das BAR Gen unter der Kontrolle des 35S Promotors exprimieren nicht mehr resistent nach Infektion mit dem Virus, der in der Natur typischerweise vorkommt (Al-Kaff et al. (2000) Natur Biotechnology 18:995-99).

Als Alternative zum CaMV 35S Promotor aus dem Bereich viraler Promotoren wurde der Sugarcane bacilliform badnavirus (ScBV) beschrieben (Schenk et al. (1999) Plant Mol Biol 39(6):1221-1230), der ein dem CamV ähnliches Expressionsmuster vermittelt. Die Aktivität des ScBV Promotors wurde in transienten Expressionsanalysen mit verschiedenen zweikeimblättrigen Pflanzen, darunter *Nicotiana tabacum* und *N. benthamiana,* Sonnenblume und Raps, und einkeimblättrigen Pflanzen, hier anhand von Banane, Mais und Hirse, analysiert. In den transienten Analysen in Mais war das ScBV Promotor vermittelte Expressionsniveau vergleichbar mit dem des Ubiquitin-Promotors aus Mais (s. unten). Weiterhin wurde die ScBV Promotor-vermittelte Expressionsrate in transgenen Bananen- und Tabakpflanzen getestet und zeigte in beiden Pflanzenspezies im wesentlichen konstitutive Expression.

Als gängige Promotoren zur Expression von Selektionsmarkern in Pflanzen werden vor allem der *nos*-Promotor, aber auch der *mas-*und oos-Promotor verwendet, die allesamt aus *Agrobacterium-*Stämmen isoliert wurden.

Der Verwendung viraler Sequenzen wird seitens des Verbrauchers oft mit großenm Vorbehalt begegnet. Diese Bedenken werden nicht zuletzt durch Untersuchungen genährt, die die Sicherheit des 35S CaMV Promotors - aufgrund eines möglichen horizontalen Gentransfers bedingt durch einen Rekombinations-"Hot Spot" - in Frage stellen (Ho MW et al. (1999) Microbial Ecology in Health and Disease 11:194-197; Cummins J et al. (2000) Nature Biotechnology 18:363). Ein Ziel künftiger biotechnologischer Arbeiten an Pflanzen ist daher der Ersatz viraler genetischer Elemente durch regulatorische pflanzliche Elemente, um so nah wie möglich am pflanzlichen System zu bleiben.

Aufgrund der herrschenden Bedenken hinsichtlich viraler Promotoren gibt es umfangreiche Bemühungen, diese durch pflanzliche Promotoren zu ersetzen. Ein den viralen Elementen vergleichbarer Promotor pflanzlicher Herkunft ist bislang jedoch noch nicht beschrieben worden.

Beschrieben wurde ein pflanzlicher Ubiquitin-Promotor aus Arabidopsis thaliana (Callis et al.(1990) J Biol Chem 265:12486-12493; Holtorf S et al. (1995) Plant Mol Biol 29;637-747). Entgegen den Befunden in den genannten Artikeln, ergaben eigene Untersuchungen, dass der Arabidopsis Ubiquitin Promotor für die Expression von Selektionsmarkergenen ungeeignet ist und seine allgemeine Anwendbarkeit aus diesem Grund in Frage gestellt werden muss (s. Vergleichsbeispiel 1 und 3).

Das vom Mais-Ubiquitin Promotor vermittelte Expressionsmuster wurde für die Ubi-1 and Ubi-2 Promotoren aus Mais beschrieben (Christensen et al. (1992) Plant Mol Biol 18(4):675-689). Während der Ubi-1 Promotor in Mais und anderen monokotylen Pflanzen gute Expressionsaktivität aufweist, zeigt er in dikotylen Tabakpflanzen nur 10% der Aktivität, die in vergleichbaren Experimenten mit dem viralen 35S Promotor erzielt worden war. Ferner wurde gezeigt, dass der Mais Ubi-1 Promotor für die Überexpression von Genen in monokotylen Pflanzensystemen geeignet ist und darüber hinaus auch hinreichend stark ist, um eine Herbizid-Resistenz durch Expression von Selektionsmarkern zu vermitteln (Christensen and Quail (1996) Transgenic Res 5(3):213-218). Für dikotyle Expressionssysteme erwies sich der Ubi-1 promotor als ungeeignet.

Ein Vergleich der Organspezifität und Stärke verschiedener konstitutiver Promotoren wurde von Holtorf (Holtorf et al. (1995) Plant Mol Biol 29(4):637-646) anhand stabil transformierter *Arabidopsis*-Pflanzen durchgeführt. Die Studie umfassßte u.a. den CaMV35S-Promotor, den blattspezifischen Thionin-Promotor aus Gerste und den *Arabidopsis* Ubiquitin-Promotor (UBQ1). Die höchste Expressionsrate zeigte der CaMV35S-Promotor. Anhand der Verwendung eines zusätzlichen translationalen Enhancers (TMV omega element) konnte die Expressionsrate des Promotors bei unveränderter Organspezifität um das Zwei- bis Dreifache gesteigert werden. Der blattspezifische Thionin-Promotor aus Gerste war in der Mehrzahl der transformierten Linien inaktiv, während der UBQ1 Promotor aus *Arabidopsis* mittlere Expressionsraten ergab.

McElroy und Mitarbeiter berichteten von einem auf dem Reis Actin 1 (Act1) Promotor basierenden Konstrukt zur Transformation monokotyler Pflanzen (McElroy et al. (1991) Mol Gen Genet 231:150-160). Insgesamt wurde aus den zuvor beschriebenen Untersuchungen geschlussßfolgert, dassß die auf dem Actl-Promotor basierenden Expressionsvektoren dazu geeignet sind, eine hinreichend starke und konstitutive Expression von Fremd-DNA in transformierten Zellen monokotyler Pflanzen zu steuern.

Als konstitutiver Promotor ist ferner der Promotor einer S-Adenosyl-L-Methionin Synthetase beschrieben (WO 00/37662). Nachteilig ist hier vor allem eine Abhänigigkeit der Expressionstärke von der Methioninkonzentration (siehe WO 00/37662; Fig. 7), wo 99/31258 beschreibt chimäre, konstitutive pflanzliche Promotoren, die sich aus verschiedenen Elementen verschiedener Promotoren mit komplementären Expressionsmustern zusammensetzen, so dass die Kombination einzelner Gewebespezifitäten additiv zu einem konstitutiven Expressionsmuster führt.

Die Ferredoxin NADPH Oxidoreduktase (FNR) ist ein Protein der Elektronentransportkette und reduziert NADP+ zu NADPH. Experimente in Spinat mit dem Spinat-FNR Promotor, der mit dem GUS Gen fusioniert wurde, deuten auf ein lichtinduzierbares Element im FNR Promotor (Oelmüller et al. (1993) Mol. Gen. Genet. 237:261-72). Aufgrund seiner Funktion wäre für den Promotor ein streng blattspezifisches Expressionsmuster zu erwarten gewesen. Aufgrund des gewebeabhängigen Expressionsmusters wäre der Promotor schlecht geeignet für die Expression von Selektionsmarkern. Hier ist eine Selektion in möglichst allen Gewebeteilen erforderlich, um eine effiziente Selektion zu gewährleisten.

Der Promotor des Triose-Phosphat-Translokators (TPT) sollte aufgrund seiner Funktion während der Photosynthese vorwiegend blattspezifisch sein. Beschrieben sind die cDNAs von Kartoffel (Schulz et al. (1993) Mol Gen Genet 238:357-61), Blumenkohl (Fischer et al. (1997) Plant Cell 9:453-62), Raps (WO 97/25346) und Mais Kammerer B (1998) The Plant Cell 10:105-117). Kammerer et al. zeigen, dass die Mais TPT mRNA Expression stark in den Blättern und den Staubgefäßen erfolgt. Keine Expression ist hingegen im Stengel oder den Wurzeln zu beobachten. Aufgrund des gewebeabhängigen Expressionsmusters wäre der Promotor schlecht geeignet für die Expression von Selektionsmarkern geeignet. Hier ist eine Selektion in möglichst allen Gewebeteilen erforderlich, um eine effiziente Selektion zu gewährleisten.

Die im Stand der Technik beschriebenen, sogenannten "konstitutiven" Promotoren weisen einen oder mehrere der nachfolgenden Nachteile auf:
1. Mangelhafte Homogenität der Expression:
   Die bekannten, sogenannten "konstitutiven" Promotoren zeigen vielfach eine unterschiedliche Expressionshöhe je nach Gewebe- oder Zelltyp. Zudem ist die Expressionseigenschaft oft stark von dem Insertionsort des Wirtsgenoms abhängig. Dies hat zur Folge, dass die durch heterologe Expression zu erzielenden Effekte nicht in dem gleichen Ausmaß homogen in der Pflanze erreicht werden können. Es kann zu Unter- oder Überdosierungen kommen. Dies kann sich nachteilig auf das Pflanzenwachstum oder den Pflanzenwert auswirken.
2. Mangelhaftes Zeitprofil:
   Die im Stand der Technik bekannten sogenannten "konstitutiven" Promotoren zeigen oft eine nicht konsistente Aktivität während der Entwicklung eines Gewebes. Damit können zum Beispiel in der frühen Phase der somatischen Embryogenese keine wünschenswerten Effekte (wie Selektion) erzielt werden, was gerade hier - aufgrund der Empfindlichkeit des Embryos gegen in vitro Bedingungen und Stressfaktoren - vorteilhaft wäre.
3. Mangelnde Anwendbarkeit auf viele Pflanzenarten:
   Die im Stand der Technik beschriebenen sogenannten "konstitutiven" Promotoren sind oft nicht in allen Arten in gleicher Weise aktiv.
4. Beim Vorliegen mehrerer Expressionskassetten mit jeweils dem gleichen "konstitutiven" Promotor in einem Organismus kann es zu Wechselwirkungen zwischen denselben und sogar zum Abschalten ("Gene Silencing") einzelner Expressionskassetten kommen (Mette et al. (1999) EMBO J. 18:241-248).
5. Promotoren viralen Ursprungs können durch Virusinfektionen der transgenen Pflanze beeinflusst werden und die gewünschte Eigenschaft dann nicht mehr ausprägen (A1-Kaff et al. (2000) Natur Biotechnology 18:995-99).
6. Die öffentliche Akzeptanz gegenüber der Verwendung von Promotoren und Elementen aus pflanzlichen Systemen ist höher als bei viralen Systemen.
7. Die Zahl der für die Expression von Selektionsmarkern in Pflanzen geeigneten Promotoren ist gering und sie sind gewöhnlicher Weise viralen oder bakteriellen Ursprungs.
8. Pollen/Antheren,-Expression: Die genannten Promotoren (wie beispielsweise 35S CaMV) zeigen eine starke Aktivität im Pollen oder den Staubbeuteln (Antheren). Dies kann unvorteilhafte Auswirkungen auf die Umwelt haben. So hatte eine unspezifische Expression von Bacillus thuringiensis Endotoxinen nicht nur den gewünschten Effekt auf Fraßinsekten durch Expression in der Wurzel, sondern auch infolge einer Expression im Pollen bedeutende Schäden im Bestand des Monarchfalters zur Folge, der den Pollen als Hauptnahrungsquelle nutzt (Losey JE et al. (1999) Nature 399, 214).

Ein idealer konstitutiver Promotor sollte möglichst zahlreiche der nachfolgenden Eigenschaften haben:
a) Möglichst homogene lokale und zeitliche Genexpression d.h. eine Expression in möglichst vielen Zelltypen oder Geweben eines Organismus während der verschiedenen Phasen des Entwicklungszyklus. Gewünscht wird ferner eine effiziente Selektion in dedifferentierten Zellen (verschiedenen Kallus-Pfhasen) aus einer Gewebekultur und weiteren Entwicklungsstadien, die für die Gewebekultur geeignet sind.
b) Möglichst breite Anwendbarkeit auf verschiedene Pflanzenarten bzw. Anwendbarkeit auf Arten, in denen mit den bisher bekannten "konstitutiven" Promotoren keine Expression erreicht werden kann.
c) Um eine Kombination von mehreren Transgenen in einer Pflanze zu realisieren, ist es wünschenswert, mehrere Transformationen hintereinander zu schalten oder Konstrukte mit mehreren Promotor-Kassetten zu verwenden, ohne dassß durch die mehrmalige Verwendung identischer regulatorischer Sequenzen Silencing-Effekte erzeugt werden.
d) Einen pflanzlichen Ursprung zur Vermeidung von Akzeptanzproblemen beim Konsumenten und eventuellen zukünftigen Zulassungsproblemen.
e) Nebenaktivitäten eines Promotors in den Antheren/Pollen sind nicht wünschenswert, zum Beispiel um Umweltschäden zu vermeiden (s.o.).

Die der vorliegenden Erfindung zugrunde liegende Aufgabe bestand also in der Bereitstellung von pflanzlichen regulatorischen Sequenzen, die möglichst viele der oben genannten Eigenschaften erfüllen, vor allem eine ubiquitäre und entwicklungsunabhängige (konstitutive) Expression einer zu exprimierenden Nukleinsäuresequenz - bevorzugt kodierend für einen Selektionsmarker - vermitteln. Trotz verschiedener pflanzlicher Promotoren für die eine konstitutive Expression zumindest in einzelnen Arten beansprucht wird, wurde bislang kein Promotor mit den oben aufgezählten gewünschten Eigenschaften beschrieben. Es bestand daher die Aufgabe, entsprechende Promotoren zu identifizieren.

Diese Aufgabe wurde gelöst durch Bereitstellung von Expressionskassetten basierend auf dem Promotor des Triose-Phosphat Translokator (TPT) Gens aus *Arabidopsis thaliana:*
1.) Promotor eines putativen Ferredoxin (pFD) Promotor aus *Arabidopsis thaliana*
   Bei der Analyse des Arabidopsis Genoms wurde der ORF eines putativen Ferredoxingens identifiziert. Die isolierten 836 bp 5'- flankierende Sequenz fusioniert mit dem Gen der Glucuronidase zeigten in transgenem Tabak überraschenderweise ein konstitutives Expressionsmuster. Die Sequenz entspricht einem Sequenzabschnitt auf Chromosom 4 von Arabidopsis thaliana wie er in der GenBank unter der Acc.-No. Z9'7337 hinterlegt ist (Version Z97337.2; Basenpaar 85117 bis 85952; das Gen beginnend ab bp 85953 ist mit "strong similarity to ferredoxin [2Fe-2S] I, Nostoc muscorum" annotiert). (Das Gen ist nicht zu verwechseln mit dem unter der GenBank Acc.-No: X51370 annotierten A. thaliana Gen für Preferredoxin; Vorst O et al. (1990) Plant Mol Biol 14(4):491-499).
   In den Antheren/Pollen der geschlossenen Blütenknospen konnte nur eine schwache Aktivität, in reifen Blüten gar keine mehr detektiert werden. Entgegen denm aus den Literaturbefunden abgeleiteten Vorbehalten gegen eine Eignung des Promotors zur effektiven Expression von Selektionsmarkern (zum Beispiel aufgrund der vermuteten Blattspezifität oder der Funktion im photosynthetischen Elektronentransport), konnte eine hocheffiziente Selektion durch Kombination mit beispielsweise dem Kanamycin-Resistenzgen (nptII) demonstriert werden.
2.) Ferredoxin NADPH Oxidoreduktase (FNR) Promotor aus *Arabidopsis thaliana*
   Ausgehend von den Informationen über die FNR-kodierende cDNA aus N. tabacum (GenBank Acc.-No.: Y14032) wurde in der Arabidopisdatenbank nach einem homologen Gen gesucht. Entsprechend dieser Sequenzinformationen wurden Primer synthetisiert. Der über PCR aus genomischer DNA von Arabidopsis thaliana amplifizierte Promotor (6354 bp), von dem eine blattspezifische Expression erwartet wurde, zeigte in transgenen Tabakpflanzen eine überraschend ubiquitäre und vom Insertionsort unabhängige Expression.
   Die Promotorsequenz entspricht teilweise einem Sequenzabschnitt auf Chromosom 5 von Arabidopsis thaliana wie er in der GenBank unter der Acc.-No. AB011474 hinterlegt ist (Version AB011474.1 vom 27.12.2000; Basenpaar 70127 bis 69493; das Gen beginnend ab bp 69492 ist mit "ferredoxin-NADP+ reductase" annotiert).
   In den Pollen konnte keine Aktivität detektiert werden. Entgegen denm aus den Literaturbefunden abgeleiteten Vorbehalten gegen eine Eignung des Promotors zur effektiven Expression von Selektionsmarkern (zum Beispiel aufgrund der vermuteten Blattspezifität oder der Funktion im photosynthetischen Elektronentransport), konnte eine hocheffiziente Selektion durch Kombination mit beispielsweise dem Phosphinothricin-Resistenzgen (bar/pat) demonstriert werden.
   Die nicht nachweisbare Aktivität des FNR-Promoters in Samen erlaubt eine Nutzung zur Expression von Genen deren Genprodukte in anderen Pflanzenteilen erwünscht und in den Samen unerwünscht sind. Beispielsweise kann eine Abwehr von Schädlingen durch Expression entsprechender Toxine, wie beispielsweise Bacillus thuringiensis Kristallproteinen erfolgen. So kann in Kartoffel eine Expression in den oberirdischen Pflanzenorganen (und damit z.B. eine Abwehr von Schädlingen, wie dem Kartoffelkäfer) erreicht werden, ohne dass zu gleich eine Expression in der als Nahrungs- oder Futtermittel genutzten Knolle erfolgt, was die Eignung und Akzeptanz erhöhen könnte.
3.) Triose-Phosphat Translokator (TPT) Promotor aus *Arabidopsis thaliana*
   Ein 2038bp langes PCR Fragment wurde amplifiziert ausgehend von Arabidopsis Genbankdaten vom Chromosom V, clone MCL19. Die Promotorsequenz entspricht zum Teil einem Sequenzabschnitt auf Chromosom 5 von Arabidopsis thaliana wie er in der GenBank unter der Acc.-No. AB006698 hinterlegt ist (Version AB006698.1 vom 27.12.2000; Basenpaar 53242 bis 55281; das Gen beginnend ab bp 55282 ist mit "phosphate/triosephosphate translocator" annotiert).
   Transgene Tabakpflanzen zeigten überraschender Weise nicht nur eine starke Aktivität in zahlreichen Pflanzenteilen. In den Pollen konnte keine Aktivität detektiert werden. Entgegen denm aus den Literaturbefunden abgeleiteten Vorbehalten gegen eine Eignung des Promotors zur effektiven Expression von Selektionsmarkern (zum Beispiel aufgrund der vermuteten Blattspezifität), konnte eine hocheffiziente Selektion durch Kombination mit beispielsweise dem Phosphinothricin-Resistenzgen (bar/pat) demonstriert werden.
   Das ubiquitäre Expressionsmuster, vor allem aber auch die Fähigkeit des TPT Promotors hinsichtlich der Expression von Selektionsmarkern stellt eine große Überraschung für den Fachmann dar, da der Triossphosphattranslokator verantwortlich für den Austausch von C3 Zuckerphosphaten zwischen Cytosol und Plastiden in photosynthetischen Blättern ist. Der TPT ist in der inneren Chloroplasten Membran lokalisiert. Farblose Plastiden enthalten typischerweise Hexosetransporter über den derer C6-Zuckerphosphat Austausch erfolgt. Es ist nicht zu erwarten, daß dass solche Gene in den frühen Callus und Embryogenese Stadien aktiv sind (Stitt (1997) Plant Metabolism, 2nd ed., Dennis eds. Longman Press, Harlow, UK, 382-400) .

Sowohl der pFD, FNR als auch der TPT Promotor erwiesen sich als hinreichend stark, um Nukleinsäuresequenzen insbesondere Selektionsmarkergene erfolgreich zu exprimieren. Ferner erwiesen sich verschiedene Deletionsvarianten der oben genannten Promotoren, insbesondere eine verkürzte Variante des pFD Promotors (699 bp) und des TPT Promotors (1318 bp), als geeignet die Expression von beispielsweise Selektionsmarkern wie der Kanamycin-Resistenz (nptII) zu gewährleisten.

Weiterhin wurden im Rahmen der genannten Arbeiten der *Arabidopsis thaliana* Ubiquitin-Promotor (Holtorf et al. (1995) Plant Mol Biol 29:637-646) und der Squalen Synthase-Promotor (Kribii et al. (1997) Eur J Biochem 249:61-69) untersucht, die aber beide überraschenderweise nicht geeignet waren, Selektionsmarkergen-Expression zu vermitteln, obwohl die Literaturdaten der Ubiquitin-Promotoren aus Monokotylen (siehe oben) hatten vermuten lassen, dassß insbesondere der Ubiquitin-Promotor einer dikotylen Pflanze als Promotor eines Selektionsmarkersystems hätte funktionieren müssen (siehe Vergleichsbeispiele 1 und 3). Ähnliches gilt für den Squalensynthase-Promotor, dessen Charakterisierung hätte erwarten lassen, dass ausreichend hohe Expressionsraten für die erfolgreiche Steuerung eines Selektionsmarkergens erzielt werden können (Del Arco and Boronat (1999) 4th European Symposium on Plant Isoprenoids, 21.-23.4.1999, Barcelona, Spanien) (siehe Vergleichsbeispiele 2 und 3).

Ein erster Gegenstand der Erfindung betrifft daher Expressionskassetten zur transgenen Expression von Nukleinsäuren enthaltend:
a) einen Promotor gemäß SEQ ID NO: 3 oder,
b) funktionelle Aquivalente aus gewählt aus der Gruppe bestehnd aus
   i) Triose-Phosphat Translokator Promoter aus *Arabidopsis thaliana* amplifizierbar Primern der mit SEQ ID No.: 17 und 18 oder SEQ ID No.: 18 und 26;
   ii) Sequenzen, die unter Bedingungen mit 0,2x SSC bei 50°C mit der Nukleinsäuresequenz kodierend für einen Promotor gemäß SEQ ID NO; 3 oder der zu ihr komplementären Nukleinsäuresequenzen hybridisieren, und
   iii)dem äquivalente Fragmente von a) beschrieben durch durch SEQ ID NO: 27,
      die im wesentlichen die gleichen Promotoraktivitäten wie a) besitzen,
   wobei a) oder b) funktionell mit einer transgen zu exprimierenden Nukleinsäuresequenz verknüpft sind und deren ubiquitäre und entwicklungsunabhängige Expression vermitteln.

Ferner betrifft die Erfindung Verfahren zur transgenen Expression von Nukleinsäuren, dadurch gekennzeichnet, dass eine Nukleinsäuresequenz in funktioneller Verknüpfung mit
a) einemn Promotor gemässgemäß SEQ ID NO:3, oder
b) einem oben genannten funktionellen Äquivalent von a), das im wesentlichen die gleichen Promotoraktivitäten wie a) besitzt,
transgen exprimiert wird.

Expression umfasst die Transkription der transgen zu exprimierenden Nukleinsäuresequenz, kann aber - im Falle eines offenen Leserasters in "sense"-Orientierung - auch die Translation der transkribierten RNA der transgen zu exprimierenden Nukleinsäuresequenz in ein korrespondierendes Polypeptid mit einschließen.

Expressionskassette zur transgenen Expression von Nukleinsäuren oder Verfahren zur transgenen Expression von Nukleinsäuren umfasst alle solche durch gentechnische Methoden zustande gekommene Konstruktionen oder Verfahren, in denen sich entweder
a) ein Promotor gemäß SEQ ID NO: 3 oder ein genanntes funktionelles Äquivalent , oder
b) die zu exprimierende Nukleinsäuresequenz, oder
c) (a) und (b)
sich nicht in ihrer natürlichen, genetischen Umgebung (d.h. an ihrem natürlichen chromosomalen Locus) befinden oder durch gentechnische Methoden modifiziert wurden, wobei die Modifikation beispielhaft eine Substitution, Addition, Deletion, Inversion oder Insertion eines oder mehrerer Nukleotidreste sein kann.

Die erfindungsgemäßen Expressionskassetten, von ihnen abgeleitete Vektoren oder die erfindungsgemäßen Verfahren können die genannten funktionelle Äquivalente zu den unter SEQ ID NO: 3 beschriebenen Promotorsequenzen umfassen. Funktionell äquivalente Sequenzen umfassen auch all die Sequenzen, die von dem komplementären Gegenstrang denr durch SEQ ID NO:3 definierten Sequenzen abgeleitet sind und im wesentlichen die gleiche Promotoraktivität aufweisen.

Funktionelle Äquivalente in bezug auf die erfindungsgemäßen Promotoren meint insbesondere natürliche oder künstliche Mutationen der unter SEQ ID NO: 3 beschriebenen Promotorsequenzen sowie derenseine Homologen aus anderen Pflanzengattungen und -arten, welche weiterhin im wesentlichen die gleiche Promotoraktivität aufweisen.

Eine Promotoraktivität wird im wesentlichen als gleich bezeichnet, wenn die Transkription eines bestimmten zu exprimierenden Gens unter Kontrolle eines bestimmten von SEQ ID NO: 3 abgeleiteten Promotors unter ansonsten unveränderten Bedingungen eine Lokalisation innerhalb der Pflanze aufweist, die zu mindestens 50%, bevorzugt mindestens 70%, besonders bevorzugt mindesten 90% ganz besonders bevorzugt mindestens 95% deckungsgleich ist mit einer Vergleichsexpression erhalten unter Verwendung eines des durch SEQ ID NO: 3 beschriebenen Promotors. Dabei kann die Expressionshöhe sowohl nach unten als auch nach oben im Vergleich zu einem Vergleichswert abweichen. Bevorzugt sind dabei solche Sequenzen, deren Expressionshöhe, gemessen anhand der transkribierten mRNA oder dem infolge translatierten Protein, unter ansonsten unveränderten Bedingungen quantitativ um nicht mehr als 50%, bevorzugt 25%, besonders bevorzugt 10 % sich von einem Vergleichswert erhalten mit einem durch SEQ ID NO: 3 beschriebenen Promotor unterscheidet. Besonders bevorzugt sind solche Sequenzen, deren Expressionshöhe, gemessen anhand der transkribierten mRNA oder dem infolge translatierten Protein, unter ansonsten unveränderten Bedingungen quantitativ um mehr als 50%, bevorzugt 100%, besonders bevorzugt 500%, ganz besonders bevorzugt 1000% eines vergleichswert erhalten mit dem durch SEQ ID NO:3 beschriebenen Promotor übersteigt. Bevorzugt ist als Vergleichs-wert die Expressionshöhe der natürlichen mRNA des jeweiligen Gens oder des natürlichen Genproduktes. Bevorzugt ist ferner als Vergleichswert die Expressionshöhe erhalten mit einer beliebigen, aber bestimmten Nukleinsäuresequenz, bevorzugt solchen Nuklein-säuresequenzen, die für leicht quantifizierbare Proteine kodieren. Ganz besonders bevorzugt sind dabei Reporter-Proteine (Schenborn E, Groskreutz D. Mol Biotechnol. 1999; 13(1):29-44) wie das "green fluorescence protein" (GFP) (Chui WL et al., Curr Biol 1996, 6:325-330; Leffel SM et al., Biotechniques. 23(5):912-8, 1997), die Chloramphenicoltransferase, eine Luziferase (Millar et al., Plant Mol Biol Rep 1992 10:324-414) oder die β-Galactosidase, ganz besonders bevorzugt ist die β-Glucuronidase (Jefferson et al. (1987) EMBO J. 6:3901-3907).

Ansonsten unveränderte Bedingungen bedeutet, dass die Expression, die durch eine der zu vergleichenden Expressionskassetten initiiert wird, nicht durch Kombination mit zusätzlichen genetischen Kontrollsequenzen, zum Beispiel Enhancer-Sequenzen, modifiziert wird. Unveränderte Bedingungen bedeutet ferner, dass alle Rahmenbedingungen wie beispielsweise Pflanzenart, Entwicklungsstadium der Pflanzen, Zuchtbedingungen, Assaybedingungen (wie Puffer, Temperatur, Substrate etc.) zwischen den zu vergleichenden Expressionen identisch gehalten werden.

Mutationen umfassen Substitutionen, Additionen, Deletionen, Inversionen oder Insertionen eines oder mehrerer Nukleotidreste. Somit werden beispielsweise auch solche Nukleotidsequenzen durch die vorliegende Erfindung mit umfasst, welche man durch Modifikation eines Promotors gemässgemäß SEQ ID NO: 3 erhält. Ziel einer solchen Modifikation kann die weitere Eingrenzung der darin enthaltenen Sequenz oder z.B. auch die Einfügung weiterer Restriktionsenzymschnittstellen, die Entfernung überflüssiger DNA oder das Hinzufügen weiterer Sequenzen, zum Beispiel weiterer regulatorischer Sequenzen, sein.

Wo Insertionen, Deletionen oder Substitutionen, wie z.B. Transitionen und Transversionen, in Frage kommen, können an sich bekannte Techniken, wie in vitro-Mutagenese, "primer repair", Restriktion oder Ligation verwendet werden. Durch Manipulationen, wie z.B. Restriktion, "chewing-back" oder Auffüllen von Überhängen für "blunt ends" können komplementäre Enden der Fragmente für die Ligation zur Verfügung gestellt werden. Zu analogen Ergebnissen kann man auch unter Verwendung der Polymerasekettenreaktion (PCR) unter Verwendung spezifischer Oligonukleotid-Primer kommen.

Unter Homologie zwischen zwei Nukleinsäuren wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2,912 | Average Mismatch:-2,003 |

Beispielhaft wird unter einer Sequenz, die eine Homologie von mindestens 50 % auf Nukleinsäurebasis mit der Sequenz SEQ ID NO: 3 aufweist, eine Sequenz verstanden, die bei einem Vergleich mit der Sequenz SEQ ID NO. 3 nach obigem Programmalgorithmus mit obigem Parametersatz eine Homologie von mindestens 50 aufweist.

Als funktionelle Hhomologe zu den obengenannten Promotoren zur Verwendung in den erfindungsgemäßen Expressionskassetten sind beispielsweise solche Sequenzen beschrieben, die die eine Homologie aufweisen von mindestens 50 %, z. B. 70 %, z. B. mindestens 80 %, z. B. mindestens 90 %, z.B. mindestens 95 %, z.B. 99% über eine Länge von von mindestens 100 Basenpaaren, z. B. mindestens 200 Basenpaaren, z. B. von mindestens 300 Basenpaaren, z. B. mindestens 400 Basenpaaren, z. B. mindestens 500 Basenpaaren.

Weitere Beispiele für die in den erfindungsgemäßen Expressionskassetten oder Vektoren zum Einsatz kommenden Promotorsequenzen lassen sich beispielsweise aus in verschiedenen Organismen, deren genomische Sequenz bekannt ist, wie beispielsweise aus Arabidopsis thaliana, Brassica napus, Nicotiana tabacum, Solanum tuberosum, Helianthium anuus, Linum sativum durch Homologievergleiche ausin Datenbanken leicht auffinden.

Funktionelle Äquivalente meint ferner DNA Sequenzen, die unter Standardbedingungen mit der Nukleinsäuresequenz kodierend für einen Promotor gemäß SEQ ID NO: 3 oder der zu ihr komplementären Nukleinsäuresequenzen hybridisieren und die im wesentlichen gleichen Eigenschaften haben. Standardhybridisierungsbedingungen ist breit zu verstehen und meint sowohl stringente als auch weniger stringente Hybridisierungsbedingungen. Solche Hybridisierungsbedingungen sind unter anderem bei Sambrook J, Fritsch EF, Maniatis T et al., in Molecular Cloning - (A Laboratory Manual), 2. Auflage, Cold Spring Harbor Laboratory Press, 1989, Seiten 9.31-9.57) oder in Current Protocols in Molecular Biology, John Wiley & Sons, N.Y. (1989), 6.3.1-6.3.6. beschrieben.

Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3 M Natriumcitrat, 3 M NaCl, pH 7.0). Darüber hinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige beispielhafte Bedingungen für Hybridisierung und Waschschritt sind infolge gegeben:
(1) Hybridisierungsbedingungen mit zum Beispiel
   a) 4X SSC bei 65°C, oder
   b) 6X SSC, 0.5% SDS, 100 µg/ml denaturierter, fragmentierte Lachssperma-DNA bei 65°C, oder
   c) 4X SSC, 50% Formamid, bei 42°C, oder
   d) 6X SSC, 0.5% SDS, 100 µg/ml denaturierter, fragmentierte Lachssperma-DNA, 50% Formamid bei 42°C, oder
   e) 2X oder 4X SSC bei 50°C (schwach stringente Bedingung), oder
   f) 2X oder 4X SSC, 30 bis 40% Formamid bei 42°C (schwach stringente Bedingung).
   g) 6x SSC bei 45 °C, oder,
   h) 50% Formamid, 4xSSC bei 42 °C, oder
   i) 50% (vol/vol) Formamid, 0,1% Rinderserumalbumin, 0,1% Ficoll, 0,1% Polyvinylpyrrolidon, 50 mM Natriumphosphatpuffer pH 6,5, 750 mM NaCl, 75 mM Natriumcitrate bei 42°C, oder
   j) 0,05 M Natriumphosphatpuffer pH 7,0, 2 mM EDTA, 1% BSA und 7% SDS.
(2) Waschschritte mit zum Beispiel
   a) 0,.1X SSC bei 65°C, oder
   b) 0,1X SSC, 0,5% SDS bei 68°C, oder
   c) 0,1X SSC, 00,5% SDS, 50% Formamid bei 42°C, oder
   d) 0,2X SSC, 0,1% SDS bei 42°C, oder
   e) 2X SSC bei 65°C (schwach stringente Bedingung), oder
   f) 40 mM Natriumphosphatpuffer pH 7,0, 1% SDS, 2 mM EDTA.

Verfahren zur Herstellung erfindungsgemäßer funktioneller Äquivalente umfasst bevorzugt die Einführung von Mutationen in einen Promotor gemäß SEQ ID NO: 3. Eine Mutagenese kann ungerichtet ("random") erfolgen, wobei die mutagenisierten Sequenzen anschließend bezüglich ihrer Eigenschaften nach einer "trial-by-error" Prozedur durchmustert werden. Besonders vorteilhafte Selektionskriterien umfassen beispielsweise eine erhöht Resistenz gegenüber einem Selektionsmarker, die Höhe der resultierenden Expression der eingeführten Nukleinsäuresequenz.

Alternativ können nicht-essentielle Sequenzen eines der erfindungsgemäßen Promotors deletiert werden ohne die genannten Eigenschaften signifikant zu beeinträchtigen. Derartige Deletionsvarianten stellen funktionell äquivalente FragmenteTeile ders Promotorens beschrieben durch SEQ ID NO: 3 dar. Beispielhaft sei für derartigeso eine Deletionsmutantent oder funktionell äquivalente Fragmente die verkürzte pFD-Promotorsequenz (pFDs) gemäß SEQ ID NO: 4 oder die verkürztet TPT-Promotorsequenz gemäß SEQ ID NO: 27 zu nennen, die als funktionell äquivalentes Teile ihrer jeweiligen Ausgangspromotoren aussdrücklich mit umfasst sindei.

Die Eingrenzung der Promotorsequenz auf bestimmte, essentielle regulatorische Regionen kann auch mit Hilfe von Suchroutine zur Suche von Promotorelementen vorgenommen werden. Oft sind in den für die Promotoraktivität relevanten Regionen bestimmte Promotorelemente gehäuft vorhanden. Diese Analyse kann beispielsweise mit Computerprogrammen wie dem Programm PLACE ("Plant Cis-acting Regulatory DNA Elements") vorgenommen werden (K. Higo et al., (1999) Nucleic Acids Research 27:1, 297-300) oder der BIOBASE Datenbank "Transfac" (Biologische Datenbanken GmbH, Braunschweig)

Verfahren zur Mutagenisierung von Nukleinsäuresequenzen sind dem Fachmann bekannt und schließen beispielhaft die Verwendung von Oligonukleotiden mit einer oder mehr Mutationen im Vergleich zu der zu mutierenden Region ein (z.B. im Rahmen einer "Sitespecific mutagenesis"). Typischerweise kommen Primer mit ungefähr 15 bis ungefähr 75 Nukleotiden oder mehr zum Einsatz, wobei bevorzugt ca. 10 bis ca. 25 oder mehr Nukleotidreste an beiden Seiten der zu verändernden Sequenz lokalisiert sind. Details und Durchführung besagter Mutageneseverfahren sind dem Fachmann geläufig (Kunkel et al., Methods Enzymol, 154:367-382, 1987; Tomic et al. (1990) Nucl Acids Res 12:1656; Upender, Raj, Weir (1995) Biotechniques 18(1):29-30; US 4,237,224), Eine Mutagenese kann auch durch Behandlung von beispielsweise Vektoren, die eine der erfindungsgemäßen Nukleinsäuresequenzen enthalten, mit mutagenisierenden Agentien wie Hydroxylamin realisiert werden.

Die in den erfindungsgemässgemäßen Expressionskassetten enthaltenen transgen zu exprimierenden Nukleinsäuresequenzen können mit weiteren genetischen Kontrollsequenzen neben einem der erfindungsgemäßen Promotoren funktionell verknüpft sein.

Unter einer funktionellen Verknüpfung versteht man zum Beispiel die sequentielle Anordnung eines Promotors, der transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierenden Nukleinsäuresequenz hinter der als Promotor fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung kann mittels gängiger Rekombinations- und Klonierungstechniken realisiert werden, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen.

Der Begriff der genetischen Kontrollsequenzen ist breit zu verstehen und meint all solche Sequenzen, die einen Einfluss auf das Zustandekommen oder die Funktion der erfindungsgemässgemäßen Expressionskassette haben. Genetische Kontrollsequenzen modifizieren zum Beispiel die Transkription und Translation in prokaryotischen oder eukaryotischen Organismen. Vorzugsweise umfassen die erfindungsgemässgemäßen Expressionskassetten 5'-stromaufwärts von der jeweiligen transgen zu exprimierenden Nukleinsäuresequenz einen der erfindungsgemäßen Promotoren und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionsteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH , (1991) J Biol Chem 1991; 266(26): 17131 -17135) und Hitzestress (Schöffl F et al.,(1989) Molecular & General Genetics 217(2-3):246-53, 1989) beschrieben.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel *E.coli* Bakterien ermöglichen. Als Pflanzenpromotoren kommen im Prinzip alle oben beschriebenen Promotoren in Frage. Vorstellbar ist zum Beispiel, dass eine bestimmte Nukleinsäuresequenz durch einen Promotor (zum Beispiel einen der erfindungsgemäßen Promotoren) in einem Pflanzengewebe als sense-RNA transkribiert und in das entsprechende Protein translatiert wird, während die gleiche Nukleinsäuresequenz durch einen anderen Promotor mit einern anderen Spezifität in einem anderen Gewebe zu anti-sense-RNA transkribiert und das entsprechende Protein herunterreguliert wird. Dieses kann durch eine erfindungsgemässgemä5e Expressionskassette realisiert werden, indem der eine Promotor vor die transgen zu exprimierende Nukleinsäuresequenz positioniert wird und der andere Promotor dahinter.

Genetische Kontrollsequenzen umfassen ferner auch die 5'-untranslatierte Region, Introns oder die nichtkodzexende 3'-Region von Genen, bevorzugt der pFD, FNR oder TPT-Genes. Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Sie können ferner die Gewebespezifität fördern (Rouster J et al.(1998) Plant J. 15:435-440.).

Umgekehrt unterdrückt die 5'-untranslatierte Region des opaque-2 Gens die Expression. Eine Deletion der entsprechenden Region führt zu einer Erhöhung der Genaktivität (Lohmer S et al. (1993) Plant Cell 5:65-73). Die unter SEQ ID NO:1, 2 oder 3 angegebene Nukleinsäuresequenz enthält beispielsweise den Abschnitt der pFD, FNR oder TPT- -Genes, der den Promotor und die 5'-untranslatierte Region bis vor das ATG-Startcodon des jeweiligen Proteins repräsentiert.

McElroy und Mitarbeiter (McElroy et al. (1991) Mol Gen Genet 231(1):150-160) berichteten von einem auf dem Reis Actin 1 (Act1) Promotor basierenden Konstrukt zur Transformation monokotyler Pflanzen. In transgenen Reiszellen führte die Verwendung des Act1-Introns in Kombination mit dem 35S-Promotor zu einer gegenüber dem isolierten 35S-Promotor um das Zehnfache gesteigerten Expressionsrate. Eine Optimierung der Sequenzumgebung der Translations-Initiationsstelle des Reportergen-Gens (GUS) resultierte in einer vierfachen Steigerung der GUS-Expression in transformierten Reiszellen. Eine Kombination der optimierten Translations-Initiationsstelle und des Actl-Introns resultierte in einer 40-fachen Steigerung der GUS-Expression durch den CaMV35S-Promotor in transformierten Reiszellen; ähnliche Ergebnisse wurden anhand von transformierten Maiszellen erzielt. Insgesamt wurde aus den zuvor beschriebenen Untersuchungen geschlussfolgert, daß dass die auf dem Actl-Promotor basierenden Expressionsvektoren dazu geeignet sind, eine hinreichend starke und konstitutive Expression von Fremd-DNA in transformierten Zellen monokotyler Pflanzen zu steuern.

Die Expressionskassette kann vorteilhafterweise eine oder mehrere sogenannte "enhancer Sequenzen" funktionell verknüpft mit dem Promotor enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien in einer der erfindungsgemäßen Expressionskassetten enthalten sein.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promotor eines bestimmten Gens gegen einen der erfindungsgemäßen Promotoren ausgetauscht werden. Methoden wie die cre/lox-Technologie erlauben eine gewebesspezifische, unter Umständen induzierbare Entfernung der Expressionskassette aus dem Genom des Wirtsorganismus (Sauer B. (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Der einzuführende Promotor kann mittels homologer Rekombination vor das transgen zu exprimierende Zielgen platziert werden, indem der Promotor mit DNA-Sequenzen verknüpft wird, die zum Beispiel zu endogenen Sequenzen homolog sind, die dem Leseraster des Zielgens vorgelagert sind. Derartige Sequenzen sind als genetische Kontrollsequenzen zu verstehen. Nachdem eine Zelle mit dem entsprechenden DNA-Konstrukt transformiert wurde, können die beiden homologen Sequenzen interagieren und so die Promotorsequenz an dem gewünschten Ort vor dem Zielgen platzieren, so dass die Promotorsequenz nun in funktioneller Verknüpfung mit dem Zielgen steht und eine erfindungsgemässgemäße Expressionskassette bildet. Die Auswahl der homologen Sequenzen bestimmt den Insertionsort des Promotors. Hierbei kann die Expressionskassette durch homologe Rekombination mittels einer einfachen oder einer doppeltenreziproken Rekombination generiert werden. Bei der einfach reziproken Rekombination wird nur eine einzelne Rekombinationssequenz verwendet und es erfolgt die Insertion der gesamten eingeführten DNA. Bei der doppelt-reziproken Rekombination ist die einzuführende DNA durch zwei homologe Sequenzen flankiert und es erfolgt die Insertion des flankierten Bereiches. Letzteres Verfahren ist geeignet, um wie oben beschrieben den natürlichen Promotor eines bestimmten Gens gegen einen der erfindungsgemäßen Promotoren auszutauschen und so den Expressionsort und -zeitpunkt dieses Gens zu modifizieren. Diese funktionelle Verknüpfung stellt eine erfindungsgemässgemäße Expressionskassette dar.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al., Plant Cell Reports 5 (1986), 81-84).

Homologe Rekombination ist ein relativ seltenes Ereignis in höheren Eukaryoten, vor allem in Pflanzen, Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben. Dieses besteht aus drei Elementen: zwei Paaren von spezifischen Rekombinationssequenzen und einer sequenzspezifischen Rekombinase. Diese Rekombinase katalysiert eine Rekombination lediglich zwischen den beiden Paaren von spezifischen Rekoxnbznatzonsequenzen. Das eine Paar dieser spezifischen DNA-Sequenzen wird aussßerhalb der zu integrierenden DNA-Sequenz, d.h. außsserhalb der beiden homologen DNA-Sequenzen lokalisiert. Im Falle einer korrekten homologen Rekombination werden diese Sequenzen nicht mit in das Genom übertragen. Im Falle einer zufälligen Integration insertieren sie in der Regel zusammen mit dem Rest des Konstruktes. Unter Einsatz einer speziellen Rekombinase und eines Konstruktes enthaltend ein zweites Paar der spezifischen Sequenzen können die zufällig insertierten Sequenzen herausgeschnitten oder durch Inversion inaktiviert werden, während die korrekt über homologe Rekombination insertierten Sequenzen im Genom verbleiben. Eine Vielzahl von sequenzspezifischen Rekombinationssystemen kann verwendet werden, beispielhaft sind das Cre/lox-System des Bacteriophagen P1, das FLP/FRT System der Hefe, die Gin Recombinase des Mu Phagen, die Pin Rekombinase aus E. coli und das R/RS System des pSR1 Plasmids genannt. Bevorzugt sind das Bacteriophagen P1 Cre/lox und das Hefe FLP/FRT System. Hier interagiert die Rekombinase (Cre oder FLP) spezifisch mit ihren jeweiligen Rekombinationssequenzen (34bp lox-Sequenz bzw. 47bp FRT-Sequenz) um die zwischengelagerten Sequenzen zu deletieren oder zu invertieren. Das FLP/FRT und cre/lox Rekombinasesystem wurde bereits in pflanzlichen Systemen angewendet (Odell et al.(,1990) Mol. Gen. Genet., 223:369-378, 1990.)

Als Kontrollsequenzen geeignete Polyadenylierungssignale sind pflanzliche Polyadenylierungssignale, sowie - vorzugsweise - solche, die im wesentlichen T-DNA Polyadenylierungssignalen aus *Agrobacterium tumefaciens,* insbesondere des Gens 3 der T-DNA (Octopin Synthase) des Ti-Plasmids pTiACHS entsprechen (Gielen et al.,(1984) EMBO J. 3 :(1984), 835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

In einer besonders bevorzugten Ausführungsform enthält die Expressionskassette eine in Pflanzen funktionelle Terminatorsequenz. In Pflanzen funktionelle Terminatorsequenzen meint allgemein solche Sequenzen, die in der Lage sind, in Pflanzen den Abbruch der Transkription einer DNA-Sequenz zu bewirken. Beispiele für geeignete Terminatorsequenzen sind der OCS (Octopin-Synthase)-Terminator und der NOS (Nopalin-Synthase)-Terminator. Besonders bevorzugt sind jedoch pflanzliche Terminatorsequenzen. Pflanzliche Terminatorsequenzen meint allgemein solche Sequenzen, die Bestandteil eines natürlichen pflanzlichen Gens sind. Besonders bevorzugt sind dabei der Terminators des Cathepsin D Inhibitor Gens aus Kartoffel (GenBank Acc. No.: X74985; Terminator: SEQ ID NO: 28) oder des Terminators der Speicherproteingens VfLE1B3 (GenBank Acc. No.: Z26489; Terminator: SEQ ID NO: 29) aus der Ackerbohne. Diese Terminatoren sind den im Stand der Technik beschriebenen viralen oder T-DNA Terminatoren mindestens gleichwertig. Das Plasmid pSUN5NPTIICat (SEQ ID NO: 24) enthält den pflanzlichen Terminator des Cathepsin D Inhibitor Gens aus Kartoffel.

Dem Fachmann ist eine Vielzahl von Nukleinsäuren bzw. Proteinen bekannt, deren rekombinante Expression gesteuert durch die erfindungsgemäßen Expressionskassetten oder Verfahren vorteilhaft ist. Ferner sind dem Fachmann eine Vielzahl von Genen bekannt, durch deren Reprimierung oder Ausschaltung mittels Expression einer entsprechenden antisense-RNA ebenfalls vorteilhafte Effekte erreicht werden können. Beispielhaft jedoch nicht einschränkend für vorteilhafte Effekte seien zu nennen:
- Erleichterte Herstellung eines transgenen Organismus beispielsweise durch die Expression von Selektionsmarkern
- Erzielen einer Resistenz gegen abiotische Stressfaktoran (Hitze, Kälte, Trockenheit, erhöhte Feuchtigkeit, Umweltgifte, UV-Strahlung)
- Erzielen einer Resistenz gegen biotische Stressfaktoren (Pathogene, Viren, Insekten und Krankheiten)
- Verbesserung von Nahrungs- oder Futtereigenschaften
- Verbesserung der Wachstumsrate oder des Ertrages.

Nachfolgend seien einige konkrete Beispiele für Nukleinsäuren genannt, deren Expression die gewünschten vorteilhaften Effekte bietet:

### 1. Selektionsmarker

Selektionsmarker umfasst sowohl positive Selektionsmarker, die eine Resistenz gegen ein Antibiotikum, Herbizid oder Biozid verleihen, als auch negative Selektionsmarker, die eine Sensitivität gegen eben diese verleihen, als auch Marker die dem transformierten Organismus einen Wachstumsvorteil gewähren (beispielsweise durch Expression von Schlüsselgenen der Cytokinbiosynthese; Ebinuma H et al. (2000) Proc Natl Acad Sci USA 94:2117-2121), Bei der positiven Selektion gedeihen nur die Organismen, die den entsprechenden Selektionsmarker exprimieren, während bei der negativen Selektion eben diese eingehen. Bei der Herstellung transgener Pflanzen ist die Verwendung eines positiven Selektionsmarkers bevorzugt. Bevorzugt ist ferner die Verwendung von Selektionsmarkern, die Wachstumsvorteile verleihen. Negative Selektionsmarker können vorteilhaft verwendet werden, wenn es darum geht, bestimmte Gene oder Genomabschnitte aus einem Organismus zu entfernen (beispielsweise im Rahmen eines Kreuzungsprozesses).

Der mit der Expressionskassette eingebrachte selektionierbare Marker verleiht den erfolgreich rekombinierten oder transformierten Zellen eine Resistenz gegen ein Biozid (zum Beispiel ein Herbizid wie Phosphinothricin, Glyphosat oder Bromoxynil), einen Metabolismusinhibitor wie 2-Desoxyglucose-6-phosphat (WO 98/45456) oder ein Antibiotikum, wie zum Beispiel Kanamycin, G 418, Bleomycin, Hygromycin. Der Selektionsmarker erlaubt die Selektion der transformierten Zellen von untransformierten (McCormick et al., Plant Cell Reports 5 (1986), 81-84). Besonders bevorzugte Selektionsmarker sind solche die eine Resistenz gegen Herbizide verleihen. Beispielhaft als Selektionsmarker seien genannt:
Dem Fachmann sind zahlreiche derartiger Selektionsmarker und die für diese kodierenden Sequenzen bekannt. Nachfolgend seien beispielhaft jedoch nicht einschränkend zu nennen:

Dem Fachmann sind zahlreiche derartiger Selektionsmarker und die für diese kodierenden Sequenzen bekannt. Nachfolgen seien beispielshaft jedoch nicht einschränkend zu nennen:

### i) Positive Selektionsmarker;

Der mit derm Expressionskassette eingebrachte selektionierbaren Marker verleiht den erfolgreich rekombinierten oder transformierten Zellen eine Resistenz gegen ein Biozid (zum Beispiel ein Herbizid wie Phosphinothricin, Glyphosat oder Bromoxynil), einen Metabolismusinhibitor wie 2-Desoxyglucose-6-phosphat (WO 98/45456) oder ein Antibiotikum, wie zum Beispiel Tetracycline, Ampicillin, Kanamycin, G 418, Neomycin, Bleomycin oder Hygromycin, verleiht. Der Selektionsmarker erlaubt die Selektion der transformierten Zellen von untransformierten (McCormick et al., Plant Cell Reports 5 (1986), 81-84). Besonders bevorzugte Selektionsmarker sind solche die eine Resistenz gegen Herbizide verleihen. Beispielhaft als Selektionsmarker seien genannt:
- DNA Sequenzen, die für Phosphinothricinacetyltransferasen (PAT) kodieren, welche die freie Aminogruppe des Glutaminsynthaseinhibitors Phosphinothricin (PPT) acetylierent und damit eine Detoxifizierung des PPT erreichent (de Block et al. 1987, EMBO J. 6, 2513-2518) (auch Bialophosrr-Resistenzgen (bar) genannt). Das bar Gen kodierend für eine Phosphinothricinacetyltransferase (PAT) kann aus beispielsweise Streptomyces hygroscopicus oder S. viridochromogenes isoliert werden. Entsprechende Sequenzen sind dem Fachmann bekannt (aus Streptomyces hygroscopicus GenBank Acc.-No.: X17220 und X05822, aus Streptomyces viridochromogenes GenBank Acc.-No.: M 22827 und X65195; US 5,489,520). Ferner sind synthetische Gene beispielsweise für die Expression in Plastiden beschrieben AJ028212. Ein synthetisches Pat Gen ist beschrieben bei Becker et al. (1994), The Plant J. 5:299-307. Ganz besonders bevorzugt ist ebenfalls die Expression des Polypeptides gemäß SEQ ID NO: 5, beispielsweise kodiert durch eine Nukleinsäuresequenz gemäß SEQ ID NO: 4. Die Gene verleihen Resisteanz gegen das Herbizid Bialaphosr oder Glufosinat und sind ein vielbenutzter Marker in transgenen Pflanzen (Vickers , JE et al. (1996). Plant Mol. BMiol. Rreporter 14:363-368; Thompson CJ et al. (1987) EMBO Journal 6:2519-2523),
- 5-Enolpyruvylshikimat-3-phosphatsynthasegene (EPSP Synthasegene), die eine Resistenz gegen Glyphosatr (N-(phosphonomethyl)glycin) verleihen. Das unselektive Herbizid Glyphosat hat die 5-Enolpyruvyl-3-phosphoshikimatsynthase (EPSPS) als molekulares Target. Diese hat eine Schlüsselfunktion in der Biosynthese aromatischer Aminosäuren in Mikcroben und Pflanzen, jedoch nicht in Säugern (Steinrucken HC et al. (1980) Biochem. Biophys. Res. Commun. 94:1207-1212; Levin JG und. Sprinson DB (1964) J. Biol. Chem. 239: 1142-1150; Cole DJ (1985) Mode of action of glyphosate; Aa literature analysis, p. 48-74. In: Grossbard E und Atkinson D (eds.). The herbicide glyphosate. Buttersworths, Boston.). Glyphosat-tolerante EPSPS Varianten werden bevorzugt als Selektionsmarker verwendet (Padgette SR et al. (1996). New weed control opportunities: development of soybeans with a Roundup Buttersworths, Boston.). Glyphosat-tolerante EPSPS. Varianten werden bevorzugt als Selektionsmarker verwendet (Padgette SR et al. (1996). New weed control opportunities: development of soybeans with a Roundup Ready™ gene. In: Herbicide Resistant Crops (Duke, S.O., ed.), pp. 53-84. CRC Press, Boca Raton, FL; Saroha MK und Malik VS (1998) J Plant Biochemistry and Biotechnology 7:65-72). Das EPSPS Gen des Agrobacterium sp. strain CP4 hat eine natürliche Toleranz gegen Glyphosat, die auf entsprechende transgene Pflanzen transferiert werden kann. Das CP4 EPSPS Gen wurde aus Agrobacterium sp. strain CP4 kloniert (Padgette SR et al.(1995) Crop Science 35(5):1451-1461). 5-Enolpyrvylshikimate-3-phosphate-synthasen, die Glyphosat-tolerant sind, wie beispielsweise beschrieben in US 5,510,471; US 5,776,760; US 5,864,425; US 5,633,435; US 5,627;061; US 5,463,175; EP 0 218 571, sind bevorzugt, wobei die jeweils in den Patenten beschriebenen Sequenzen auch in der GenBank hinterlegt sind. Weitere Sequenzen sind beschrieben unter GenBank Accession X63374. Ferner ist das aroA Gen bevorzugt (M10947 S. typhimurium aroA locus 5-enolpyruvylshikimate-3-phosphate synthase (aroA protein) gene).
- das für das Glyphosat® degradierende Enzyme kodierende gox Gen (Glyphosatoxidoreduktase). GOX (beispielsweise die Glyphosatoxidoreductase aus Achromobacter sp.) katalysiert die Spaltung einer C-N Bindung inm Glyphosat, welchesas so zu Aminomethylphosphonsäure (AMPA) und Glyoxylat umgesetzt wird. GOX kann dadurch eine Resistenz gegen Glyphosat vermitteln (Padgette SR et al. (1996) J Nutr. 1996 Mar;126(3):702-16; Shah D et al. (1986) Science 233: 478-481).
- das deh Gen (kodierend für eine Dehalogenase, die Dalapon® inaktiviert), (GenBank Acc.-No.: AX022822, AX022820 sowie WO99/27116)
- bxn Gene, die für Bromoxynil® degradierende Nitrilaseenzyme kodieren. Beispielsweise die Nitrilase aus Klebsiella ozanenae. Sequenzen sind in der GgenBbank beispielsweise unter den Acc.-No: E01313 (DNA encoding bpromoxynil specific nitrilase) und J03196 (K. pneumoniae bromoxynil-specific nitrilase (bxn) gene, complete cds) zu finden.
   Sequenzen können aus der GenBank erhalten werden (AF080390 Minitransposon mTn5-GNm; AF080389 Minitransposon mTn5-Nm, complete sequence). Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden (AF234316 pCAMBIA-2301; AF234315 pCAMBIA-2300, AF234314 pCAMBIA-2201). Das NPTII Gen kodiert für eine Aminoglycosid-3'O-phosphotransferase aus E.coli, Tn5 (GenBank Acc.-No: U00004 Position 1401-2300; Beck et al. (1982) Gene 19 327-336).
- das DOG^{R}1-Gen. Das Gen DOG^{R}1 wurde aus der Hefe Saccharomyces cerevisiae isoliert (EP 0 807 836). Es codiert für eine 2-Desoxyglukose-6-phosphat Phosphatase, die Resistenz gegenüber 2-DOG verleiht (Randez-Gil et al. 1995, Yeast 11, 1233-1240; Sanz et al. (1994) Yeast 10:1195- 1202, Sequenz: GenBank Acc.-No.: NC001140 chromosom VIII, Saccharomyces cervisiae Position 194799-194056).
- Sulfonylharnstoff- und Imidazolinon inaktivierende Acetolactatsynthasen, die eine Resistenz gegen Imidazolinon/Sulfonylharnstoff-Herbizide verleihen. Beispielhaft seien für Imidazolinon-Herbizide die Wirkstoffe Imazamethabenz-methyl, Imazzamox, Imazapyr, Imazaquin, Imazethapyr zu nennen. Für Sulfonylharnstoff-Herbizide seien beispielhaft Amidosulforon, Azimsulfuron, Chlorimuronethyl, Chlorsulfuron, Cinosulfuron, Imazosulforon, Oxasulforon, Prosulforon, Rimsulforon, Sulfosulforon zu nennen. Dem Fachmann sind zahlreiche weitere Wirkstoffe der genannten Klassen bekannt. Geeignet sind beispielsweise die Nukleinsäuren unter der GenBank Acc-No.: X51514 hinterabgelegte Sequenz für das Arabidopsis thaliana Csr 1.2 Gen (EC 4.1.3.18) (Sathasivan K et al. (1990) Nucleic Acids Res. 18(8):2188). Acetolactatesynthasen die eine Resistenz gegen Imidazolinon-Herbizide verleihen sind ferner beschrieben unter den GenBank Acc.-No.:
   a) AB049823 Oryza sativa ALS mRNA for acetolactate synthase, complete cds, herbicide resistant biotype
   b) AF094326 Bassia scoparia herbicide resistant acetolactate synthase precursor (ALS) gene, complete cds
   c) X07645 Tobacco acetolactate synthase gene, ALS SuRB (EC 4.1.3.18)
   d) X07644 Tobacco acetolactate synthase gene, ALS SuRA (EC 4.1.3.18)
   e) A19547 Synthetic nucleotide mutant acetolactate synthase
   f) A19546 Synthetic nucleotide mutant acetolactate synthase
   g) A19545 Synthetic nucleotide mutant acetolactate synthase
   h) I05376 Sequence 5 from Patent EP 0257993
   i) I05373 Sequence 2 from Patent EP 0257993
   j) AL133315

   Bevorzugt ist die Expression einer Acetolactatsynthase gemäß SEQ ID NO: 7, beispielsweisae kodiert durch eine Nukleinsäuresequenz gemäß SEQ ID NO: 6.
- Hygromycinphosphotransferasen (X74325 P. pseudomallei gene for hygromycin phosphotransferase) die eine Resistenz gegen das Antibiotikum Hygromycin verleihen. Das Gen ist Bestandteil zahlreicher Expressionsvektoren und kann unter Verwendung von dem Fachmann geläufigen Verfahren (wie beispielsweise Polymerasekettenreaktion) aus diesen isoliert werden (AF294981 pINDEX4; AF234301 pCAMBIA-1380; AF234300 pCAMBIA-1304; AF234299 pCAMBIA-1303; AF234298 pCAMBIA-1302; AF354046 pCAMBIA-1305.; AF354045 pCAMBIA-1305.1)
- Resistenzgene gegen
   a) Chloramphenicol (Chloramphenicolacetyltransferase),
   b) Tetracyclin, verschiedene Resistenzgene sind beschrieben z.B. X65876 S. ordonez genes class D tetA and tetR for tetracycline resistance and repressor proteins X51366 Bacillus cereus plasmid pBC16 tetracycline resistance gene. Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden
   c) Streptomycin, verschiedene Resistenzgene sind beschrieben z.B. mit der GenBank Acc.-No.:AJ278607 Corynebacterium acetoacidophilum ant gene for streptomycin adenylyltransferase.
   d) Zeocin, das entsprechende Resistenzgen ist Bestandteil zahlreicher Klonierungsvektoren (z.B. L36849 Cloning vector pZEO) und kann unter Verwendung von dem Fachmann geläufigen Verfahren (wie beispielsweise Polymerasekettenreaktion) aus diesen isoliert werden.
   e) Ampicillin (β-Lactamase Gen; Datta N, Richmond MH.(1966) Biochem J. 98(1):204-9; Heffron F et al (1975) J. Bacteriol 122: 250-256; das Amp Gen wurde zuerst zur Herstellung des E. coli Vektors pBR322 kloniert; Bolivar F et al. (1977) Gene 2:95-114). Die Sequenz ist Bestandteil zahlreicher Klonierungsvektoren und kann unter Verwendung von dem Fachmann geläufigen Verfahren (wie beispielsweise Polymerasekettenreaktion) aus diesen isoliert werden.
- Gene wie die Isopentenyltransferase aus Agrobacterium tumefaciens (strain:P022) (Genbank Acc.-No.: AB025109). Das ipt Gen ist ein Schlüsselenzym der CytokinBiosynthese. Seine Überexpression erleichtert die Regeneration von Pflanzen (z.B. Selektion auf Cytokinfreienm Medium). Das Verfahren zur Nutzung des ipt Gens ist beschrieben (Ebinuma H et al. (2000) Proc Natl Acad Sci USA 94:2117-2121; Ebinuma, H et al. (2000) Selection of Marker-free transgenic plants using the oncogenes (ipt, rol A, B, C) of Agrobacterium as selectable markers, In Molecular Biology of Woody Plants. Kluwer Academic Publishers).

Verschiedene weitere positive Selektionsmarker, die den transformierten Pflanzen einen Wachstumsvorteil gegenüber nicht-transformierten verleihen, sowie Verfahren zu ihrer Verwendung sind u.a. beschrieben in EP-A 0 601 092. Beispielhaft sind zu nennen β-Glucuronidase (in Verbindung mit z.B. Cytokininglucuronid), Mannose-6-phosphat-Isomerase (in Verbindung mit Mannose), UDP-Galaktose-4-Epimerase (in Verbindung mit z.B. Galactose), wobei Mannose-6-phosphat-Isomerase in Verbindung mit Mannose besonders bevorzugt ist.

### ii) Negative Selektionsmarker

Negative Selektionsmarker ermöglichen beispielsweise die Selektion von Organismen mit erfolgreich deletierten Sequenzen, die das Markergen umfassen (Koprek T et al. (1999) The Plant Journal 19(6):719-726) Bei der negativen Selektion wird beispielsweise durch den in die Pflanze eingebrachten negativen Selektionsmarker eine Verbindung, die ansonsten für die Pflanze keine nachteilige Wirkung hat, in eine Verbindung mit nachteiliger Wirkung umgesetzt. Ferner sind Gene geeignet, die per se eine nachteilige Wirkung haben, wie zum Beispiel TK thymidine kinase (TK), and Ddiphtheria Ttoxin A Ffragment (DT-A), das codA Genprodukt kodierend für eine Cytosindeaminase (Gleave AP et al. (1999) Plant Mol Biol. 40(2):223-35; Perera RJ et al. (1993) Plant Mol. Biol 23(4): 793-799; Stougaard J; (1993) Plant J 3:755-761), das Cytochrom P450 Gen (Koprek et al. (1999) Plant J. 16:719-726), Gene kodierend für eine Haloalkan Dehalogenase (Naested H (1999) Plant J. 18:571-576), das iaaH Gen (Sundaresan V et al. (1995) Genes & Development 9:1797-1810) oder das tms2 Gen (Fedoroff NV & Smith DL 1993, Plant J 3: 273-289).

Die jeweils für die Selektion verwendeten Konzentrationen der Antibiotika, Herbizide, Biozide oder Toxine müssen an die jeweiligen Testbedingungen bzw. Organismen angepasst werden. Beispielhaft seien für Pflanzen zu nennen Kanamycin (Km) 50 mg/l, Hygromycin B 40 mg/l, Phosphinothricin (Ppt) 6 mg/l.

Ferner können funktionelle Analoga der genannten Nukleinsäuren kodierend für Selektionsmarker exprimiert werden. Funktionelle Analoga meint hier all die Sequenzen, die im wesentlichen die gleiche Funktion haben d.h. zu einer Selektion transformierter Organismen befähigt sind. Dabei kann das funktionelle Analogon sich in anderen Merkmalen durchaus unterscheiden. Es kann zum Beispiel eine höhere oder niedrigere Aktivität haben oder auch über weitere Funktionalitäten verfügen.

2. Verbesserter Schutz der Pflanze gegen abiotische Stxessfaktoren wie Trockenheit, Hitze, oder Kälte zum Beispiel durch Überexpression von dem "antifreeze"-pPolypeptidens aus Myoxocephalus Scorpius (WO 00/00512), Myoxocephalus octodecemspinosus, dem Arabidopsis thaliana Transkriptionsaktivator CBF1, Glutamatdehydrogenasen (WO 97/12983, WO 98/11240), Calcium-abhängigen Proteinkinasegenen (WO 98/26045), Calcineurinen (WO 99/05902), Farnesyltransferasen (WO 99/06580), Pei ZM et al., Science 1998, 282: 287-290), Ferritin (Deak M et al., Nature Biotechnology 1999, 17:192-196), Oxalatoxidase (WO 99/04013; Dunwell JM Biotechnology and Genetic Engeneering Reviews 1998, 15:1-32), DREBIA-Faktor (dehydration response element B 1A; Kasuga M et al., Nature Biotechnology 1999, 17:276-286), Genen der Mannitol- oder Trehalosesynthese wie der Trehalosephosphatsynthase oder der Trehalosephosphatphosphatase (WO 97/42326), oder durch Inhibition von Genen wie der Trehalase (WO 97/S0561). Besonders bevorzugt sind Nukleinsäuren, die für den transkriptionellen Aktivator CBF1 aus Arabidopsis thaliana (GenBank Acc.-No.: U77378) oder das "antifreeze protein" aus Myoxocephalus octodecemspinosus (GenBank Acc.-No.: AF306348) oder funktionelle Äquivalente derselben kodieren.

3. Expression von Stoffwechselenzymen zur Verwendung im Futter- und Nahrungsmittelbereich, zum Beispiel die Expression von Phytase und Cellulasen. Besonders bevorzugt sind Nukleinsäuren wie die künstliche für eine mikrobielle Phytase kodierende cDNA (GenBank Acc.-No.: A19451) oder funktionelle Äquivalente derselben.

4. Erreichen einer Resistenz zum Beispiel gegen Pilze, Insekten, Nematoden und Krankheiten durch gezielte Absonderung oder Anreicherung bestimmter Metaboliten oder Proteine in der Epidermis des Embryos. Beispielhaft seien genannt Glucosinolate (Abwehr von Herbivoren), Chitinasen oder Glucanasen und andere Enzyme die die Zellwand von Parasiten zerstören, Ribosom-inaktivierende Proteine (RIPs) und andere Proteine der pflanzlichen Resistenz- und Stressreaktion, wie sie bei Verletzung oder mikrobiellenm Befall von Pflanzen oder chemisch durch zum Beispiel Salicylsäure, Jasmonsäure oder Ethylen induziert werden, Lysozyme aus nichtpflanzlichen Quellen wie zum Beispiel T4 Lysozym oder Lysozm aus verschiedenen Säugern, insektizide Proteine wie *Bacillus thuringiensis* Endotoxin, α-Amylaseinhibitor oder Proteaseinhibitoren (cowpea Trypsininhibitor), Glucanasen, Lectine wie Phytohemagglutinin, Schneeglöckchenlectin, Weizenkeimagglutinin, RNAsen oder Ribozyme. Besonders bevorzugt sind Nukleinsäuren, die für die chit42 Endochitinase aus Trichoderma harzianum (GenBank Acc.-No.: S78423) oder für das N-hydroxylierende, multifunktionelle Cytochrom P-450 (CYP79) Proteine aus Sorghum bicolor (GenBank Acc.-No.: U32624) oder funktionelle Aquivalente derselben kodieren.

Bekannt ist die Akkumulation von Glucosinolaten in Pflanzen der Gattung der Cardales insbesondere der Ölsaaten zum Schutz vor Schädlingen (Rask L et al. (2000) Plant Mol Biol 42:93-113; Menard R et al. (1999) Phytochemistry 52:29-35), die Expression des *Bacillus thuringiensis* Endotoxin unter Kontrolle des 35 S CaMV Promotors (Vaeck et al. (1987) Nature 328:33-37) oder der Schutz des Tabaks gegen Pilzbefall durch Expression einer Chitinase aus der Bohne unter Kontrolle des CaMV Promotors (Broglie et al. (1991) Science 254:1194-1197).

Durch Expression des Lectins Agglutinin aus Schneeglöckchen (*Galanthus nivalis*) kann eine Resistenz gegen Schädlinge wie den Reisschädling *Nilaparvata lugens* beispielsweise in transgenen Reispflanzen erreicht werden (Rao et al. (1998) Plant J. 15(4):469-77.). *Nilaparvata lugens* zählt zu den Phloem-saugenden Schädlingen und fungiert darüberhinaus als Überträger bedeutender Virus-bedingter Pflanzenkrankheiten.

Die Expression synthetischer cryIA(b) and cryIA(c) Gene, die für Lepidopteren-spezifische delta-Endotoxine aus Bacillus *thuringiensis* kodieren, kann in verschiedenen Pflanzen eine Resistenz gegen Schadinsekten bewirken. So kann in Reis eine Resistenz gegen zwei der wichtigsten Reisschädlinge, den gestreiften Stengelbohrer (*Chilo suppressalis*) und den gelben Stengelbohrer (*Scirpophaga incertulas*)*,* erzielt werden (Cheng X et al. (1998) Proc Natl Acad Sci USA 95(6):2767-2772; Nayak P et al. (1997) Proc Natl Acad Sci USA 94(6):2111-2116).

5. Expression von Genen, die eine Akkumulation von Feinchemikalien, wie von Tocopherolen, Tocotrienolen oder Carotinoiden bewirken. Beispielhaft sei die Phytoendesaturase genannt. Bevorzugt sind Nukleinsäuren, die für die Phytoendesaturase aus Narcissus pseudonarcissus (GenBank Acc.-No.: X78815) oder funktionelle Äquivalente derselben kodieren.

6. Produktion von Neutraceuticals wie zum Beispiel polyungesättigten Fettsäuren wie beispielsweise Arachidonsäure oder EP (Eicosapentaensäure) oder DHA (Docosahexaensäure) durch Expression von Fettsäureelongasen und/oder -desaturasen oder Produktion von Proteinen mit verbessertem Nahrungswert wie zum Beispiel mit einem hohen Anteil an essentiellen Aminosäuren (z.B. das methioninreiche 2S Albumingens der Brasilnuss). Bevorzugt sind Nukleinsäuren, die für das methioninreiche 2S Albumin aus Bertholletia excelsa (GenBank Acc.-No.:AB044391), die Δ6-Acyllipiddesaturase aus Physcomitrella patens (GenBank Acc.-No.: AJ222980; Girke et al 1998, The Plant Journal 15:39-48), die Δ6-Desaturase aus Mortierella alpina (Sakuradani et al 1999 Gene 238:445-453), die Δ5-Desaturase aus Caenorhabditis elegans (Michaelson et al. 1998, FEBS Letters 439:215-218), die Δ5-Fettsäuredesaturase (des-5) aus Caenorhabditis elegans (GenBank Acc.-No.: AF078796), die Δ5-Desaturase aus Mortierella alpina (Michaelson et al. JBC 273:19055 - 19059), die Δ6-Elongase aus Caenorhabditis elegans (Beaudoin et al. 2000, PNAS 97:6421-6426), die Δ6-Elongase aus Physcomitrella patens (Zank et al. 2000, Biochemical Society Transactions 28:654-657) oder funktionelle Äquivalente derselben kodieren.

7. Produktion von Feinchemikalien (wie beispielsweise Enzymen) und Pharmazeutika (wie zum Beispiel Antikörpern oder Vakzinen wie beschrieben bei Hood EE, Jilka JM. (1999) Curr Opin Biotechnol. 10(4):382-6; Ma JK, Vine ND (1999) Curr Top Microbiol Immunol 236:275-92). Beispielsweise konnte rekombinantes Avidin aus Hühnereiweiß und bakterieller β-Glucuronidase (GUS) in großen Maßstab in transgenen Maispflanzen produziert werden (Hood et al. (1999) Adv Exp Med Biol 464;127-47. Review.). Diese rekombinanten Proteine aus Maispflanzen werden von der Firma Sigma (Sigma Chemicals Co.) als hochreine Biochemikalien vertrieben.

8. Erreichen einer erhöhten Speicherfähigkeit in Zellen, die normalerweise weniger Speicherproteine oder -lipide enthalten mit dem Ziel, den Ertrag an diesen Substanzen zu erhöhen, zum Beispiel durch Expression eine Acetyl-CoA Carboxylase. Bevorzugt sind Nukleinsäuren, die für die Acetyl-CoA Carboxylase (Accase) aus Medicago sativa (GenBank Acc.-No.: L25042) oder funktionelle Äquivalente derselben kodieren.

Weitere Beispiele für vorteilhafte Gene sind zum Beispiel genannt bei Dunwell JM, Transgenic approaches to crop improvement, J Exp Bot. 2000;51 Spec No; Seite 487-96.

Ferner können funktionelle Analoga der genannten Nukleinsäuren bzw. Proteine exprimiert werden. Funktionelle Analoga meint hier all die Sequenzen, die im wesentlichen die gleiche Funktion haben d.h. zu der Funktion (zum Beispiel einer Substratumsetzung oder einer Signaltransduktion) befähigt sind wie auch das beispielhaft genannte Protein. Dabei kann das funktionelle Analogon sich in anderen Merkmalen durchaus unterscheiden. Es kann zum Beispiel eine höhere oder niedrigere Aktivität haben oder auch über weitere Funktionalitäten verfügen. Funktionelle Analoga meint ferner Sequenzen, die für Fusionsproteine bestehend aus einem der bevorzugten Proteine und anderen Proteinen zum Beispiel einem weiteren bevorzugten Protein oder aber auch einer Signalpeptidsequenz kodieren.

Die Expression der Nukleinsäuren unter Kontrolle der Erfindungsgemäßen Promotoren ist in jedem gewünschten Zellkompartiment, wie z.B, dem Endomembransystem, der Vakuole und den Chloroplasten möglich. Durch Nutzung des sekretorischen Weges sind gewünschte Glykosylierungsreaktionen, besondere Faltungen u.ä. möglich. Auch die Sekretion des Zielproteins zur Zelloberfläche bzw. die Sezernierung ins Kulturmedium, beispielsweise bei Nutzung suspensionskultivierter Zellen oder Protoplasten ist möglich. Die dafür notwendigen Targetsequenzen können sowohl in einzelnen Vektorvariationen berücksichtigt werden als auch durch Verwendung einer geeigneten Klonierungsstrategie gemeinsam mit dem zu klonierenden Zielgen in den Vektor mit eingebracht werden. Als Targetsequenzen können sowohl gGen eigene, sofern vorhanden, oder heterologe Sequenzen genutzt werden. Zusätzliche, heterologe zur funktionellen Verknüpfung bevorzugte aber nicht darauf beschränkte Sequenzen sind weitere Targeting-Sequenzen zur Gewährleistung der subzellulären Lokalisation im Apoplasten, in der Vakuole, in Plastiden, imn Mitochondrieum, im Endoplasmatischen Retikulum (ER), im Zellkern, in Ölkörperchen oder anderen Kompartimenten; sowie Translationsverstärker wie die 5'-Leadersequenz aus dem Tabak-Mosaik-Virus (Gallie et al., Nucl. Acids Res. 15 (1987), 8693-8711) und dergleichen. Das Verfahren, an sich nicht in den Plastiden lokalisierte Proteine, gezielt in die Plastiden zu transportieren, ist beschrieben (Klosgen RB und Weil JH (1991) Mol Gen Genet 225(2):297-304; Van Breusegem F et al. (1998) Plant Mol Biol. 38(3):491-496). Bevorzugte Sequenzen sind:
a) kleine Untereinheit (SSU) der Ribulosebisphosphatcarboxylase (Rubisco ssu) aus Erbse, Mais, Sonnenblume
b) Transitpeptide abgeleitet von Genen der pflanzlichen Fettsäurebiosynthese wie das Transitpeptid des plastidären "Acyl Carrier Protein" (ACP), die Stearyl-ACP-Desaturase, β-Ketoacyl-ACP Synthase oder die Acyl-ACP-Thioesterase.
c) das Transitpeptid für GBSSI ("Starch Granule Bound Starch Synthase I")
d) LHCP II Gene.

Die Zielsequenzen können mit anderen, von dem Transitpeptid kodierenden Sequenzen verschiedenen, Targeting-Sequenzen verknüpft sein, um eine subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten zu gewährleisten. Ferner können sowie Translationsverstärker wie die 5'-Leadersequenz aus dem Tabak-Mosaik-Virus (Gallie et al. (1987), Nucl. Acids Res. 15: (1987), 8693-8711) und dergleichen zum Einsatz kommen.

Dem Fachmann ist ferner bekannt, dass er die oben beschriebenen Gene nicht direkt unter Verwendung der für diese Gene kodierenden Nukleinsäuresequenzen exprimieren oder zum Beispiel durch anti-sense reprimieren muss. Er kann auch zum Beispiel künstliche Transkriptionsfaktoren vom Typ der Zinkfingerproteine verwenden (Beerli RR et al. (2000) Proc Natl Acad Sci USA 97(4):1495-500). Diese Faktoren lagern sich in den regulatorischen Bereichen der zu exprimierenden oder zu reprimierenden endogenen Gene an und bewirken, je nach Gestaltung des Faktors, eine Expression oder Repression des endogenen Gens. So kann man die gewünschten Effekte auch durch Expression eines entsprechenden Zinkfinger-Transkriptionsfaktors unter Kontrolle eines der erfindungsgemäßen Promotoren erreichen.

Die erfindungsgemäßen Expressionskassetten können ebenso zur Unterdrückung bzw. Reduktion von Replikation oder/und Translation von Targetgenen durch "gene silencing" eingesetzt werden.

Die erfindungsgemäßen Expressionskassetten können auch eingesetzt werden, um Nukleinsäuren zu exprimieren, die sogenannte "antisense" Effekte vermitteln und so beispielsweise zur Verminderung der Expression eines Zielproteins befähigt sind.

Bevorzugte Gene bzw. Proteine, deren Suppression einen vorteilhaften Phänotyp bedingt, umfassen beispielshaft, aber nicht einschränkend:
a) Polygalakturonase zur Verhinderung von Zellabbau und "Matschig"-werden von Pflanzen und Früchten beispielsweise Tomaten. Bevorzugt werden dazu Nukleinsäuresequenzen wie die des Polygalakturonase-Gens der Tomate (GenBank Acc.-No.: X14074) oder dessen Homologe aus anderen Gattungen und Arten verwendet.
b) Verminderung der Expression von allergenen Proteinen wie beispielsweise beschrieben bei Tada Y et al. (1996) FEBS Lett 391(3):341-345 oder Nakamura R (1996) Biosci Biotechnol Biochem 60(8) :1215-1221ö.
c) Veränderung der Blütenfarbe durch Suppression der Expression von Enzymen der Anthocyanbiosynthese. Entsprechende vorgehensweisen sind beschrieben (beispielsweise bei Forkmann G, Martens S. (2001) Curr Opin Biotechnol 12(2):155-160). Bevorzugt werden dazu Nukleinsäuresequenzen wie die der Flavonoid-3'-hydroxyLase (GenBank Acc.-No.: AB045593), der Dihydroflavanol-4-reduktase (GenBank Acc.-No.: AF017451), der Chalconisomerase (GenBank Acc.-No.: AF276302), der Chalconsynthase (GenBank Acc.-No.: AB061022), der Flavanone-3-beta-hydroxylase (GenBank Acc.-No.: X72592) oder der Flavonesynthase II (GenBank Acc.-No.: AB045592) oder derssen Homologe aus anderen Gattungen und Arten verwendet.
d) Verschiebung des Amylose/Amylopektingehaltes in Stärke durch Suppression des Verzweigungsenzyms Q, das für die α-1,6-glykosidische Verknüpfung verantwortlich ist. Entsprechende Vorgehensweisen sind beschrieben (beispielsweise bei Schwall GP et al. (2000) Nat Biotechnol 18(5):551-554). Bevorzugt werden dazu Nukleinsäuresequenzen wie die des Starch branching enzyme II der Kartoffel (GenBank Acc.-No.: AR123356; US 6,169,226) oder dessen Homologe aus anderen Gattungen und Arten verwendet.

Eine "antisense" Nukleinsäure meint zunächst eine Nukleinsäuresequenz die ganz oder teilweise zu zumindest einem Teil des "sense"-Stranges besagten Zielproteins komplementär ist. Dem Fachmann ist bekannt, dass er alternativ diee cDNA oder das korrespondierendes Gen als Ausgangsmatrize für entsprechende antisense-Konstrukte verwenden kann. Bevorzugt ist die "antisense" Nukleinsäure komplementär zu dem kodierenden Bereich des Zielproteins oder einem Teil desselben. Die "antisense" Nukleinsäure kann aber auch zu der nicht-kodierenden Region oder einem Teil derselben komplementär sein. Ausgehend von der Sequenzinformation zu einem Zielprotein, kann eine antisense Nukleinsäure unter Berücksichtigung der Basenpaarregeln von Watson und Crick in der dem Fachmann geläufigen Weise entworfen werden. Eine antisense Nukleinsäure kann komplementär zu der gesamten oder einem Teil der Nukleinsäuresequenz eines Zielproteins sein. In einer bevorzugten Ausführungsform ist die antisense Nukleinsäure ein Oligonukleotid mit einer Länge von zum Beispiel 15, 20, 25, 30, 35, 40, 45 oder 50 Nukleotiden.

Die antisense Nukleinsäure umfasst in einer bevorzugten Ausführungsform α-anomere Nukleinsäuremoleküle. α-Anomere Nukleinsäuremoleküle bilden besondere doppelsträngige Hybride mit komplementärer RNA in denen im Unterschied zu den normalen β-Einheiten die Stränge parallel zu einander verlaufen (Gaultier et al. (1987) Nucleic Acids. Res. 15:6625-6641).

Ebenso umfasst ist die Verwendung der oben beschriebenen Sequenzen in sense-Orientierung, was wie dem Fachmann geläufig ist, zu einer Kosuppression führen kann. In Tabak, Tomate und Petunie ist demonstriert worden, dass die Expression von sense-RNA zu einem endogenen Gen, die Expression desselben vermindern oder ausschalten kann, ähnlich wie es für antisense Ansätze beschrieben wurde (Goring et al. (1991) Proc. Natl Acad Sci USA, 88:1770-1774; Smith et al. (1990) Mol Gen Genet 224:447-481; Napoli et al. (1990) Plant Cell 2;279-289; Van der Krol et al. (1990) Plant Cell 2:291-299). Dabei kann das eingeführte Konstrukt das zu vermindernde Gen ganz oder nur teilweise repräsentieren. Die Möglichkeit zur Translation ist nicht erforderlich.

Ganz besonders bevorzugt ist auch die Verwendung von Verfahren wie der Genregulation mittels doppelsträngiger RNA ("doublestranded RNA interference"). Entsprechende Verfahren sind dem Fachmann bekannt und im Detail beschrieben (z.B. Matzke MA et al. (2000) Plant Mol Biol 43:401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Hier wird durch gleichzeitige Einbringung von Strang- und Gegenstrang eine hocheffiziente Unterdrückung nativer Gene bewirkt.

Vorteilhaft kann die antisense-Strategie mit einem Ribozym-Verfahren gekoppelt werden. Ribozyme sind katalytisch aktive RNA Sequenzen, die gekoppelt an die antisense Sequenzen, die Zielsequenzen katalytisch spalten (Tanner NK. FEMS Microbiol Rev. 1999; 23 (3) :257-75) . Dies kann die Effizienz einer anti-sense Strategie erhöhen. Die Expression von Ribozymen zur Verminderung bestimmter Proteine ist dem Fachmann bekannt und beispielsweise beschrieben in EP-A1 0 291 533, EP-A1 0 321 201 und EP-A1 0 360 257. Geeignete Zielsequenzen und Ribozyme können zum Beispiel wie bei Steinecke (Ribozymes, Methods in Cell Biology 50, Galbraith et al eds Academic Press, Inc. (1995), 449-460) beschrieben, durch Sekundärstrukturberechnungen von Ribozym- und Ziel-RNA sowie durch deren Interaktion bestimmt werden (Bayley CC et al., Plant Mol Biol. 1992; 18(2):353-361; Lloyd AM and Davis RW et al., Mol Gen Genet. 1994 Mar;242(6):653-657). Beispielhaft sind "hammerhead"-Ribozyme zu nennen (Haselhoff and Gerlach (1988) Nature 334:585-591). Bevorzugte Ribozyme basieren auf Derivaten der Tetrahymena L-19 IVS RNA (US 4,987,071; US 5,116,742). Weitere Ribozyme mit Selektivität für eine L119 mRNA können selektioniert werden (Bartel D und Szostak JW (1993) Science 261:1411-1418).

In einer weiteren Ausführungsform kann Verminderung der Zielprotein-Expression unter Verwendung von Nukleinäsäuresequenzen bewirkt werden, die komplementär zu regulativen Elementen der Zielprotein-Gene sind, mit diesen eine triple-helikale Struktur ausbilden und so die Gen-Transkription verhindern (Helene C (1991) Anticancer Drug Des. 6(6):569-84; Helene C et al. (1992) Ann NY Acad Sei 660:27-36; Maher LJ (1992) Bioassays 14(12):807-815).

Die erfindungsgemässgemäßen Expressionskassetten und die von ihnen abgeleiteten Vektoren können weitere Funktionselemente enthalten.

Der Begriff Funktionselement ist breit zu verstehen und meint all solche Elemente, die einen Einfluss auf Herstellung, Vermehrung oder Funktion der erfindungsgemässgemäßen Expressionskaoassetten oder von diesen abgeleitete Vektoren oder Organismen haben. Beispielhaft aber nicht einschränkend seien zu nennen:
a) Reportergene, die für leicht quantifizierbare Proteine kodieren und über Eigenfarbe oder Enzymaktivität eine Bewertung der Transformationseffizienz, des Expressionsortes oder -zeitpunktes gewährleisten. Ganz besonders bevorzugt sind dabei Gene kodierend für Reporter-Proteine (siehe auch Schenborn E, Groskreutz D. Mol Biotechnol. 1999; 13(1):29-44) wie
   - "green fluorescence protein" (GFP) (Chui WL et al., Curr Biol 1996, 6:325-330; Leffel SM et al., Biotechniques. 23(5):912-8, 1997; Sheen et al.(1995) Plant Journal 8(5):777-784; Haseloff et al.(1997) Proc Natl Acad Sci USA 94(6);2122-2127; Reichel et al.(1996) Proc Natl Acad Sci USA 93(12):5888-5893; Tian et al. (1997) Plant Cell Rep 16:267-271; WO 97/41228).
   - Chloramphenicoltransferase (Fromm et al. (1985) Proc. Natl. Acad. Sci. USA 82:5824-5828),
   - Luziferase (Millar et al., Plant Mol Biol Rep 1992 10:324-414; Ow et al. (1986) Science, 234:856-859); erlaubt Bioluminescenzdetektion.
   - β-Galactosidase, kodiert für ein Enzym für das verschiedenen chromogene Substrate zur Verfügung stehen,
   - β-Glucuronidase (GUS) (Jefferson et al., EMBO J. 1987, 6, 3901-3907) oder das uidA Gen, das ein Enzym für verschiedene chromogene Substrate kodiert.
   - R-Locus Genprodukt:Protein, das die Produktion von Anthocyaninpigmenten (rote Färbung) in pflanzlichen Gewebe reguliert und so eine direkte Analyse der Promotoraktivität ohne Zugabe zusätzlicher Hilfsstoffe oder chromogener Substrate ermöglicht (Dellaporta et al., In: Chromosome Structure and Function: Impact of New Concepts, 18th Stadler Genetics Symposium, 11:263-282, 1988).
   - β-Lactamase (Sutcliffe (1978) Proc Natl Acad Sci USA 75:3737-3741), Enzym für verschiedene chromogene Substrate (z.B. PADAC, eine chromogenes Cephalosporin).
   - xylE Genprodukt (Zukowsky et al. (1983) Proc Natl Acad Sci USA 80:1101-1105), Catecholdioxygenase, die chromogene Catechole umsetzen kann.
   - Alpha-Amylase (Ikuta et al. (1990) Bio/technol. 8:241-242).
   - Tyrosinase (Katz et al.(1983) J Gen Microbiol 129:2703-2714), Enzym, das Tyrosin zu DOPA und Dopaquinon oxidiert, die infolge das leicht nachweisbare Melanin bilden.
   - Aequorin (Prasher et al.(1985) Biochem Biophys Res Commun 126(3):1259-1268), kann in der Calcium-sensitiven Bioluminescenzdetektion verwendet werden.
b) Replikationsursprünge, die eine Vermehrung der erfindungsgemässgemäßen Expressionskassetten oder Vektoren in zum Beispiel E. coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
c) Elemente zum Beispiel "Bordersequenzen", die einen Agrobakterien-vermittelten Transfer in Pflanzenzellen für die Übertragung und Integration ins Pflanzengenom ermöglichen, wie zum Beispiel die rechte oder linke Begrenzung der T-DNA oder die vir-Region.
d) Multiple Klonierungsregionen (MCS) erlauben und erleichtern die Insertion eines oder mehrerer Nukleinsäuresequenzen.

Dem Fachmann sind verschiedene Wege bekannt, um zu einer erfindungsgemäßen Expressionskassette zu gelangen. Die Herstellung einer erfindungsgemäßen Expressionskassette erfolgt beispielsweise durch Fusion eines der erfindungsgemäßen Promotoren (oder erfindungsgemäßen Äquivalenten mit einer zu exprimierenden Nukleotidsequenz, gegebenenfalls einer für eine Transitpeptid kodierenden Sequenz, vorzugsweise ein chloroplastenspezifisches Transitpeptid, welches vorzugsweise zwischen dem Promotor und der jeweiligen Nukleotidsequenz angeordnet ist, sowie einem Terminator- oder Polyadenylierungssignal. Dazu verwendet man gängige Rekombinations- und Klonierungstechniken, wie sie beispielsweise in T. Maniatis T, E.F. Fritsch EF und J. Sambrook J, Molecular Cloning: A Laboratory Manual, Cold Spring Harbor Laboratory, Cold Spring Harbor, NY (1989) sowie in T.J. Silhavy TJ, M.L. Berman ML und L.W. Enquist LW, Experiments with Gene Fusions, Cold Spring Harbor Laboratory, Cold Spring Harbor, NY (1984) und in Ausubel, F.M. et al.,(1987) Current Protocols in Molecular Biology, Greene Publishing Assoc. and Wiley Interscience (1987) beschrieben sind.

Unter einer Expressionskassette sind aber auch solche Konstruktionen zu verstehen, bei denen der Promotor, ohne dass er zuvor mit einer zu exprimierenden Nukleinsäuresequenz funktionell verknüpft wurde, zum Beispiel über eine gezielte homologe Rekombination oder eine zufällige Insertion in ein Wirtsgenom eingeführt wird, dort regulatorische Kontrolle über mit ihm dann funktionell verknüpfte Nukleinsäuresequenzen übernimmt und die transgene Expression derselben steuert. Durch Insertion des Promotors - zum Beispiel durch eine homologe Rekombination - vor eine für ein bestimmtes Polypeptid kodierende Nukleinsäure erhält man eine erfindungsgemässgemäße Expressionskassette, die die Expression des bestimmten Polypeptides in der Pflanze steuert. Ferner kann die Insertion des Promotors auch derart erfolgen, dass antisense-RNA zu der für ein bestimmtes Polypeptid kodierenden Nukleinsäure exprimiert wird. Damit wird die Expression des bestimmten Polypeptides in Pflanzen herunterreguliert oder ausgeschaltet.

Analog kann auch eine transgen zu exprimierende Nukleinsäuresequenz zum Beispiel durch eine homologe Rekombination hinter den endogenen, natürlichen Promotor plaziert werden, wodurch man eine erfindungsgemässgemäße Expressionskassette erhält, die die Expression der transgen zu exprimierenden Nukleinsäuresequenz in den Keimblättern des pflanzlichen Embryos steuert.

Erfindungsgemässgemäß sind ferner Vektoren, die die oben beschriebenen Expressionskassetten enthalten. Vektoren können beispielhaft Plasmide, Cosmide, Phagen, Viren oder auch Agrobakterien sein.

Ein anderer Gegenstand der Erfindung betrifft transgene Organismen, transformiert mit wenigstens einer erfindungsgemässgemäßen Expressionskassette oder einem erfindungsgemässgemäßen Vektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen.

Unter Organismus, Ausgangs- oder Wirtsorganismen werden prokaryotische oder eukaryotische Organismen, wie beispielsweise Mikroorganismen oder pflanzliche Organismen verstanden. Bevorzugte Mikroorganismen sind Bakterien, Hefen, Algen oder Pilze.

Bevorzugte Bakterien sind Bakterien der Gattung Escherichia, Erwinia, Agrobacterium, Flavobacterium, Alcaligenes oder Cyanobakterien zum Beispiel der Gattung Synechocystis.

Bevorzugt sind vor allem Mikroorganismen, welche zur Infektion von Pflanzen und damit zur Übertragung der erfindungsgemässgemäßen Kassetten befähigt sind. Bevorzugte Mikroorganismen sind solche aus der Gattung Agrobakterium und insbesondere der Art Agrobakterium tumefaciens.

Bevorzugte Hefen sind Candida, Saccharomyces, Hansenula oder Pichia.

Bevorzugte Pilze sind Aspergillus, Trichoderma, Ashbya, Neurospora, Fusarium, Beauveria oder weitere in Indian Chem Engr. Section B. Vol 37, No 1,2 (1995) auf Seite 15, Tabelle 6 beschriebene Pilze.

Als transgene Organismen bevorzugte Wirts- oder Ausgangsorganismen sind vor allem Pflanzen. Eingeschlossen sind im Rahmen der Erfindung alle Gattungen und Arten höherer und niedrigerer Pflanzen des Pflanzenreiches. Eingeschlossen sind ferner die reifen Pflanzen, Saatgut, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium.

Einjährige oder mehrjährige, dicotyledone Pflanzen sind bevorzuge Wirtsorganismen für die Herstellung transgener Pflanzen. Die Expression von Genen ist ferner vorteilhaft bei allen Schmuckpflanzen, Nutz- oder Zierbäumen, Blumen, Schnittblumen, Sträuchern oder Rasen. Beispielhaft aber nicht einschränkend seien zu nennen dicotyledone Pflanzen ausgewählt aus der Gruppe bestend aus Angiospermen, Bryophyten wie zum Beispiel Hepaticae (Leberblümchen) und Musci (Moose); Pteridophyten wie Farne, Schachtelhalm und Lycopoden; Gymnospermen wie Koniferen, Cycaden, Ginkgo und Gnetalen; Algen wie Chlorophyceae, Phaeophpyceae, Rhodophyceae, Myxophyceae, Xanthophyceae, Bacillariophyceae (Diatomeen) und Euglenophyceae.

Bevorzugt sind dicotyledone Pflanzen nachfolgender Pflanzenfamilien: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae, ümbelliferae.

Monokotyle Pflanzen sind z. B. die monokotyle Kulturpflanzen, wie zum Beispiel die Familie der Gramineae wie Reis, Mais, Weizen oder andere Getreidearten wie Gerste, Hirse, Roggen, Triticale oder Hafer sowie Zuckerrohr sowie alle Arten von Gräsern.

Bevorzugte dikotyle Pflanzen sind insbesondere ausgewählt aus den dikotylen Kulturpflanzen, wie zum Beispiel
- Asteraceae wie Sonnenblume, Tagetes oder Calendula und andere mehr,
- Compositae, besonders die Gattung Lactuca, ganz besonders die Art sativa (Salat) und andere mehr,
- Cruciferae, besonders die Gattung Brassica, ganz besonders die Arten napus (Raps), campestris (Rübe), oleracea cv Tastie (Kohl), oleracea cv Snowball Y (Blumenkohl) und oleracea cv Emperor (Broccoli) und weitere Kohlarten; und der Gattung Arabidopsis, ganz besonders die Art thaliana sowie Kresse oder Canola und andere mehr,
- Cucurbitaceae wie Melone, Kürbis oder Zucchini und andere mehr,
- Leguminosae besonders die Gattung Glycine, ganz besonders die Art max (Sojabohne) Soja sowie Alfalfa, Erbse, Bohnengewächsen oder Erdnuss und andere mehr
- Rubiaceae, bevorzugt der Unterklasse Lamiidae wie beispielsweise Coffea arabica oder Coffea liberica (Kaffestrauch) und andere mehr,
- Solanaceae besonders die Gattung Lycopersicon, ganz besonders die Art esculentum (Tomate) und die Gattung Solanum, ganz besonders die Art tuberosum (Kartoffel) und melongena (Aubergine) sowie Tabak oder Paprika und andere mehr,
- Sterculiaceae, bevorzugt der Unterklasse Dilleniidae wie beispielsweise Theobroma cacao (Kakaostrauch) und andere mehr,
- Theaceae, bevorzugt der Unterklasse Dilleniidae wie beispielsweise Camellia sinensis oder Thea sinensis (Teestrauch) und andere mehr,
- Umbelliferae, besonders die Gattung Daucus (ganz besonders die Art carota (Karotte) und Apium (ganz besonders die Art graveolens dulce (Selarie) und andere mehr; und die Gattung Capsicum, ganz besonders die Art annum (Pfeffer) und andere mehr,
sowie Lein, Soja, Baumwolle, Hanf, Flachs, Gurke, Spinat, Möhre, Zuckerrübe und den verschiedenen Baum-, Nuss- und Weinarten, insbesondere Banane und Kiwi.

Umfasst sind ferner dicotyledone Schmuckpflanzen, dicotyledone Nutz- oder zierbäumen, Blumen, Schnittblumen, Sträuchern oder Rasen. Beispielhaft für diese sind Dicotyledone zu nennen die ausgewählt werden aus der Gruppe bestehend aus Angiospexmen, Bryophyten wie zum Beispiel Hepaticae (Lebexblümchen) und Musci (Moose); Pteridophyten wie Farne, Schachtelhalm und Lycopoden; Gymnospermen wie Koniferen, Cycaden, Ginkgo und Gnetalen, die Familien der Rosaceae wie Rose, Ericaceae wie Rhododendrons und Azaleen, Euphorbiaceae wie Weihnachtssterne und Kroton, Caryophyllaceae wie Nelken, Solanaceae wie Petunien, Gesneriaceae wie das Usambaraveilchen, Balsaminaceae wie das Springkraut, Orchidaceae wie Orchideen, Iridaceae wie Gladiolen, Iris, Freesie und Krokus, Compositae wie Ringelblume, Geraniaceae wie Geranien, Liliaceae wie der Drachenbaum, Moraceae wie Ficus, Araceae wie Philodendron und andere mehr.

Am meisten bevorzugt sind Arabidopsis thaliana, Nicotiana tabacum, Tagetes erecta, Calendula officinalis und Brassica napus sowie alle Gattungen und Arten, die als Nahrungs- oder Futtermittel zum Einsatz kommen, wie die beschriebenen Getreidearten, oder sich zur Herstellung von ölen eignen, wie Ölsaaten (wie zum Beispiel Raps), Nussarten, Soja, Sonnenblume, Kürbis und Erdnuss.

Pflanzliche Organismen im Sinne der Erfindung sind weiterhin weitere photosynthetisch aktive befähigte Organismen, wie zum Beispiel Algen oder Cyanobakterien, sowie Moose.

Bevorzugte Algen sind Grünalgen, wie beispielsweise Algen der Gattung Haematococcus, Phaedactylum tricornatum, Volvox oder Dunaliella.

Die Herstellung eines transformierten Organismus oder einer transformierten Zelle erfordert, dass die entsprechende DNA in die entsprechende Wirtszelle eingebracht wird. Für diesen Vorgang, der als Transformation bezeichnet wird, steht eine Vielzahl von Methoden zur Verfügung (siehe auch Keown et al. 1990 Methods in Enzymology 185;527-537). So kann die DNA beispielhaft direkt durch Mikroinjektion oder durch Bombardierung mit DNAbeschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden.

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte parzicle bombardment Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Diese Stämme enthalten ein Plasmid (Ti bzw. Ri Plasmid), das auf die Pflanze nach Agrobacterium-Infektion übertragen wird. Ein Teil dieses Plasmids, genannt T-DNA (transferred DNA), wird in das Genom der Pflanzenzelle integriert.
Die Agrobacterium-vermittelte Transformation ist am besten für dicotyledone, diploide Pflanzenzellen geeignet, wohingegen die direkten Transformationstechniken sich für jeden Zelltyp eignen.

Die Einführung einer erfindungsgemässgemäßen Expressionskassette in Zellen, bevorzugt in pflanzliche Zellen, kann vorteilhaft unter Verwendung von Vektoren realisiert werden.

In einer vorteilhaften Ausführungsform wird die Einführung der Expressionskassette mittels Plasmidvektoren realisiert.
Bevorzugt sind solche Vektoren, die eine stabile Integration der Expressionskassette in das Wirtsgenom ermöglichen.

Im Falle von Injektion oder Elektroporation von DNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Transformationstechniken sind für verschiedene monokotyle und dikotyle pflanzliche Organismen beschrieben, ferner stehen verschiedene mögliche Plasmidvektoren für die Einführung fremder Gene in Pflanzen zur Verfügung, die in der Regel einen Replikationsursprung für eine Vermehrung in E.coli und ein Markergen für eine Selektion transformierter Bakterien enthalten. Beispiele sind pBR322, pUC Reihe, M13mp Reihe, pACYC184 etc.

Die Expressionskassette kann in den Vektor über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide zu verleihen vermag. Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind das bar Gen, dass Resistenz gegen das Herbizid Phosphinothricin verleiht (Rathore KS et al.,Plant Mol Biol. 1993 Mar;21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht.

Je nach Methode der DNA-Einführung können weitere Gene auf dem Vektorplasmid erforderlich sein. Werden Agrobacteria verwendet, so ist die Expressionskassette in spezielle Plasmide zu integrieren, entweder in einen Zwischenvektor (englisch: shuttle or intermediate vector) oder einen binären Vektor. Wenn zum Beispiel ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden. Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al.,Mol. Gen. Genet. 163 (1978), 181-187). Das Selektionsmarkergen erlaubt eine Selektion transformierter Agrobacteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden.

Die Verwendung von T-DNA zur Transformation pflanzlicher Zellen ist intensiv untersucht und beschrieben (EP 120516; Hoekema, In: The Binary Plant Vector System, Offsetdrukkerij Kanters B.V., Alblasserdam, Chapter V; Fraley et al., Crit. Rev. Plant. Sci., 4:1-46 and An et al., EMBO J. 4 (1985), 277-287). Verschiedene binäre Vektoren sind bekannt und teilweise kommerziell erhältlich wie zum Beispiel pBIN19 (Clontech Laboratories, Inc. U.S.A.).

Für den Transfer der DNA inauf die pflanzliche Zelle werden pflanzliche Explantate mit Agrobacterium tumefaciens oder Agrobacterium rhizogenes kokultiviert. Ausgehend von infiziertem Pflanzenmaterial (z.B. Blatt-, Wurzel- oder Stengelteile, aber auch Protoplasten oder Suspensionen von Pflanzenzellen) können ganze Pflanzen unter Verwendung eines geeigneten Mediums, dass zum Beispiel Antibiotika oder Biozide zur Selektion transformiertenr Zellen enthalten kann, regeneriert werden. Die erhaltenen Pflanzen können dann auf die Präsenz der eingeführten DNA, hier der erfindungsgemässgemäßen Expressionskassette, durchmustert werden. Sobald die DNA in das Wirtsgenom integriert ist, ist der entsprechende Genotyp in der Regel stabil und die entsprechende Insertion wird auch in den Nachfolgegenerationen wiedergefunden. In der Regel enthält die integrierte Expressionskassette einen Selektionsmarker, der der transformierten Pflanze eine Resistenz gegen ein Biozid (zum Beispiel ein Herbizid), einen Metabolismusinhibitor wie 2-DOG oder ein Antibiotikum wie Kanamycin, G 418, Bleomycin, Hygromycin oder Phosphinothricin etc. verleiht. Der Selektionsmarker erlaubt die Selektion von transformierten Zellen von untransformierten (McCormick et al.,(1986) Plant Cell Reports 5: (1986), 81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die genannten Verfahren sind beispielsweise in B. Jenes et al., Techniques for Gene Transfer, in: Transgenic Plants, Vol. 1, Engineering and Utilization, herausgegeben von S.D. Kung SD und R. Wu R, Academic Press (1993), S.128 - 143 sowie in Potrykus , (1991) Annu. Rev. Plant Physiol. Plant Molec. Biol. 42: (1991), 205 - 225) beschrieben. Vorzugsweise wird das zu exprimierende Konstrukt in einen Vektor kloniert, der geeignet ist, Agrobakterium tumefaciens zu transformieren, beispielsweise pBin19 (Bevan et al.,(1984) Nucl. Acids Res. 12: (1984), 8711f.).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Die Wirksamkeit der Expression der transgen exprimierten Nukleinsäuren kann beispielsweise *in vitro* durch Sprossmeristemvermehrung unter Verwendung einer der oben beschriebenen Selektionsmethoden ermittelt werden.

Erfindungsgemässgemäß sind ferner von den oben beschriebenen transgenen Organismen abgeleitete Zellen, Zellkulturen, Teilewie zum Beispiel bei transgenen pflanzlichen Organismen Wurzeln, Blätter etc.-, und transgenes Vermehrungsgut wie Saaten oder Früchte.

Von Menschen und Tieren verzehrbare erfindungsgemässgemäße, genetisch veränderte Pflanzen können auch beispielsweise direkt oder nach an sich bekannter Aufbereitung als Nahrungsmittel oder Futtermittel verwendet werden.

Ein weiterer Gegenstand der Erfindung betrifft die Verwendung der oben beschriebenen erfindungsgemässgemäßen, transgenen Organismen und der von ihnen abgeleitete Zellen, Zellkulturen, Teile - wie zum Beispiel bei transgenen pflanzlichen Organismen Wurzeln, Blätter etc.- , und transgenes Vermehrungsgut wie Saaten oder Früchte, zur Herstellung von Nahrungs- oder Futtermitteln, Pharmazeutika oder Feinchemikalien.

In Verfahren zur rekombinanten Herstellung von Pharmazeutika oder Feinchemikalien in Wirtsorganismen kann ein Wirtsorganismus mit einer der oben beschriebenen Expressionskaassetten oder Vvektoren transformiert werden und diese Expressionskassette ein oder mehrere Strukturgene enthalten, die für die gewünschte Feinchemikalie kodieren oder die Biosynthese der gewünschten Feinchemikalie katalysieren, der transformierte Wirtsorganismus gezüchtet werden und die gewünschte Feinchemikalie aus dem Züchtungsmedium isoliert werden. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar, beispielsweise die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mittels der dem Fachmann bekannten Verfahren. Die Produktion von Pharmazeutika, wie zum Beispiel Antikörpern oder Vakkzinen ist beschrieben bei Hood EE, Jilka JM (1999). Curr Opin Biotechnol. 1999 Aug;10(4):382-6; Ma JK, Vine ND (1999) .Curr Top Microbiol Immunol. 1999;236:275-92.

### Sequenzen

1. SEQ ID NO: 1
   Promotor and 5'-untranslatierte Region des Arabidopsis thaliana pFD-Promotors.
2. SEQ ID NO: 2
   Promotor and 5'-untranslatierte Region des Arabidopsis thaliana FNR-Promotors.
3. SEQ ID NO: 3
   Promotor and 5'-untranslatierte Region des Arabidopsis thaliana TPT-Promotors (2038 bp).
4. SEQ ID NO: 4
   Promotor and 5'-untranslatierte Region des verkürzten Arabidopsis thaliana pFDs-Promotors
5. SEQ ID NO: 5
   Nukleinsäure kodierend für eine Phosphinothricinacetyltransferase.
6. SEQ ID NO: 6
   Aminosäuresequenzs kodierend für eine Phosphinothricinacetyltransferase.
7. SEQ ID NO: 7
   Nukleinsäure kodierend für eine Acetolactatsynthase.
8. SEQ ID NO: 8
   Aminosäuresequenzs kodierend für eine Acetolactatsynthase.
9. SEQ ID NO: 9 - Oligonukleotid-Primer pWL35
   5'-GTC GAC GAA TTC GAG AGA CAG AGA GAC GG-3'
10. SEQ ID NO: 10 - Oligonukleotid-Primer pWL36
   5'-GTC GAC GGT ACC GAT TCA AGC TTC ACT GC-3'
11. SEQ ID NO: 11 - Oligonukleotid-Primer pFD1
   5'-GAG AAT TCG ATT CAA GCT TCA CTG C-3'
12. SEQ ID NO: 12 - Oligonukleotid-Primer pFD2
   5'-CCA TGG GAG AGA CAG AGA GAC G-3'
13. SEQ ID NO: 13 - Oligonukleotid-Primer pFD3
   5'-acggatccgagagacagagagacggagacaaaa-3'
14. SEQ ID NO: 14 - Oligonukleotid-Primer pFD5
   5'-gcggatccaacactcttaaeaccaaatcaaca-3'
15. SEQ ID NO: 15 - Oligonukleotid-Primer L-FNR ara
   5'-GTCGACGGATCCGGTTGATCAGAAGAAGAAGAAGAAGATGAACT-3'
16. SEQ ID NO: 16 - Oligonukleotid-Primer R-FNR ara
   5'-GTCGACTCTAGATTCATTATTTCGATTTTGATTTCGTGACC -3'
17. SEQ ID NO: 17 - Oligonukleotid-Primer L-TFTara
   5'-AAGTCGACGGATCCATAACCAAAAGCAACTCTGATCATGTACGTACCCATT-3'
18. SEQ ID NO: 18 - Oligonukleotid-Primer R-TPTara
   5'-AGACGTCGACTCTAGAATGAAATCGAAATTCAGAGTTTTGATAGTGAGAGC-3'
19. SEQ ID NO: 19 - Oligonukleotid-Primer ubi5
   5'-CCAAACCATGGTAAGTTTGTCTAAAGCTTA-3'
20. SEQ ID NO: 20 - Oligonukleotid-Primer ubi3
   5'-CGGATCCTTTTGTGTTTCGTCTTCTCTCACG-3'
21. SEQ ID NO: 21 - Oligonukleotid-Primer sqs5
   5'-GTCTAGAGGCAAACCACCGAGTGTT-3'
22. SEQ ID NO: 22 - Oligonukleotid-Primer sqs3
   5'-CGGTACCTGTTTCCAGAAAATTTTGATTCAG-3'
23. SEQ ID NO: 23
   Binärplasmid pSUN3 (Sungene GmbH & Co KGaA)
24. SEQ ID NO: 24
   Binärplasmid pSUN5NPTIICat (Sungene GmbH & Co KGaA)
25. SEQ ID NO: 25
   Binärplasmid pSUN3PatNos (Sungene GmbH & Co KGaA)
26. SEQ ID NO: 26 - Oligonukleotidprimer 5-TPTara
   5'-AAGTCGACGGATCCTGATAGCTTATACTCAAATTCAACAAGTTAT-3'
27. SEQ ID NO: 27
   Verkürzter Promotor and 5'-untranslatierte Region des Arabidopsis thaliana TPT-Promotors (1318 bp).
28. SEQ ID NO: 28
   Nukleinsäuresequenz des Terminators des Cathepsin D Inhibitor Gens aus Kartoffel (GenBank Acc. No.: X74985)
29. SEQ ID NO: 29
   Nukleinsäuresequenzoder des Terminators der Speicherproteingens VfLElB3 aus der Ackerbohne (GenBank Acc. No.: Z26489).

### Beispiele

### Beschreibung der Abbildungen

1. Abbildung la-c: Der TPT und der FNR Promotor zeigen ein vergleichbares Expressionsmuster im grünen Gewebe sowie in Blüten von Tabak und Kartoffel. Gezeigt sind GUS histochemische Färbungen. Die Intensität der GUS-Blaufärbung entspricht den wiedergegebenen Graustufen. Gezeigt sind:
Abbildung 1a:
   - A:: Kartoffelblätter mit einer homogenen, intensiven Färbung über den gesamten Blattbereich.
   - B:: Tabak Petiolen, intensive Blaufärbung vor allem im Randbereich und den vaskulären Bereichen (siehe Pfeil)
Abbildung 1b:
   - C:: Tabak Stengel, intensive Blaufärbung vor allem im Randbereich (siehe Pfeil)
   - D:: Tabak Internodien
Abbildung 1c:
   - E:: Tabak Blüte; Blaufärbung vor allem in Kelch- und Blütenblättern

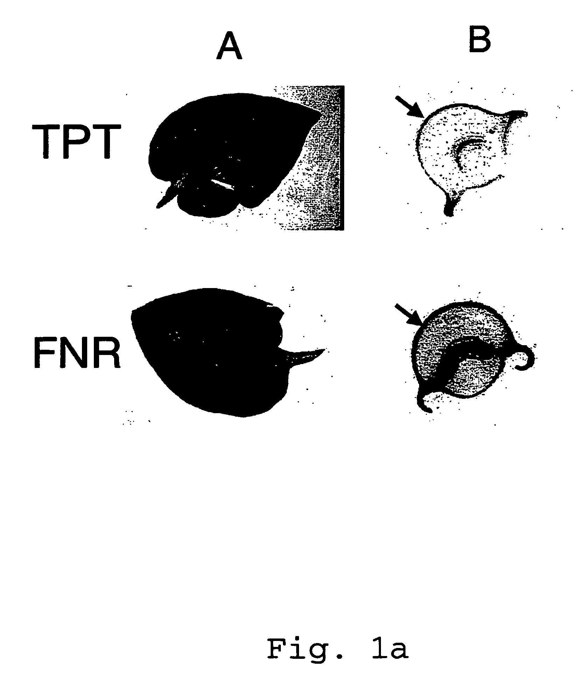

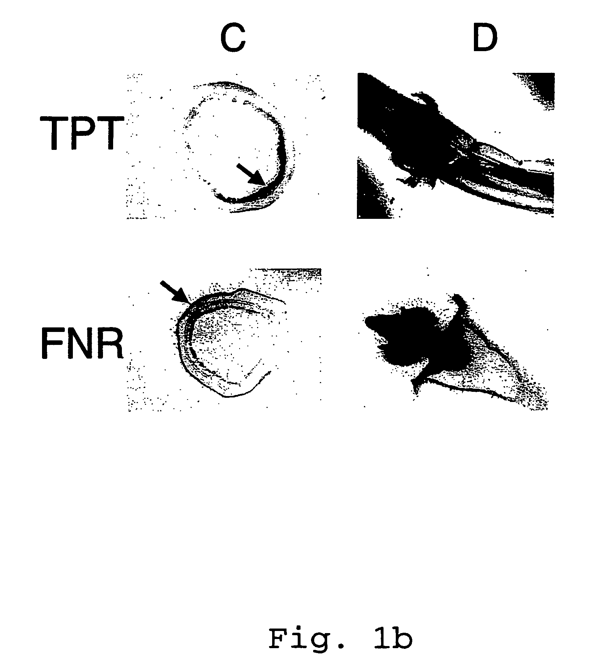

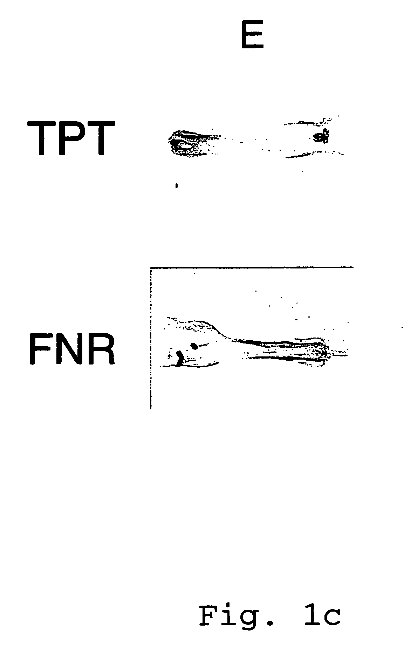

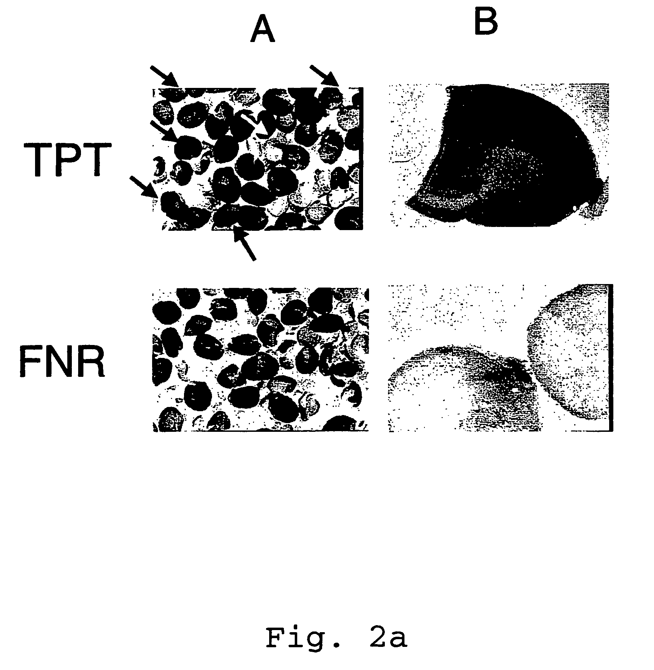

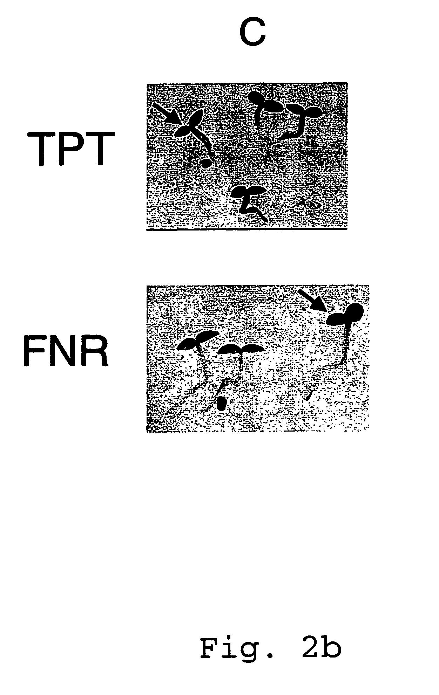

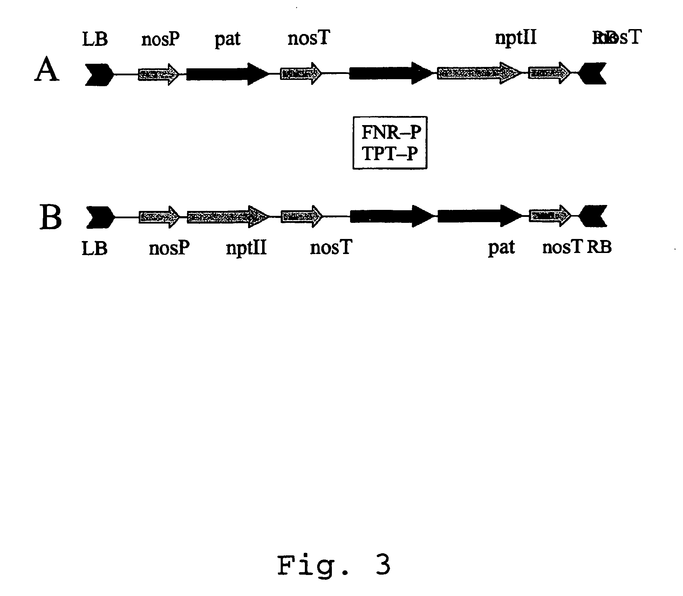

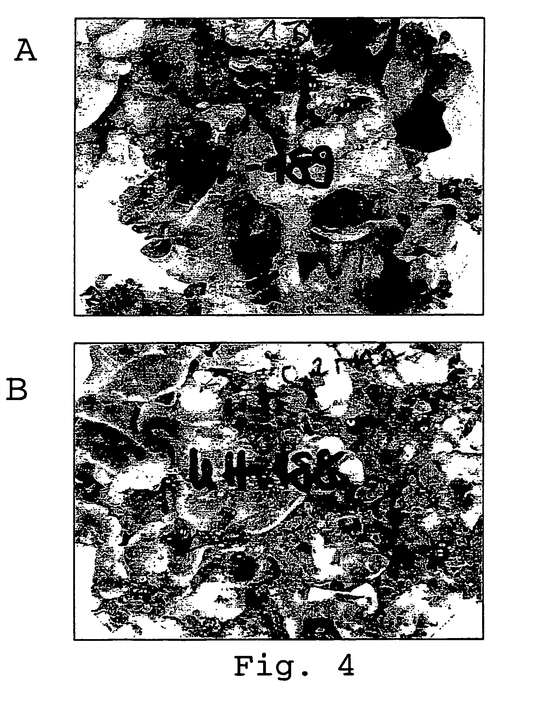

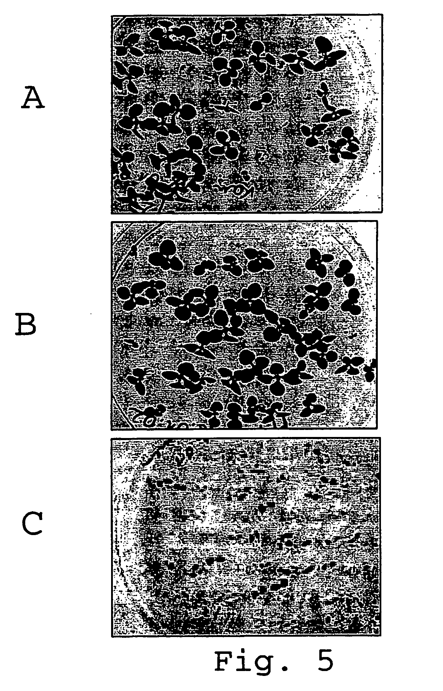

2. Abbildung 2a-b: Der TPT und der FNR Promotor zeigen ein unterschiedliches Expressionsmuster im vegetativen und germinativen Speichergewebe von Tabak und Kartoffel. Während der TPT Promotor hier aktiv ist, zeigt der FNR Promotor keine Expression. Gezeigt sind GUS histochemische Färbungen von Tabaksamen und Tabaksämlingen sowie von Kartoffelknollen. Beide Promotoren zeigen jedoch wieder eine vergleichbare Aktivität in Sämlingen. Die Intensität der GUS-Blaufärbung entspricht den wiedergegebenen Graustufen. Gezeigt sind:
Abbildung 2a:
   - A:: Tabaksamen. Beim TPT Promotor sind einzelne blau gefärbte Samenkörner erkennbar (siehe Pfeil). Beim FNR Promotor sind keine Färbungen zu detektieren.
   - B:: Kartoffelknollen. Beim TPT Promotor ist eine homogene, starke Blaufärbung der Kartoffelknolle erkennbar. Beim FNR Promotor ist keine oder nur eine sehr schwache Färbung zu detektieren.
Abbildung 2b:
   - C:: Tabaksämlinge (10 Tage alt). Beide Promotoren zeigen eine vergleichbare Blaufärbung (siehe Pfeil).

3. Expressionskassetten für die Expression von Kanamycin-(nptII) und Phosphinothricin- (pat) Resistenzmarkern. Kassette A gewährt eine Expression von Kanamycinresistenz unter dem TPT bzw. FNR Promotor, neben einer Phosphinothricinresistenz unter dem NOS Promotor. Kassette B gewährt eine Expression von Phosphinothricinresistenz unter dem TPT bzw. FNR Promotor, neben einer Kanamycinresistenz unter dem NOS Promotor.

| | |
|---|---|
| LB, RB: | Linke bzw. rechte Begrenzung der Agrobacterium T-DNA |
| nosP: | NOS Promotor |
| pat: | Nukleinsäuresequenz kodierend für Phosphinothricin-acetyltransferase (pat) |
| nptII: | Kanamycinresistenzgen (Neomycinphosphotransferase) |
| nosT: | NOS Terminator |
| FNR-P: | FNR Promotor |
| TPT-P; | TPT Promotor |

4. Regeneration transformierter Tabaksprossknospen unter einem Kanamycinselektionsdruck (100 mg/l Kanamycin). A: Tranformation mit einem FNR-Promotor - nptII Konstrukt. B: Tranformation mit einem TPT-Promotor - nptII Konstrukt. Eine vergleichbar effiziente Regeneration von transformierten Tabakpflanzen konnte beobachtet werden.
5. Keimung transformierter Tabakpflänzchen aus transgener Tabaksaat unter einem Phosphinothricinselektionsdruck (10 mg/l Phosphinotricin).
- A:: Transformiert mit einem FNR-Promotor - pat Konstrukt.
- B:: Transformiert mit einem TPT-Promotor - pat Konstrukt.
- C:: Kontrolle mit nicht-transformierter Tabaksaat.

Eine vergleichbar effiziente Keimung von mit dem FNR-Promotor-pat Konstrukt und TPT-Promotor-pat Konstrukt transformierten Tabakpflanzen konnte beobachtet werden, während entsprechend behandelte nicht-transformierte Tabakpflanzen keine Resistenz aufwiesen.

### Beispiele

### Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophoresen, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma ABI nach der Methode von Sanger (Sanger et al. (1977) Proc Natl Acad Sci USA 74:5463-5467),

### Beispiel 1: Isolierung von genomischer DNA aus Arabidopsis thaliana (CTAB-Methode)

Zur Isolierung genomischer DNA aus *Arabidopsis thaliana* werden ca. 0,25 g Blattmaterial junger Pflanzen im vegetativen Stadium in flüssigem Stickstoff zu feinem Pulver gemörsert. Das pulverisierte Pflanzenmaterial wird zusammen mit 1 ml 65°C-warmem CTAB I-Puffer (CTAB: Hexadecyltrimethylammoniumbromid, auch genannt Cetyltrimethylammoniumbromid; Sigma Kat.-Nr.: H6269) und 20 µl β-Mercaptoethanol in einen vorgewärmten zweiten Mörser gegeben und nach vollständiger Homogenisierung wird der Extrakt in ein 2 ml Eppendorf-Gefäß überführt und für 1 h bei 65°C unter regelmäßiger, vorsichtiger Durchmischung inkubiert. Nach Abkühlung auf Raumtemperatur wird der Ansatz mit 1 ml Chloroform/Octanol (24:1, mit IM Tris/HC1, pH8,0 ausgeschüttelt) durch langsames Invertieren extrahiert und zur Phasentrennung für 5 min bei 8.500 rpm (7.500 x g) und Raumtemperatur zentrifugiert. Anschließend wird die wäßrwässrige Phase erneut mit 1 ml Chloroform/Octanol extrahiert, zentrifugiert und durch Invertieren mit 1/10 Volumen auf 65°C vorgewärmtem CTAB II-Puffer sorgfältig gemischt. Anschließend wird der Ansatz durch vorsichtiges Schwenken mit 1 ml Chloroform/Octanol-Gemisch (siehe oben) versetzt und zur erneuten Phasentrennung für 5 min bei 8.500 rpm (7.500 x g) und Raumtemperatur zentrifugiert. Die wässßrige untere Phase wird in ein frisches Eppendorff-Gefäß überführt und die obere organische Phase wird in einem frischen Eppendorff-Gefäß erneut für 15 min bei 8.500 rpm (7.500 x g) und Raumtemperatur zentrifugiert. Die hieraus resultierende wäßrwässrige Phase wird mit der wässßrigen Phase des vorherigen Zentrifugationsschrittes vereinigt und der gesamte Ansatz mit exakt demselben Volumen vorgewärmtem CTAB III-Puffer versetzt. Es folgt eine Inkubation bei 65°C, bis die DNA in Flocken ausfällt. Dies kann bis zu 1 h dauern oder durch Inkubation bei 37°C über Nacht erfolgen. Das aus dem anschließenden Zentrifugationsschritt (5 min, 2000 rpm (500 x g), 4°C) resultierende Sediment wird mit 250 µl auf 65°C vorgewärmtem CTAB IV-Puffer versetzt und für mindestens 30 min bzw. bis zur vollständigen Auflösung des Sediments bei 65°C inkubiert. Anschließend wird die Lösung zur Fällung der DNA mit 2,5 Volumina eiskaltem Ethanol vermischt und für 1h bei -20°C inkubiert. Alternativ kann der Ansatz mit 0.6 Volumina Isopropanol vermischt und ohne weitere Inkuabation sofort für 15 min bei 8.500 rpm (7.500 x g) und 4°C zentrifugiert werden. Dieas sedimentierte DNA wird durch Invertieren des Eppendorff-Gefäßes zweimal mit je 1 ml 80%igem eiskaltem Ethanol gewaschen, nach jedem Waschschritt erneut zentrifugiert (5 min, 8.500 rpm (7.500 x g), 4°C) und anschließend für ca. 15 min luftgetrocknet. Abschließend wird die DNA in 100 µl TE mit 100 µg/ml RNase resuspendiert und für 30 min bei Raumtemperatur inkubiert. Die DNA Lösung ist nach einer weiteren Inkubationsphase über Nacht bei 4°C homogen und kann für weiterführende Experimente verwendet werden.

### Lösungen für CTAB;

Lösung I (für 200 ml):
   100 mM Tris/HCl pH 8,0 (2,42 g)
   1,4 M NaCl (16,36 g)
   20 mM EDTA (8,0 ml von 0,5 M Stammlösung)
   2 (w/v) CTAB (4,0 g)

Jeweils vor der Verwendung werden frisch zugesetzTen: 2% β-Mercaptoethanol (20 µl für 1 ml Lösung I).
Lösung II (für 200 ml):
   0,7 M NaC1 (8,18 g)
   10 %(w/v) CTAB (20 g)
Lösung III (für 200 ml) :
   50 mM Tris/HCl pH 8,0 (1,21 g)
   10 mM EDTA (4 ml 0,5 M von 0,5 M Stammlösung)
   1 %(w/v) CTAB (2,0 g)
Lösung IV (High-salt TE) (für 200 ml):
   10 mM Tris/ HCl pH 8,0 (0,242 g)
   0,1 mM EDTA (40 µl 0.5 M Stammlösung)
   1 M NaCl (11, 69 g)
Chloroform/Octanol (24:1) (für 200 ml):
   192 ml Chloroform
   8 ml Octanol

Die Mischung wird 2x mit 1 M TrisHCl pH 8,0 ausgeschüttelt und vor Licht geschützt gelagert.

### Beispiel 2: TTabaktransformation von Tabak, Raps und Kartoffel

Die Transformation von Tabak erfolgte über Infektion mit *Agrobacterium tumefaciens.* gemäß der von Horsch entwickelten Methode (Horsch et al. (1985) Science 227: 1229-1231). Alle zur Transformation verwendeten Konstrukte wurden anhand der Gefrier/Tau-Methode (wiederholtes Auftauen und Einfrieren) in Agrobacterium tumefaciens transformiert. Die das gewünschte Konstrukt enthaltenden Agrobacterium-Kolonien wurden auf Mannitol/Glutamat-Medium mit 50 µg/ml Kanamycin, 50 µg/ml Ampicilin und 25 µg/ml Rifampicin selektioniert.

Zur Transformation von Tabakpflanzen (*Nicotiana tabacum* L. cv. *Samsun* NN) wurden 10 ml einer unter Selektion gewachsenen Übernachtkultur von *Agrobacterium tumefaciens* abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1cm) in dieser Bakterienlösung gebadet. Anschließend wurden die Blattscheiben in Petrischalen auf MS-Medium (Murashige und Skoog (1962) Physiol Plant 15:473ff.) mit 2% Saccharose und 0.8% Bacto-Agar ausgelegt. Nach 2-tägiger Inkubation im Dunkeln bei 25°C wurden sie auf MS-Medium mit 100 mg/l Kanamycin, 500 mg/l Claforan, 1mg/l Benzylaminopurin (BAP), 0,2 mg/l Naphtylessigsäure (NAA), 1,6% Glukose und 0.8% Bacto-Agar übertragen und die Kultivierung (16 Stunden Licht / 8 Stunden Dunkelheit) fortgesetzt. Wachsende Sprosse wurden auf hormonfreies MS-Medium mit 2% Saccharose, 250 mg/l Claforan und 0,8% Bacto-Agar überführt.

Die Transformation von Raps erfolgte mittels der Petiolentransformation nach Moloney et al. (Moloney MM, Walker JM & Sharma KK (1989) Plant Cell Reports 8:238-242).

Zur Transformation von Kartoffel (Solanum tuberosum) wurden Blattscheiben und Internodien von in vitro Planzen mit Agrobacterium tumefaciens in flüssigem Murashige Skoog Medium für 20 Minuten infiziert, und anschließend für 2 d im Dunkeln cocultiviert. Nach der Co-cultur wurden die Explantate auf festes MS Medium, das anstelle von Saccharose 1,6% Glucose enthält (MG) und mit 5 mg/l NAA, 0,1 mg/l BAP, 250 mg/l Timentin und 30 bis 40 mg/l Kanamycin ergänzt worden ist (KIM), bei 21°C in einem Licht/Dunkel-Rhythmus von 16h/8h kultiviert. Nach diese Kallusphase wurden die Explantate auf Sproßinduktionsmedium (SIM) gelegt. SIM war wie folgt zusammengesetzt: MG + 2 mg/l Zeatinribosid, 0,02 mg/l NAA, 0,02 mg/l GA3, 250 mg/l Timentin, 30 bis 40 mg/l Kanamycin. Alle zwei Wochen wurden die Explantate auf frisches SIM transferiert. Die sich bildenden Sprosse wurden auf MS Medium mit 2% Saccharose und 250 mg/l Timentin und 30 bis 40 mg/l Kanamycin bewurzelt.

### Vergeichsbeispiel 3: Untersuchungen zur Eignung des putativen Ferredoxin (pFD) Promotors

### a) Klonierung des pFD Promotors aus Arabidopsis thaliana

Der putative Ferredoxin-Promotor wurde mittels PCR aus genomischer *Arabidopsis thaliana* DNA mit den Primern pWL35 und pWL36 amplifiziert. Der Primer pWL35 beginnt mit den in Fettdruck hervorgehobenen *Sal*I und *Eco*RI Restriktionsschnittstellen unmittelbar vor der kodierenden Region des pFD-Gens. Der Primer pWL36 beginnt mit den in Fettdruck hervorgehobenen *Sal*I und *Asp*718 Restriktionsschnittstellen.
Primer pWL35 (SEQ ID NO: 9)
5' GTC GAC GAA TTC GAG AGA CAG AGA GAC GG 3'
Primer pWL36 (SEQ ID NO: 10)
5' GTC GAC GGT ACC GAT TCA AGC TTC ACT GC 3'

| Reaktionsansatz: | |
|---|---|
| 1 µl | Genomische Arabidopsis DNA (ca. 250 ng) |
| 0,5 µl | Tth-Polymerase (2U/µl) |
| 3 µl | Mg(OAc)₂ (25mM, final conc. 1,5 mM Mg²⁺) |
| 15,2 µl | 3,3 x Puffer |
| 4 µl | dNTPs (jeweils 2,5 mM, Takara, Endkonzentration: jeweils 200 µM) |
| 24,3 µl | H₂O |

| PCR-Bedingungen: | |
|---|---|
| 1 | Zyklus mit 3 min. bei 95°C |
| 10 | Zyklen mit 94°C für 10 sec, 50°C für 20 sec und 72°C für 1 min. |
| 20 | Zyklen mit 94°C für 10 sec, 65°C für 20 sec und 72°C für 1 min. |
| 1 | Zyklus mit 72°C für 5 min., anschließssend Kühlung auf 4°C bis zur Weiterverarbeitung. |

### b) Konstruktion der pFD-Promotor-GUS-Expressionskassette

Das pFD-Promotor PCR-Produkt wurde in den Vektor pCRII (Invitrogen) kloniert und anschließend mittels der durch das Primerpaar eingeführten *Sal*I-Restriktionsschnittstellen isoliert und gelelektrophoretisch gereinigt. Für die Fusion mit dem GUS Gen wurde das ca. 850 bp umfassende pFD-Promotorfragment in den *Sal*I-restringiertengeschnittenen Binärvektor pBI101.2 (Clontech Inc.) kloniert und die Orientierung des Fragments wurde anschließend anhand von Restriktionsanalysen unter Verwendung der Endonukleasen *Bgl*II und *Bam*HI verifiziert. Das resultierende Plasmid pFD::GUS wurde in Tabak transformiert. Die erzeugten Tabakpflanzen wurden pFD:GUS genannt.

### c) Konstruktion der pFD-Promotor-nptII-Expressionskassette

Der putative Ferredoxin-Promotor wurde mittels PCR aus genomischer *Arabidopsis thaliana* DNA amplifiziert. Dabei wurden über die Primer die Restriktionsstellen EcoRI und NcoI angefügt.
Primer pFD1 (SEQ ID NO: 11)
5' GAG AAT TCG ATT CAA GCT TCA CTG C
Primer pFD2 (SEQ ID NO: 12)
5' CCA TGG GAG AGA CAG AGA GAC G

| Reaktionsansatz: | |
|---|---|
| 37,5 µl | H2O |
| 5,0 µl | 10X Reaktionspuffer (Endkonzentration Mg²⁺ 1,5 mM) |
| 4,0 µl | dNTP mix (jeweils 2,5 mM) |
| 1,0 µl | Primer pFD1 (10 µM) |
| 1,0 µl | Primer pFD2 (10 µM) |
| 0,5 µl | Taq-Polymerase (Takara, 2U/µl) |
| 1,0 µl | genomischer Arabidopsis DNA (ca. 250 ng) |

| PCR-Bedingungen: | |
|---|---|
| 1 | Zyklus mit 3 min. bei 94°C |
| 10 | Zyklen mit 94°C für 10 sec, 48°C für 20 sec und 72°C für 1 min |
| 25 | Zyklen mit 94°C für 10 sec, 65°C für 20 sec und 72°C für 1 min |
| 1 | Zyklus mit 72°C für 5 min. |

Das PCR Produkt wurde in das Plasmid pCRII (Invitrogen) subkloniert. Das Plasmid pCAMBIA 2300 (CAMBIA, GPO Box 3200, Canberra ACT 2601, Australia; GenBank Acc.-No: AF234315; Binary vector pCAMBIA-2300, complete sequence; Hajdukiewicz P et al. (1994) Plant Mol Biol 25(6):989-994) wurde mit EcoRI/NcoI geschnitten und das pFD Promotorfragment als EcoRI/NcoI Fragment aus dem pCRII Plasmid in diesen Vektor umkloniert. Dabei wurde der 35S Promotor aus dem Cambiavektor entfernt. Das resultierende Plasmid wurde als pFD-Promotor:NPTII bezeichnet und in Tabak transformiert.

### d) Ergebnisse der GUS Analyse der transgenen Tabakpflanzen

Im Rahmen histochemischer Untersuchungen zeigten transgene pFD :: GUS Tabakpflanzen eine starke GUS-Färbung in 'source'-Blättern und eine schwache GUS-Färbung in den Geweben aller Blütenorgane. Eine starke Färbung im Wurzelgewebe wurde nur bei *in vitro-*Pflanzen beobachtet, deren Wurzeln der Belichtung ausgesetzt waren. Anhand von Blattscheiben, die aus als pFD::GUS-positiv identifizierten Pflanzen ausgestanzt worden waren, wurde Kalluswachstum induziert. Das Kallusgewebe sowie die sich daraus entwickelnden Pflanzensprosse zeigten eine GUS-Färbung, die in ihrer Intensität der GUS-Färbung von CaMV35S::GUS (in pCambia 1304; CAMBIA, GPO Box 3200, Canberra ACT 2601, Australia; GenBank Acc.-No.: AF234300, Binary vector pCAMBIA-1304, complete sequence, Hajdukiewicz P et al. (1994) Plant Mol. Biol. 25(6):989-994) (1994)) transgenen Pflanzen vergleichbar war. In der unten aufgeführten Tabelle (Tab. 3) wurden die Daten zur Quantifizierung der GUS-Aktivität in den Antheren (Staubbeuteln) und 'source'-Blättern ausgewählter transgener pFD::GUS Tabakpflanzen zusammengestellt.

**Tabelle 3: Quantifizierung der GUS-Aktivität in den Antheren und 'source'-Blättern ausgewählter transgener pFD::GUS Tabakpflanzen**

| | GUS-Aktivität (pmol [4MU]/mg[protein]/min) | |
|---|---|---|
| pFD :: GUS Pflanze No. | Antheren | 'Source' Blätter |
| pFD5 | 275 | 3785 |
| pFD11 | 174 | 6202 |
| pFD14 | 362 | 2898 |
| pFD15 | 57 | 2678 |

Die Antheren der reifen Blüten zweigen keine Promotoraktivität. Bei geschlossenen Blüten ist sie schwach ausgeprägt.

### e) Ergebnisse der Analyse der Kanamycinresistenz der transgenen Tabakpflanzen

Für die Untersuchung der pFD-Promotor gestützten Vermittlung von Resistenz gegen Kanamycin wurde das pFD-Promotor:NPTII Plasmid in Tabak transformiert. Die selektive Regeneration der Tabakpflänzchen erfolgte auf Kanamycin (100 mg/1). Die aus den sich entwickelnden Sprossknospen regenerierten Pflanzen enthielten das Kanamycin, was zeigte, daß dass der pFD Promotor das NPTII Gen exprimiert hat und somit die Selektion ermöglichte. Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist Selektionsmarker effektiv zu exprimieren und weist eine nur geringe Aktivität im Pollen auf. Die Aktivität in den Antheren beträgt in der Regel weniger als 10% der Aktivität in den Source-Blättern.

### f) Ergebnisse der GUS Analyse der transgenen Kartoffelpflanzen

Das Plasmid pFD::GUS (vgl. Beispiel 3 b) wird entsprechend der in Beispiel 2 beschriebenen Methode in Kartoffeln transformiert. Ergebnis der funktionellen Untersuchungen: Der pFD-Promotor wird stark in den Blättern der analysierten transgenen Kartoffelpflanzen exprimiert. Eine stärkere GUS Färbung in den Blättern der Kartoffelpflanzen als in den Blättern der beschriebenen Tabakpflanzen wurde beobachtet. Schwache Färbung der Blüten bzw. fehlende Färbung der Knollen zeigte geringe Expression in den Blüten und keine Expression in den Knollen.

Die Daten zeigen, dass dieser Promoter keine Aktivität in den Knollen von Kartoffelpflanzen hat und geeignet ist für die Expression von Genen, beispielsweise von Insektiziden, in den Blättern und anderen oberirdischen Organen von Pflanzen, deren Genprodukte in den Speicherorganen unerwünscht sind.

### g) Herstellung von Deletionsvarianten des pFD Promotors

Eine weitere Variante des pFD Promotors stellt die Deletion pFDshort (pFds) dar. Dazu wurde der Abschnitt von Basenpaar 137 bis 837 des pFD Promotors unter Verwendung nachfolgender Primer amplifiziert:
pFD3 (SEQ ID NO: 13):
5'-acggatccgagagacagagagacggagacaaaa-3'
pFD5 (SEQ ID NO: 14) :
5'gcggatccaacactcttaacaccaaatcaaca-3'

| Reaktionsansatz: | |
|---|---|
| 37,5 µl | H₂O |
| 5,0 µl | 10X Reaktionspuffer ("genomic PCR") |
| 4,0 µl | dNTP mix (jeweils 2,5 mM) |
| 2,2 µl | 25 mM Mg (OAc) 2 (Endkonzentration 1,1 mM) |
| 1,0 µl | Primer pFD3 (10 µM) |
| 1,0 µl | Primer pFD5 (10 µM) |
| 0,5 µl | Pfu-turbo Polymerase Mix |
| 1,0 µl | Genomische Arabidopsis DNA (ca. 250 ng) |

| PCR-Bedingungen: | |
|---|---|
| 1 | Zyklus mit 5 min. bei 95°C |
| 25 | Zyklen mit 94°C für 30 sec, 50°C für 60 sec und 72°C für 1 min. |
| 1 | Zyklus mit 50°C für 60 sec, 72°C für 10 min., anschließend Kühlung auf 4°C bis zur Weiterverarbeitung |

Die Primer enthielten Erkennungssequenzen für das Restriktionsenzym BamHI. Nach Spaltung des PCR Produktes mit BamHI wurde es in das Plasmid pGUSINT37 (s.o.), das ebenfalls BamHI geschnitten und dephosphoryliert wurde, ligiert. Mit dem resultierenden Konstrukt pFDsGUSINT wurden Tabakblätter mit der Biolistics (BioRad) beschossen. Dabei wurden Microcarrier (25 µg Gold, Heraeus 0,3 bis 3 µm) mit 10 µg Plasmid DNA, 2,5 M CaCl₂, und 0,1 M Spermidine behandelt, mit Alkohol gewaschen und bei einem Vakuum von 26 inch und einem Druck von 1100 psi auf die Blätter, die auf MS-Medium lagen, geschossen. Die Explantate wurden anschließend für 24 h in MS-Medium mit 2% Sucrose inkubiert und dann mit X-gluc histochemisch gefärbt. Blaue Spots zeigten die Aktivität des Promotors.

### h) Fusion des pFds Promotors mit dem NPTII Gen

Als BamHI Fragment wird der pFDs Promotor aus pFDsGUSINT herausgeschnitten und seine Enden mittels Klenow-"Fill-In" geglättet. das erhaltene Fragment wird vor das NPTII Gens des Plasmids pSUNSNPTIICat (SEQ ID NO: 24), EcoRV geschnitten und dephosphoryliert, kloniert. Das Plasmid pSUN5NFTII ist ein Derivat des Plasmids pSUN3 (SEQ ID NO: 23), welches neben einer nosP/Pat Kassette noch ein promotorloses NPTII Gen enthält. Mit diesem Konstrukt ist es möglich Promotoren auf ihre Fähigkeit zurdie Expression von NPTII zu testen. Parallel kann die Selektion auf Phosphinothricin enthaltenden Medium erfolgen.

Das resultierende Plasmid pSun5FdsNPTII wird in Tabak transformiert. Regenerierte und selektierte Sprosse zeigten, dassß der pFDs Promotor die Selektion auf NPTII erlaubt.

### Vergleichsbeispiel 4: Untersuchungen zur Eignung des Ferredoxin NADPH Oxidoreduktase (FNR) Promotors

### a) Klonierung des FNR Promotors aus Arabidopsis thaliana

Die putative Promotorregion des FNR-Gens wurde unter Verwendung der Oligonukleotid-primer L-FNRara und R-FNRara aus genomischer DNA amplifiziert wobei das ATG-Startcodon des FNR-Gens ausgespart und vier putative Stop-Codons des stromaufwärts gelegenen offenen Leserahmens beibehalten wurden. Anhand der Primer L-FNRara und R-FNRara wurde der FNR-Promotor als ein 635 bp großes Fragment entsprechend dem Abschnitt des Klons K2A18.15 von Position 69493 bis Position 70127 (beide Nukleotide inklusive) mittels PCR aus genomischer *Arabidopsis thaliana* DNA amplifiziert. Der Primer L-FNRara beginnt mit den in Fettdruck hervorgehobenen *Sal*I und *Bam*HI Restriktionsschnittstellen und befindet sich stromaufwärts vor den vier Stop-Codons des dem FNR-Promotor vorgeschalteten Gens. Der Primer R-FNRara beginnt mit den in Fettdruck hervorgehobenen *Sal*I und *Xba*I Restriktionsschnittstellen unmittelbar vor dem ATG-Startcodon des FNR-Gens.
Primer L-FNR ara (44 mer) (SEQ ID NO: 15):
   5' **GTC GAC GGA TCC** GGT TGA TCA GAA GAA GAA GAA GAA GAT GAA CT 3'
Primer R-FNR ara (41 mer) (SEQ ID NO: 16):
   5' **GTC GAC TCT AGA** TTC ATT ATT TCG ATT TTG ATT TCG TGA CC 3'

Zur Amplifizierung des FNR-Promotors wurde ein sogenanntes 'touchdown' PCR-Protokoll unter Verwendung des ,Advantage Genomic Polymerase Mix' (Clontech Laboratories, Inc; Katalog-Nr. #8418-1) benutzt. Der obengenannte Polymerase-Mix enthält eine thermostabile DNA-Polymerase aus *Thermus thermophilus* (*Tth* DNA Polymerase), gemischt mit einem kleineren Anteil der "Vent Proofreading 3'-5' Polymerase", und den *Tth* Start Antikörper, der "hot-start" PCR ermöglicht.

| Reaktionsansatz: | |
|---|---|
| 36, µl | H₂O |
| 5 µl | 10X Reaktionspuffer ("genomic PCR") |
| 1 µl | dNTP mix (jeweils 102,5 mM) |
| 2,2 µl | 25 mM Mg(OAc)₂ (Endkonzentration 1,1 mM) |
| 1 µl | Primer L-FNR ara (10 µM) |
| 1 µl | Primer R-FNR ara (10 µM) |
| 1 µl | 50x Polymerase Mix |
| 2 µl | Genomische *Arabidopsis* DNA (ca. 500 ng) |

| PCR-Bedingungen: | |
|---|---|
| 1 | Zyklus mit 1 min. bei 94°C |
| 10 | Zyklen mit 94°C für 30 sec und 70°C für 3 min. |
| 32 | Zyklen mit 94°C für 30 sec und 65°C für 3 min. |
| 1 | Zyklus mit 65°C für 4 min., anschließend Kühlung auf 4°C bis zur Weiterverarbeitung. |

### b) Konstruktion der FNR-Promotor-GUS-Expressionskassette

Das FNR-Promotor PCR-Produkt wurde nach Gel-elektrophoretischer Auftrennung und Reinigung aus dem Gel mit dem Quiagen-PCR-Reinigungskit per TA-Klonierung in den Vektor pCRII (Invitrogen) kloniert. Das Promotorfragment wurde anschließend aus dem resultierenden Plasmid pATFNR1 mittels der durch das Primerpaar eingeführten *Xba*I und *Bam*HI-Restriktionsschnittstellen durch Verdau mit XbaI/BamHI isoliert und gelelektrophoretisch gereinigt. Für die Fusion mit dem GUS Gen wurde das ca. 600bp umfassende FNR-Promotorfragment in den *Xba*I/*Bam*HI-restringierten Binärvektor pBI101 kloniert. Die korrekte Insertion des richtigen Fragments im resultierenden Plasmid pATFNR-Bi wurde anschließend anhand einer Restriktrionsanalyse unter Verwendung der Endonukleasen *Eco*RV verifiziert. Das Plasmid pATFNR-Bi wurde zur Tabaktransformation verwendet.

Für die Transformation in Raps wurde der FNR Promoter als SalI Fragment des Plasmids pCR_ATFNR in den mit SalI und XhoI geschnittenen Vektor pS3NitGUS, den Nitrilase Promotor ersetzend, kloniert.

### c) Konstruktion der FNR-Promotor-PAT-Expressionskassette

Für die Untersuchung der FNR-Promotor gestützten Vermittlung von Resistenz gegen Phosphinothricin wurde der FNR-Promotor als *SalI* Fragment aus dem Plasmid pATFNRI vor das Phosphinothricin Resistenzgen in das *SalI* geschnittene Plasmid pSUN3PatNos (SEQ ID NO: 25) kloniert.

### d) Konstruktion der FNR-Promoter-NptII-Expressionskassette

Für die Vermittlung von Resistenz gegen Kanamycin wurde der FNR Promotor als *Sal*I Fragment vor das NptII-Resistenzgen in das *XhoI* geschnittene, dephosphorylierte Plasmid pSUN5NptIICat (Sungene GmbH & Co KGaA, SEQ ID NO: 24) kloniert. Das resultierende Plasmid mit der Bezeichnung pS5FNRNptII wurde in Tabak und in Raps transformiert.

### e) Ergebnisse der GUS Analyse der transgenen Tabakpflanzen

### Qualitative Daten:

Transgene FNR::GUS-TabakPpflanzen zeigten starke GUS-Expression in allen grünen Geweben, vor allem in, source'-Blättern, Blattstielen und Internodien sowie allen Blütenorganen voll entwickelter Blüten (Ovarium, Stigma, Kelch- und Kronblätter) mit Ausnahme von Pollen, der keine GUS-Aktivität zeigte; eine geringe Färbungsintensität wurde in Antheren detektiert. Bei der ersten Analyse von Blattscheibchen von 80 *in vitro*-Pflanzen zeigten 70 Pflanzen eine starke GUS-Färbung mit geringer Variation in der Färbeintensität zwischen den individuellen Pflanzen. Das wurde als Hinweis darauf gewertet, daß dass der FNR-Promotor ein Element enthält, daß dassfür limitierte Positionseffekte sorgt. In den Gewebekulturpflanzen war die GUS-Aktivität der FNR::GUS Pflanzen deutlich geringer als die der TPT::GUS Pflanzen. Transgene Rapspflanzen zeigten das gleiche Färbemuster.

Samenmaterial (F1) der Linien FNR 13, FNR 45 und FNR 28 wurden bezüglich seinerihrer GUS-Aktivität analysiert. Dabei zeigte sich, dassß weder in ruhenden Samen noch in ausgebrachten Keimlingen (3.5 Tage nach Aussaat) GUS-Aktivität detektiert werden konnte. In späteren Keimlingsstadien (6 und 10 Tage nach Aussaat) wurde starke GUS-Aktivität in Keimblättern und im oberen Bereich der Keimlingsachse detektiert, wogegen keine GUS-Färbung in den Wurzeln gefunden wurde. Bei Keimlingen, die im Dunkeln angezogen wurden, war die GUS-Aktivität auf die Kotyledonen beschränkt und war insgesamt geringer als bei lichtgekeimten Sämlingen.

In späteren Keimlingsstadien (6 und 10 Tage nach Aussaat) wurde starke GUS-Aktivität in Keimblättern und im oberen Bereich der Keimlingsachse detektiert, wogegen keine GUS-Färbung in den Wurzeln gefunden wurde. Bei Keimlingen, die im Dunkeln angezogen wurden, war die GUS-Aktivität auf die Kotyledonen beschränkt und war insgesamt geringer als bei lichtgekeimten Sämlingen.

### Quantitative Daten;

Die quantitative Untersuchung der GUS-Aktivität in FNR::GUS transgenen Tabakpflanzen (transformiert mit Plasmid pATFNR-Bi) wurde an den ersten voll entwickelten Blättern von Tabakpflanzen 21 Tage nach dem Transfer von der Gewebekultur ins Gewächshaus analysiert. Die Daten entsprechen dem Mittelwert von vier unabhängigen Messungen.

**Tabelle 4: Quantifizierung der GUS-Aktivität in Blaättermaterial ausgewählter transgener FNR::GUS Tabakpflanzen (transformiert mit PlasmidpATFNR-Bi)**

| FNR::GUS No. der Pflanze | Rang (x-stärkste GUS-Aktivität unter 50 Pflanzen) | GUS Aktivität (pmol 4-MU/mg Protein/min) | Standardab weichung |
|---|---|---|---|
| 13 | 1 | 86,491 | 2974 |
| 45 | 2 | 41,726 | 1829 |
| 14 | 7 | 23,951 | 2443 |
| 28 | 9 | 22,148 | 401 |
| 17 | 10 | 21,557 | 1157 |
| 30 | 20 | 13,444 | 744 |
| 40 | 26 | 11,972 | 1144 |
| 25 | 35 | 7,662 | 519 |
| 35 | 39 | 5,643 | 96 |
| 21 | 43 | 2,858 | 194 |
| C2- (WT) | 49 | 2,8 | 4 |

### f) Ergebnisse der Analyse der Phosphinothricinresistenz der transgenen Tabakpflanzen

Das Plasmid pSUN3FNRPat wurde unter Verwendung des Agrobacterium tumefaciens Stamm EHA101 zur Tabaktransformation, wie unter 3. beschrieben, verwendet. Die selektive Regeneration der Tabakpflänzchen erfolgte einerseits auf Phosphinothricin (5 mg/l) und andererseits zur Kontrolle auf Kanamycin (100 mg/l). 97% der Explantate, die unter Kanamycindruck (nosP:NPTII) und 40% der Explantate, die unter Phosphinothricindruck (FNR:Bar) selektiert wurden, entwickleten Sprossknospen. Die unter Phosphinotricindruckdaraus regenerierten Pflanzen enthielten sowohl das Kanamycin als auch das Phosphinothrieingen was zeigte, dass der FNR Promotor das Phosphinothricinacetyltransferase Gen exprimiert hat und somit die Selektion ermöglichte. Samen der transgenen Tabakpflanzen wurden auf MS Medium, welches 10 mg/l Phosphinothricin enthielt, ausgelegt und die Keimungsrate bestimmt. Im Gegensatz zur Kontrolle mit nicht transformierten Tabaksamen entwickelten sich die Keimlinge normal. In den Nachkommenschaften der Linien wurde das übertragene und durch den FNR Promotor exprimierte Gen der Phosphinothricinacetyltransferase mittels PCR nachgewiesen. Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist, Selektionsmarker effektiv zu exprimieren und weist keine Aktivität im Pollen auf.

### g) Ergebnisse der Analyse der Kanamycinresistenz der transgenen Tabak- und Rapspflanzen

Für die Untersuchung der FNR-Promotor gestützten Vermittlung von Resistenz gegen Kanamycin wurde der FNR-Promotor mit dem NptII Gen kombiniert. Das resultierende Konstrukt pS5FNRNptII wurde in den Agrobakterium tumefaciens Stamm GV3101[mp90] für die Transformation in Tabak und Raps transformiert.

Samen der transgenen Tabakpflanzen wurden auf MS Medium, welches 100 mg/l Kanamycin enthielt, ausgelegt und die Keimungsrate bestimmt. Im Gegensatz zur Kontrolle mit nicht transformierten Tabaksamen entwickelten sich die Keimlinge normal. In den Nachkommenschaften der Linien wurde das übertragene und durch den FNR Promotor exprimierte Gen der Neomycin Phosphotransferase (nptII) mittels PCR nachgewiesen.

Die resultierenden Stämme sind für die Transformation, wie unter Beispiel 2 beschrieben, eingesetzt worden. Die selektive Regeneration wurde in Gegenwart von 100 mg/l (bzw. 18 mg/l für Raps) Kanamycin erreicht. Die unter Kanamycindruck regenerierten Pflanzen enthielten sowohl das Kanamycin als auch das Phosphinothricingen was zeigte, dass der FNR Promotor das NptII Gen exprimiert hat und somit die Selektion der Pflanzen ermöglichte.

Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist, Selektionsmarker effektiv zu exprimieren und weißt keine Aktivität im Pollen auf.

### h) Ergebnisse der GUS Analyse der transgenen Kartoffelpflanzen

Die Analyse von putativ transgenen Kartoffelpflanzen zeigte bei 20 Linien eine starke GUS Färbung in den Blättern, vergleichbar mit dem Expressionspattern der Tabakpflanzen. Mit der Ausnahme von 5 Pflanzen, die eine sehr schwache Färbung in den Kartoffelknollen aufwiesen, konnte keine Expression des FNR Promoters in den verbleibenden Pflanzen nachgewiesen werden.

Die Daten zeigen, dass dieser Promoter keine oder sehr schwache Aktivität in den Speicherorganen von Kartoffelpflanzen hat und geeignet ist für die Expression von Genen, beispielsweise von Insektiziden, in den Blättern und anderen oberiridschen Organen von Pflanzen, deren Genprodukte in den Speicherorganen unerwünscht sind.

### Beispiel 5: Untersuchungen zur Eignung des Triose-Phosphat Translokator (TPT) Promotors

### a) Klonierung des TPT Promotor aus Arabidopsis thaliana

Die putative Promotor-Region des TPT-Gens aus Arabidopsis thaliana wurde durch Amplifizierung mit den Oligonukleotid-Primern L-TPTara und R-TPTara isoliert, wobei das ATG-Startcodon des TPT-Gens ausgespart wurde. Anhand der Primer L-TPTara und R-TPTara wurde der TPT-Promotor als ein 20398 bp großes Fragment mittels PCR aus genomischer *Arabidopsis thaliana* DNA amplifiziert (SEQ ID NO: 3), Der Primer L-TPTara beginnt mit den in Fettdruck hervorgehobenen *Sal*I und *Bam*HI Restriktionsschnittstellen. Der Primer R-TPTara beginnt mit den in Fettdruck hervorgehobenen *Aat*II, *Sal*I und *Xba*I Restriktionsschnittstellen unmittelbar vor dem ATG-Startcodon des TPT-Gens.
Primer L-TPTara (SEQ ID NO: 17):
   5' AAG TCG ACG GAT CCA TAA CCA AAA GAA CTC TGA TCA TGT ACG TAC CCA TT 3'
Primer R-TPTara (SEQ ID NO: 18):
   5' AGA CGT CGA CTC TAG ATG AAA TCG AAA TTC AGA GTT TTG ATA GTG AGA GC 3'

Zur Amplifizierung des TPT-Promotors wurde ein sogenanntes 'touchdown' PCR-Protokoll unter Verwendung des ,Advantage Genomic Polymerase Mix' (Clontech Laboratories, Inc; Katalog-Nr. #8418-1) benutzt. Der obengenannte Polymerase-Mix enthält eine thermostabile DNA-Polymerase aus *Thermus thermophilus* (Tth DNA polymerase), gemischt mit einem kleineren Anteil der Vent proofreading 3'-5' Polymerase, und demn *Tth* Start Antikörper, der "hot-start" PCR ermöglicht.

| Reaktionsansatz: | |
|---|---|
| 36,8 µl | H₂O |
| 5 µl | 10X Reaktionspuffer ("genomic PCR") |
| 1 µl | dNTP mix (jeweils 102,5 mM) |
| 2,2 µl | 25mM Mg(OAc)₂ (Endkonzentration 1,1 mM) |
| 1 µl | Primer L-FNR ara (10 µM) |
| 1 µl | Primer R-FNR ara (10 µM) |
| 1 µl | 50x Polymerase Mix |
| 2 µl | Genomische *Arabidopsis* DNA (ca. 500 ng) |

| PCR-Bedingungen: | |
|---|---|
| 1 | Zyklus mit 1 min. bei 94°C |
| 10 | Zyklen mit 94°C für 30 sec und 70°C für 3 min. |
| 32 | Zyklen mit 94°C für 30 sec und 65°C für 3 min. |
| 1 | Zyklus mit 65°C für 4 min., anschließend Kühlung auf 4°C bis zur Weiterverarbeitung. |
| | |
| 10 | Zyklen mit 94°C für 30 sec und 70°C für 3 min. |
| 32 | Zyklen mit 99°C für 30 sec und 65°C für 3 min. |
| 1 | Zyklus mit 65°C für 4 min., anschließend Kühlung auf 4°C bis zur Weiterverarbeitung, |

### b) Konstruktion der TPT-Promotor-GUS-Expressionskassette

Das TPT-Promotor PCR-Produkt wurde nach Gel-elektrophoretischer Auftrennung und Reinigung aus dem Gel mit dem Quiagen-PCR-Reinigungskit per TA-Klonierung in den Vektor pCRII (Invitrogen) kloniert. Das Promotorfragment wurde anschließend aus dem resultierenden Plasmid pATTPT mittels der durch das Primerpaar eingeführten *Sal*I und *Xba*I -Restriktionsschnittstellen isoliert und gelelektrophoretisch gereinigt. Für die Fusion mit dem GUS Gen wurde das ca. 2,.20 kb umfassende TPT-Promotorfragment in den *Sal*Il*Xba*I -restringierten Binärvektor pBI101 kloniert. Die korrekte Insertion des richtigen Fragments im resultierenden Plasmid pATTPT-Bi wurde anschließend anhand einer Restriktrionsanalyse unter Verwendung der Endonukleasen *Hin*dIII verifiziert. Das Plasmid pATTPT-Bi wurde zur Tabaktransformation verwendet.

Für die Transformation in Raps wurde der TPT Promoter als SalI Fragment des Plasmids pATTPT in den mit SalI und XhoI geschnittenen Vektor pS3NitGUS, den Nitrilase Promotor ersetzend, kloniert.

### c) Konstruktion der TPT-Promotor-PAT-Expressionskassette

Für die Untersuchung der TPT-Promotor gestützten Vermittlung von Resistenz gegen Phosphinothricin wurde der TPT-Promotor als *Sal*I Fragment aus dem Plasmid pATTPT vor das Phosphinothricin Resistenzgen in das *SalI* geschnittene Plasmid pSUN3PatNos kloniert. Das resultierende plasmid pSUN3TPTPat wurde unter Verwendung des *Agrobacterium tumefaciens* Stamm EHA101 zur Tabaktransformation verwendet. Die selektive Regeneration der Tabakpflänzchen erfolgte einerseits auf Phosphinothricin (5 mg/l) und andererseits zur Kontrolle auf Kanamycin (100 mg/l).

### d) Konstruktion der TPT-Promoter-NptII-Expressionskassette

Für die Vermittlung von Resistenz gegen Kanamycin wurde der TPT Promotor als *Sal*I Fragment vor das NptII-Resistenzgen in das *XhoI* geschnittene, dephosphorylierte Plasmid pSun5NptIICat (Sungene GmbH & Co KGaA; SEQ ID NO: 24) kloniert. Das resultierende Plasmid mit der Bezeichnung pS5TPTNptII wurde in Tabak und in Raps transformiert.

### e) Ergebnisse der GUS Analyse der transgenen Tabakpflanzen

### Qualitative Daten

Transgene TPT::GUS-TabakpPflanzen zeigten starke GUS-Expression in allen grünen Geweben, vor allem in 'source'-Blättern, hier insbesondere in den Trichomen und den Blütenorganen junger und voll entwickelter Blüten. Die GUS-Aktivität im Bereich der Blüten war am stärksten in den Ovarien und im Stigma; die Färbung der Kelch- und Kronblätter war etwas schwächer. In den Antheren war die GUS-Aktivität am geringsten und schwer zu detektieren. Im Pollen was keine GUS-Aktivität zu detektieren. Transgene Rapspflanzen zeigten das gleiche Färbemuster.

Bei der ersten Analyse von Blattscheibchen von 80 *in vitro-*Pflanzen zeigten 22 Pflanzen überhaupt keine Färbung und 22 Pflanzen eine starke GUS-Färbung nach nur dreistündiger Färbung. Die übrigen Pflanzen zeigten eine große Bandbreite verschieden intensiver GUS-Färbungen bei den individuellen Pflanzen. In den Gewebekulturpflanzen war die GUS-Aktivität der TPT::GUS Pflanzen deutlich stärker als die der FNR::GUS Pflanzen. Samenmaterial (F1) der Linien TPT 55 und TPT 60 wurden bezüglich ihrer GUS-Aktivität analysiert. Dabei zeigte sich, daß dass sowohl in ruhenden Samen als auch in ausgebrachten Keimlingen (3.5 Tage nach Aussaat) eine starke GUS-Aktivität detektiert werden konnte. In späteren Keimlingsstadien (6 und 10 Tage nach Aussaat) wurde die stärkste GUS-Aktivität in Keimblättern und im oberen Bereich der Keimlingsachse detektiert und eine schwächere GUS-Färbung in den Wurzeln. Keimlinge, die im Dunkeln angezogen wurden, zeigten ein unverändertes GUS-Färbemuster.

### Quantitative Daten

Die quantitative Untersuchung der GUS-Aktivität in TPT::GUS transgenen Tabakpflanzen (transformiert mit PlasmidpAT-TPT-Bi) wurde an den ersten voll entwickelten Blättern von Tabakpflanzen 19 Tage nach dem Transfer von der Gewebekultur ins Gewächshaus analysiert. Die Daten entsprechen dem Mittelwert von vier unabhängigen Messungen.

**Tabelle 5: Quantifizierung der GUS-Aktivität in Blattgewebe ausgewählter transgener TPT::GUS Tabakpflanzen (transformiert mit PlasmidpAT-TPT-Bi) . WT2: Kontrollwerte von untransformierten Wildtyp-Pflanzen.**

| TPT::GUS No. der Pflanze | Rang (x-stärkste GUS-Aktivität unter 50 Pflanzen) | GUS Aktivität (pmol 4-MU/mg Protein/min) | Standardab weichung |
|---|---|---|---|
| 55 | 1st | 62.910 | 3576 |
| 15 | 2nd | 58.251 | 2533 |
| 10 | 5th | 36.759 | 1008 |
| 60 | 10th | 19.536 | 1783 |
| 56 | 11th | 18.876 | 1177 |
| 43 | 12th | 18.858 | 1404 |
| 27 | 35th | 7.390 | 233 |
| 44 | 59th | 311 | 24 |
| 80-WT2 | 80th | 5 | 13 |

### f) Ergebnisse der GUS Analyse der transgenen Kartoffelpflanzen

Die Analyse von putativ transgenen Kartoffelpflanzen zeigte bei 28 Linien eine starke GUS Färbung in den Blättern, vergleichbar mit dem Expressionspattern der Tabakpflanzen. In den Kartoffelknollen der transgenen Pflanzen wurde ebenfalls eine starke Färbung nachgewiesen. Dies zeigt, daß der TPT Promotor auch in Kartoffeln stark und ubiquitär exprimiert wird.

### g) Ergebnisse der Analyse der Phosphinothricinresistenz der transgenen Tabakpflanzen

Das Plasmid pSUN3TPTPat wurde unter Verwendung des Agrobacterium tumefaciens Stamm EHA101 zur Tabaktransformation, wie unter 3. beschrieben, verwendet. Die selektive Regeneration der Tabakpflänzchen erfolgte einerseits auf Phosphinothricin (5 mg/1) und andererseits zur Kontrolle auf Kanamycin (100 mg/1). 97% der Explantate, die unter Kanamycindruck und 70% der Explantate, die unter Phosphinothricindruck selektiert wurden, entwickelten Sproßknospen. Die unter Phosphinotricindruckdaraus regenerierten Pflanzen enthielten sowohl das Kanamycin als auch das Phosphinothricingen was zeigte, dassß der TPT Promotor das Phosphinothricinacetyltransferase Gen exprimiert hat und somit die Selektion ermöglichte. Samen der transgenen Tabakpflanzen wurden auf MS Medium, welches 10 mg/l Phosphinothricin enthielt, ausgelegt und die Keimungsrate bestimmt. Im Gegensatz zur Kontrolle mit nicht transformierten Tabaksamen entwickelten sich die Keimlinge normal. In den Nachkommenschaften der Linien wurde das übertragene und durch den TPT Promotor exprimierte Gen der Phosphinothricinacetyltransferase mittels PCR nachgewiesen. Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist Selektionsmarker effektiv zu exprimieren und weist keine Aktivität im Pollen auf.

### h) Ergebnisse der Analyse der Kanamycinresistenz der transgenen Tabak- und Rapspflanzen

Für die Untersuchung der TPT-Promotor gestützten Vermittlung von Resistenz gegen Kanamycin wurde der TPT-Promotor mit dem NptII Gen kombiniert. Das resultierende Konstrukt pS5TPTNptII wurde in den Agrobakterium tumefaciens Stamm GV3101[mp90] für die Transformation in Tabak und Raps transformiert.

Die resultierenden Stämme sind für die Transformation, wie unter Beispiel 2 beschrieben, eingesetzt worden. Die selektive Regeneration wurde in Gegenwart von 100 mg/l (bzw. 16 mg/l für Raps) Kanamycin erreicht. Die unter Kanamycindruck regenerierten Pflanzen enthielten sowohl das Kanamycin als auch das Phosphinothricingen was zeigte, dass der TPT Promotor das NptII Gen exprimiert hat und somit die Selektion der Pflanzen ermöglichte.

Samen der transgenen Tabakpflanzen wurden auf MS Medium, welches 100 mg/l Kanamycin enthielt, ausgelegt und die Keimungsrate bestimmt. Im Gegensatz zur Kontrolle mit nicht transformierten Tabaksamen entwickelten sich die Keimlinge normal. In den Nachkommenschaften der Linien wurde das übertragene und durch den TPT Promotor exprimierte Gen der Neomycin Phosphotransferase (nptII) mittels PCR nachgewiesen.

Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist, Selektionsmarker effektiv zu exprimieren und weißt keine Aktivität im Pollen auf.

### i) Klonierung des verkürzten TPT Promotors (STPT)

Die verkürzte putative Promotorregion des TPT-Gens (STPT) aus Arabidopsis thaliana wurde durch Amplifikation mittels PCR unter Verwendung der Primer 5-TPTara (SEQ-ID NO: 26) und R-TPTara (s.o., SEQ ID NO: 18) aus dem Plasmid pATTPT isoliert (SEQ ID NO: 27).

| Reaktionsansatz: | |
|---|---|
| 37,8 µl | H₂O |
| 5 µl | 10X Reaktionspuffer ("genomic PCR") |
| 1 µl | dNTP mix (jeweils 2,5 mM) |
| 2,2 µl | 25 mM Mg (OAc)₂ (Endkonzentration 1,1 mM) |
| 1 µl | Primer 5-TPTara (10 µM) |
| 1 µl | Primer R-TPTara (10 µM) |
| 1 µl | 50x Polymerase Mix ("Advantage Genomic Polymerase Mix"; Clontech Lab., Inc.; Kat.-Nr.: #8418-1) |
| 1 µl | pATTPT Plasmid DNA (1 ng) |

| PCR-Bedingungen: | |
|---|---|
| 1 | Zyklus mit 5 min. bei 94°C |
| 25 | Zyklen mit 94°C für 30 sec und 52°C für 1 min. |
| 1 | Zyklus mit 52°C für 4 min., anschließend Kühlung auf 4 °C bis zur Weiterverarbeitung. |

Primer 5-TPTara (SEQ-ID NO: 26)
   5'-AAG TCG ACG GAT CCT-GAT-AGC-TTA-TAC-TCA-AAT-TCA-ACA-AGT-TAT-3'

### Vergleichsbeispiel 1: Untersuchungen zur Eignung des Ubiquitin-Promotors

Das 1,3 kb große PCR-Produkt des verkürzten TPT-Promotors wurde nach gelelektrophoretischer Auftrennung und Reinigung aus dem Gel mit dem "SureClone Ligation Kit" (Amersham Pharmacia Biotech; Code-Nr.: 27-9300-01) in den SmaI geschnittenen und dephosphorylierten Vektor pUC18 kloniert. Das resultierende Plasmid trägt die Bezeichnung pATSTPT. Die Sequenz wurde mittels Sequenzierung überprüft.

### j) Konstruktion der STPT-Promoter-NptII-Expressionskassette

Für die Vermittlung von Resistenz gegen Kanamycin wurde der STPT Promotor (SEQ ID NO: 27) als *SalI* Fragment vor das NptII-Resistenzgen in das *XhoI* geschnittene, dephosphorylierte Plasmid pSun5NptIICat (Sungene GmbH & Co KGaA; SEQ ID NO: 24) kloniert. Das resultierende Plasmid mit der Bezeichnung pSSSTPTNptII wurde in Tabak und in Raps transformiert.

### k) Ergebnisse der Analyse der Kanamycinresistenz der transgenen Tabak- und Rapspflanzen

Für die Untersuchung der STPT-Promotor gestützten Vermittlung von Resistenz gegen Kanamycin wurde das Plasmid pS5STPTNptII in den Agrobakterium tumefaciens Stamm GV3101[mp90] für die Transformation in Tabak und Raps transformiert. Der resultierenden Stamm ist für die Transformation, wie unter Beispiel 2 beschrieben, eingesetzt worden. Die selektive Regeneration wurde in Gegenwart von 100 mg/l (bzw. 18 mg/l für Raps) Kanamycin erreicht.

Die Ergebnisse demonstrieren, dass die isolierte Nukleinsäuresequenz die gewünschten vorteilhaften Promotoreigenschaften besitzt, d.h. sie zeigt eine Promotoraktivität, die geeignet ist, Selektionsmarker effektiv zu exprimieren.

### Beispiel 6: Vergleich der Transformationseffizienz von FNR-und TPT-Promotor mit dem NOS-Promotor

In einem vergleichenden Experiment wurde die Effizienz bei der Transformation von Tabak unter der Verwendung des FNR-Promotors (FNR-P), des TPT-Promotors (TPT-P) und des Nos-Promotors (Nos-P) bestimmt. Die Promotoren waren, wie beschrieben, mit dem NptII Gen jeweils fusioniert. Nach erfolgter Sproßknospenbildung bzw, der Bewurzelung der Sprosse auf kanamycinhaltigen Medium wurden die resistenten Transformanten gezählt und deren Zahlen verglichen. Eine PCR, mit der das NptII Gen nachgewiesen wurde, zeigte den hohen Anteil der transgenen Pflanzen.

| | **NOS-P** | **FNR-P** | **TPT-P** |
|---|---|---|---|
| Sprossbildung | 100 % | 68 % | 76 % |
| Bewurzelte Pflanzen | 80 % | 81 % | 80 % |
| Transgene Pflanzen | 92 % | 100 % | 100 % |

### Tabelle 6: Transformationseffizienz

### Vergleichsbeispiel 7: Untersuchungen zur Eignung des Ubiquitin-Promotors

### a) Klonierung des Ubiquitin Promotor aus Arabidopsis thaliana

Der Ubiquitin Promotor wurde mittels PCR aus genomischer *Arabidopsis thaliana* DNA mit den Primern ubi5 und ubi3 amplifiziert.
ubi5 (SEQ ID NO: 19) :
5'-CCAAACCATGGTAAGTTTGTCTAAAGCTTA-3'
ubi3 (SEQ ID NO: 20):
5'-CGGATCCTTTTGTGTTTCGTCTTCTCTCACG-3'

| Reaktionsansatz: | |
|---|---|
| 37,5 µl | H₂O |
| 5 µl | 10X Reaktionspuffer ("genomic PCR") |
| 4 µl | dNTP mix (jeweils 2,5 mM) |
| 2,2 µl | 25 mM Mg(CAc)₂ (Endkonzentration 1, 1 mM) |
| 1 µl | Primer ubi3 (10 µM) |
| 1 µl | Primer ubi5 (10 µM) |
| 0,5 µl | Pfu-turbo polymerase mix |
| 1 µl | Genomische *Arabidopsis* DNA (ca. 250 ng) |

| PCR-Bedingungen: | |
|---|---|
| 1 | Zyklus mit 5 min. bei 94°C |
| 25 | Zyklen mit 94°C für 30 sec, 52°C für 1 min. und 72°C für 1 min. |
| 1 | Zyklus mit 52°C für 1 min. und 72°C für 10 min., anschließend Kühlung auf 4°C bis zur Weiterverarbeitung. |
| 25 | Zyklen mit 94°C für 30 sec, 52°C für 1 min. und 72°C für 1 min. |
| 1 | Zyklus mit 52°C für 1 min. und 72°C für 10 min., anschließend Kühlung auf 4°C bis zur Weiterverarbeitung. |

Das entstandene PCR Fragment wurde als *Hin*dIII/*Bam*HI Fragment in das mit *Hin*dIII/*Bam*HI geschnittene Plasmid pGUSINT37 kloniert (pUBI42GUS) und mittels Sequenzanalyse verifiziert.

### b) Klonierung des Ubiquitin Promotors vor das PAT-Gen

Für die Untersuchung der Ubiquitin-Promotor gestützten Vermittlung von Resistenz gegen Phosphinothricin wurde der Ubiquitin -Promotor als *Bam*HI/*Hin*dIII Fragment vor das Phosphinothricin Resistenzgen in das *Bam*HI/*Hin*dIII geschnittene Plasmid pSUN3PatNos kloniert. Das resultierende Plasmid pSUN3UBIPat wurde unter Verwendung des *Agrobacterium tumefaciens* Stamm EHA101 zur Tabaktransformation verwendet. Die selektive Regeneration der Tabakpflänzchen erfolgte einerseits auf Phosphinothricin (5 mg/1) und andererseits zur Kontrolle auf Kanamycin (100 mg/l).

### c) Ergebnisse der Analyse der Phosphinothricinresistenz der transgenen Tabakpflanzen

Im Gegensatz zur Selektion auf Kanamycin, die normal verlief, konnten unter Selektion auf Phosphinothricin keine Kalli oder Sprosse erhalten werden. So ist der Ubiquitin Promotor nicht geeignet für die Expression eines selektiven Markers für den *Agrobacterium tumefaciens*-vermittelten Gentransfer mit anschließender Regeneration von Geweben.

### Vergleichsbeispiel 8: Untersuchungen zur Eignung des Squalen Synthase (SQS) Promotors

### a) Klonierung des Squalen Synthase (SQS) Promotors aus Arabidopsis thaliana

Der Squalen Synthase Promotor wurde mittels PCR aus genomischer *Arabidopsis* thaliana DNA mit den Primern sqs5 und sqs3 amplifiziert.
sqs5 (SEQ ID NO: 21):
5'-GTCTAGAGGCAAACCACCGAGTGTT-3'
sqs3 (SEQ ID NO: 22);
5'-CGGTACCTGTTTCCAGAAAATTTTGATTCAG-3'

| Reaktionsansatz: | |
|---|---|
| 37,5 µl | H₂O |
| 5 µl | 10X Reaktionspuffer ("genomic PCR") |
| 4 µl | dNTP mix (jeweils 2,5 mM) |
| 2,2 µl | 25 mM Mg(OAc)₂ (Endkonzentration 1,1 mM) |
| 1 µl | Primer sqs3 (10 µM) (10 µM) |
| 1 µl | Primer sqs5 (10 µM) |
| 0,5 µl | Pfu-turbo polymerase mix |
| 1 µl | Genomische *Arabidopsis* DNA (ca. 250 ng) |

| PCR-Bedingungen: | |
|---|---|
| 1 | Zyklus mit 5 min. bei 94°C |
| 25 | Zyklen mit 94°C für 30 sec, 52°c für 1 min. und 72°C für 1 min. |
| 1 | Zyklus mit 52°C für 1 min. und 72°C für 10 min., anschließend Kühlung auf 4°C bis zur Weiterverarbeitung. |
| 25 | Zyklen mit 94°C für 30 sec, 52°C für 1 min. und 72°C für 1 min, |
| 1 | Zyklus mit 52°C für 1 min. und 72°C für 10 min., anschließend Kühlung auf 4°C bis zur Weiterverarbeitung. |

Das entstandene PCR Fragment wurde als *Xba*II/*Bam*HI Fragment in das mit *Xba*II/*Bam*HI geschnittene Plasmid pGUSINT37 kloniert (pSQSPGUS) und mittels Sequenzanalyse verifiziert.

### b) Klonierung des Squalen Synthase Promotors vor das PAT-Gen

Für die Untersuchung der Squalen Synthase -Promotor gestützten Vermittlung von Resistenz gegen Phosphinothricin wurde der Squalen synthase-Promotor als *Bam*HI/*Sal*I Fragment vor das Phosphino-thricin Resistenzgen in das *Bam*HI/*Sal*I geschnittene Plasmid pSUN3PatNos kloniert. Das resultierende Plasmid pSUN3SQSPat wurde unter Verwendung des *Agrobacterium tumefaciens* Stamm EHA101 zur Tabaktransformation verwendet. Die selektive Regeneration der Tabakpflänzchen erfolgte einerseits auf Phosphinothricin (5 mg/l) und andererseits zur Kontrolle auf Kanamycin (100 mg/l).

### c) Ergebnisse der Analyse der Phosphinothricinresistenz der transgenen Tabakpflanzen

Im Gegensatz zur Selektion auf Kanamycin, die normal verlief, konnten unter Selektion auf Phosphinothricin keine Kalli oder Sprosse erhalten werden. So ist der Ubiquitin Promotor nicht geeignet für die Expression eines selektiven Markers für den *Agrobacterium tumefaciens*-vermittelten Gentransfer mit anschließender Regeneration von Geweben.

### Vergleichsbeispiel 9: Testung der Promotoraktivität des Ubiquitin- und Squalen Synthase-Promotors mittels Partikelkanone

Um transient die Aktivität des Ubiquitin- und des Squalen Synthase-Promotors zu testen, wurden sterile Tabakblätter mit Plasmid-DNA der Plasmide pUBI42GUS, pSQSPGUS und pGUSINT37 mit der Partikelkanone "Biolistics" von BioRad beschossen. Dabei wurden Microcarrier (25 µg Gold, Hereus 0,3 bis 3 µm) mit 10 µg Plasmid DNA, 2,5 M CaCl2₂, und 0,1 M Spermidine behandelt, mit Alkohol gewaschen und bei einem Vakuum von 26 inch und einem Druck von 1100 psi auf die Blätter, die auf MS-Agarmedium lagen, geschossen. Die Explantate wurden anschließend für 24 h in MS-Medium mit 2% Sucrose inkubiert und dann mit X-gluc histochemisch gefärbt.

Der Ubiquitin-Promotor und der Squalen Synthase-Promotor zeigten hierbei im Gegensatz zum Vergleichskonstrukt pGUSINT37, wo das GUS Gen unter der Kontrolle des 35S Promotors exprimiert wurde, nur sehr wenige und sehr schwache Punkte mit GUS-Färbung.
Dies zeigt, daß dass die Aktivität des Ubiquitin- und des Squalen Synthase-Promotors deutlich schwächer ist, als die des CaMV35S-Promotors.

## Patentansprüche

1. Expressionskassette zur transgenen Expression von Nukleinsäuren enthaltend
a) einen Promotor gemäß SEQ ID NO: 3 oder
b) funktionelle Äquivalente ausgewählt aus der Gruppe bestehend aus
i) Triose-Phosphat Translokator Promoter aus *Arabidopsis thaliana* amplifizierbar mit Primern der SEQ ID No.: 17 und 18 oder SEQ ID No.: 18 und 26;
ii) Sequenzen, die unter Bedingungen mit 0,2x SSC bei 50°C mit der Nukleinsäuresequenz kodierend für einen Promotor gemäß SEQ ID NO: 3 oder der zu ihr komplementären Nukleinsäuresequenzen hybridisieren, und
iii) dem äquivalente Fragmente von a) beschrieben durch durch SEQ ID NO: 27,
die im wesentlichen die gleichen Promotoraktivitäten wie a) besitzen,
wobei a) oder b) funktionell mit einer transgen zu exprimierenden Nukleinsäuresequenz verknüpft sind und deren ubiquitäre und entwicklungsunabhängige Expression vermitteln.

2. Expressionskassette nach Anspruch 1, **dadurch gekennzeichnet, dass**
a) die zu exprimierende Nukleinsäuresequenz zur ubiquitären und entwicklungsunabhängigen Expression mit weiteren genetischen Kontrollsequenzen ausgewählt aus der Gruppe bestehend aus 5'-untranslatierte Regionen, Introns, nichtkodierenden 3'-Region von Genen, expressionssteigernden Enhancer Sequenzen, Rekombinationssequenzen, Polyadenylierungssignale, Terminatoren funktionell verknüpft ist, oder
b) die Expressionskassette zur ubiquitären und entwicklungsunabhängigen Expression zusätzliche Funktionselemente ausgewählt aus der Gruppe bestehend aus Reportergenen, Replikationsursprüngen, Border-Sequenzen und multiplen Klonierungsregionen enthält, oder
c) a) und b) gegeben sind.

3. Expressionskassette nach einem der Ansprüchen 1 oder 2, wobei die transgen zu exprimierende Nukleinsäuresequenz
a) eine Nukleinsäuresequenz kodierend ein Protein, oder
b) eine Nukleinsäuresequenz kodierend eine sense oder anti-sense-RNA umfasst.

4. Expressionskassette nach einem der Ansprüche 1 bis 3, wobei die transgen zu exprimierende Nukleinsäuresequenz ausgewählt ist aus Nukleinsäuren kodierend für Selektionsmarker, Reportergene, Cellulasen, Chitinasen, Glucanasen, Ribosom-inaktivierende Proteine, Lysozyme, Bacillus thuringiensis Endotoxin, α-Amylaseinhibitor, Proteaseinhibitoren, Lektine, RNAasen, Ribozyme, Acetyl-CoA-carboxylasen, Phytasen, das 2S Albumin aus Bertholletia excelsa, "antifreeze"-Proteinen, Trehalosephosphatsynthase, Trehalosephosphatphosphatase, Trehalase, DREBIA-Faktor, Farnesyltransferasen, Ferritin, Oxalatoxidase, Calcium-abhängigen Proteinkinasen, Calcineurinen, Glutamatdehydrogenasen, das N-hydroxylierende, multifunktionelle Cytochrom P-450, den transkriptionellen Aktivator CBF1, Phytoendesaturasen, Polygalakturonasen, Flavonoid-3'-hydroxylasen, Dihydroflavanol-4-reduktasen, Chalconisomerasen, Chalconsynthasen, Flavanone-3-beta-hydroxylasen, Flavonsynthase II, Verzweigungsenzyms Q, "Starch Branching" Enzyme.

5. Transgene Expressionskassette nach einem der Ansprüche 1 bis 4 wobei die transgen zu exprimierenden Nukleinsäuresequenz ausgewählt ist aus der Gruppe bestehend aus positiven Selektionsmarkern, und negativen Selektionsmarkern.

6. Transgene Expressionskassette nach Anspruch 6, **dadurch gekennzeichnet, dass** der Selektionsmarker ausgewählt ist aus der Gruppe bestehend aus Proteinen, die eine Resistenz gegen Antibiotika, Metabolismus-Inhibitoren, Herbizide oder Biozide verleihen.

7. Transgene Expressionskassette nach einem der Ansprüche 6 oder 7, **dadurch gekennzeichnet, dass** der Selektionsmarker ausgewählt ist aus der Gruppe bestehend aus Proteinen, die eine Resistenz verleihen gegen Phosphinothricin, Glyphosat, Bromoxynil, Dalapon, 2-Desoxyglucose-6-phosphat, Tetracycline, Ampicillin, Kanamycin, G 418, Neomycin, Paromomycin, Bleomycin, Zeocin, Hygromycin, Chloramphenicol, Sulfonylharnstoff-Herbizide, Imidazolinon-Herbizide.

8. Transgene Expressionskassette nach einem der Ansprüche 6 bis 8, **dadurch gekennzeichnet, dass** der Selektionsmarker ausgewählt ist aus der Gruppe bestehend aus Phosphinothricinacetyltransferasen, 5-Enolpyruvylshikimat-3-phosphatsynthasen, Glyphosatoxidoreduktasen, Dehalogenase, Nitrilasen, Neomycinphosphotransferasen, DOG^{R}1-Genen, Acetolactatsynthasen, Hygromycinphosphotransferasen, chloramphenicolacetyltransferasen, Streptomycinadenylyltransferasen, β-Lactamasen, tetA Genen, tetR Genen, Isopentenyltransferasen, Thymidinkinasen, Diphtheriatoxin A, Cytosindeaminase (codA), Cytochrom P450, Haloalkandehalogenasen, iaaH Gene, tms2 Gene, β-Glucuronidasen, Mannose-6-phosphat-Isomerasen, UDP-Galaktose-4-Epimerasen.

9. Transgene Expressionskassette nach einem der Ansprüche 6 bis 9, **dadurch gekennzeichnet, dass** der Selektionsmarker kodiert wird durch Nukleinsäuresequenzen
i) beschrieben durch SEQ ID NO: 5 oder 6,

10. Vektoren enthaltend eine Expressionskassette gemäß einem der Ansprüche 1 bis 10.

11. Verfahren zur transgenen ubiquitären oder entwicklungsunabhängigen Expression von Nukleinsäuren, **dadurch gekennzeichnet, dass** eine Nukleinsäuresequenz in funktioneller Verknüpfung mit
a) einen Promotor gemäß SEQ ID NO: 3 oder
b) einem funktionellen Äquivalent ausgewählt aus der Gruppe bestehend aus
i) Triose-Phosphat Translokator Promoter aus *Arabidopsis thaliana* amplifizierbar mit Primern der SEQ ID No.: 17 und 18 oder SEQ ID No.: 18 und 26;
ii) Sequenzen, die unter Bedingungen mit 0,2x SSC bei 50°C mit der Nukleinsäuresequenz kodierend für einen Promotor gemäß SEQ ID NO: 3 oder der zu ihr komplementären Nukleinsäuresequenzen hybridisieren, und
iii) dem äquivalenten Fragment von a) beschrieben durch SEQ ID NO: 27,
transgen exprimiert wird;
wobei a) oder b) funktionell mit einer transgen zu exprimierenden Nukleinsäuresequenz verknüpft sind und deren ubiquitäre und entwicklungsunabhängige Expression vermitteln..

12. Verfahren zur Herstellung von Zelle, Gewebe oder Teil eines dicotoylen Organismus oder Vermehrungsgut abgeleitet von dictoylen Organismen oder dictoylem pflanzlicher Organismus, wobei die Pflanze oder das Teil davon mit einem Nukleinsäuremolekül umfassend die Expressionskassette nach einem der Ansprüche 1 bis 11 transformiert wird.

13. Verfahren nach einem der Ansprüche 10 bis 12, **dadurch gekennzeichnet, dass**
a) die zu exprimierende Nukleinsäuresequenz zur ubiquitären und entwicklungsunabhängigen Expression mit weiteren genetischen Kontrollsequenzen ausgewählt aus der Gruppe bestehend aus 5'-untranslatierte Regionen, Introns, nichtkodierenden 3'-Region von Genen, expressionssteigernden Enhancer Sequenzen, Rekombinationssequenzen, Polyadenylierungssignale, Terminatoren funktionell verknüpft ist, oder
b) die Expressionskassette zur ubiquitären und entwicklungsunabhängigen Expression zusätzliche Funktionselemente ausgewählt aus der Gruppe bestehend aus Reportergenen, Replikationsursprüngen, Border-Sequenzen und multiplen Klonierungsregionen enthält,

14. Verfahren nach einem der Ansprüchen 10 bis 13, **dadurch gekennzeichnet, dass** die transgen zu exprimierende Nukleinsäuresequenz
a) eine Nukleinsäuresequenz kodiertend ein Protein, oder
b) eine Nukleinsäuresequenz kodierend eine sense- oder anti-sense-RNA
umfasst.

15. verfahren nach einem der Ansprüche 10 bis 14 , wobei die transgen zu exprimierende Nukleinsäuresequenz ausgewählt ist aus Nukleinsäuren kodierend für Selektionsmarker, Reportergene, Cellulasen, Chitinasen, Glucanasen, Ribosom-inaktivierende Proteine, Lysozyme, Bacillus thuringiensis Endotoxin, α-Amylaseinhibitor, Proteaseinhibitoren, Lektine, RNAasen, Ribozyme, Acetyl-CoA-carboxylasen, Phytasen, das 2S Albumin aus Bertholletia excelsa, "antifreeze"-Proteinen, Trehalosephosphatsynthase, Trehalosephosphatphosphatase, Trehalase, DREB1A-Faktor, Farnesyltransferasen, Ferritin, Oxalatoxidase, Calcium-abhängigen Proteinkinasen, Calcineurinen, Glutamatdehydrogenasen,das N-hydroxylierende, multifunktionelle Cytochrom P-450, den transkriptionellen Aktivator CBF1, Phytoendesaturasen, Polygalakturonasen, Flavonoid-3'-hydroxylasen, Dihydroflavanol-4-reduktasen, Chalconisomerasen, Chalconsynthasen, Flavanone-3-beta-hydroxylasen, Flavonsynthase II, Verzweigungsenzyms Q, "Starch Branching" Enzyme.

16. Verfahren zur Selektion transformierter pflanzlicher Organismen, **dadurch gekennzeichnet, dass** eine Nukleinsäuresequenz kodierend für einen Selektionsmarker in funktioneller, transgener Verknüpfung mit
a) einem Promotor gemäß SEQ ID NO: 3, oder
b) einem funktionellen Äquivalent ausgewählt aus der Gruppe bestehend aus
i) Triose-Phosphat Translokator Promoter aus *Arabidopsis thaliana* amplifizierbar mit Primern der SEQ ID No.: 17 und 18 oder SEQ ID No.: 18 und 26;
ii) Sequenzen, die unter Bedingungen mit 0,2x SSC bei 50°C mit der Nukleinsäuresequenz kodierend für einen Promotor gemäß SEQ ID NO: 3 oder der zu ihr komplementären Nukleinsäuresequenzen hybridisieren, und
iii) dem äquivalenten Fragment von a) beschrieben durch SEQ ID NO: 27,
wobei a) oder b) funktionell mit einer transgen zu exprimierenden Nukleinsäuresequenz verknüpft sind und deren ubiquitäre und entwicklungsunabhängige Expression vermitteln.
in einen Organismus eingebracht wird, die Expression des Selektionsmarkers erfolgt und eine Selektion ausgeübt wird.

17. Verfahren nach Anspruch 16 , **dadurch gekennzeichnet, dass** der Selektionsmarker ausgewählt ist aus den in den Ansprüchen 5 bis 8 genannten Gruppen von Selektionsmarkern.

18. Zelle, Zellkultur, Gewebe, oder Teil von einem dicotylen Organismus, oder Vermehrungsgut abgeleitet von einem dicotylen Organismus oder dictoyler pflanzlicher Organismus hergestellt nach dem Verfahren nach einem der Ansprüche 12 bis 17.

19. Zelle, Zellkultur, Gewebe, oder Teil von einem dicotylen Organismus, oder Vermehrungsgut abgeleitet von einem dicotylen Organismus oder dictoyler pflanzlicher Organismus transformiert mit einer Expressionskassette gemäß den Ansprüchen 1 bis 9 oder einem Vektor gemäß Anspruch 10 oder diese(n) enthaltend.

20. Zelle oder Gewebe nach Anspruch 18 oder 19 ausgewählt aus der Gruppe bestehend aus Bakterien, Hefen, Pilzen und abgeleitet von einem dicotylen pflanzlichen Organismen.

21. Pflanze nach einem der Ansprüche 18 bis 20 ausgewählt aus der Gruppe bestehend aus Arabidopsis, Tomate, Tabak, Kartoffeln, Raps, Sonnenblumen, Rübe, Zuckerrübe, Bohnengewächse und Soja.

22. Zellkulturen, Teile oder transgenes Vermehrungsgut abgeleitet von einem transgenen Organismus nach einem der Ansprüche 18 bis 21 enthaltend die Experessionskassette nach einem der Ansprüche 1 bis 9 oder den Vektor nach Anspruch 10.

23. Verwendung eines transgenen Organismus nach einem der Ansprüche 18 bis 21 oder von diesem abgeleitete Zellkulturen, Teile oder transgenes Vermehrungsgut nach Anspruch 24 zur Herstellung von Nahrungs-, Futtermitteln, Saatgut, Pharmazeutika oder Feinchemikalien.

## Claims

1. An expression cassette for transgenic expression of nucleic acids, comprising
a) a promoter according to SEQ ID NO: 3 or
b) functional equivalents selected from the group consisting of
i) *Arabidopsis thaliana* triose phosphate translocator promoter, amplifiable with primers of SEQ ID NO: 17 and 18 or SEQ ID NO: 18 and 26;
acid sequence coding for a promoter according to SEQ ID NO: 3 or the nucleic acid sequences complementary thereto under conditions including 0.2x SSC at 50 °C, and
iii) the equivalent fragment of a), specified by SEQ ID NO 27,
which essentially possess the same promoter activities as a),
a) or b) being functionally linked to a nucleic acid sequence to be expressed transgenically and mediating ubiquitous and development-independent expression thereof.

2. The expression cassette according to claim 1, wherein
a) the nucleic acid sequence to be expressed, for ubiquitous and development-independent expression, is functionally linked to further genetic control sequences selected from the group consisting of 5'-untranslated regions, introns, noncoding 3' region of genes, expression-increasing enhancer sequences, recombinant sequences, polyadenylation signals, terminators, or
b) the expression cassette, for ubiquitous and development-independent expression, comprises additional functional elements selected from the group consisting of reporter genes, origins of replication, border sequences and multiple cloning regions, or
c) a) and b) apply.

3. The expression cassette according to either of claims 1 and 2, wherein the nucleic acid sequence to be expressed transgenically comprises
a) a nucleic acid sequence encoding a protein, or
b) a nucleic acid sequence encoding a sense or antisense RNA.

4. The expression cassette according to any of claims 1 to 3, wherein the nucleic acid sequence to be expressed transgenically is selected from nucleic acids coding for selection markers, reporter genes, cellulases, chitinases, glucanases, ribosome-inactivating proteins, lysozymes, *bacillus thuringiensis* endotoxin, α-amylase inhibitor, protease inhibitors, lectins, RNAases, ribozymes, acetyl-CoA carboxylases, phytases, the 28 albumin from *Bertholletia excelsa,* antifreeze proteins, trehalose phosphate synthase, trehalose phosphate phosphatase, trehalase, DREB1a factor, farnesyl transferases, ferritin, oxalate oxidase, calcium-dependent protein kinases, calcineurine, glutamate dehydrogenases, the N-hydroxylating multifunctional cytochrome P-450, the transcriptional activator CBP1, phytoene desaturases, polygalacturonases, flavonoid 3,-hydroxylases, chalcone synthases, flavanone 3-beta-hydroxylases, flavone synthase II, branching enzyme Q, starch branching enzyme.

5. The transgenic expression cassette according to any of claims 1 to 4, wherein the nucleic acid sequence to be expressed transgenically is selected from the group consisting of positive selection markers and negative selection markers.

6. The transgenic expression cassette according to claim 6, wherein the selection marker is selected from the group consisting of proteins which impart a resistance to antibiotics, metabolism inhibitors, herbicides or biocides.

7. The transgenic expression cassette according to either of claims 6 and 7, wherein the selection marker is selected from the group consisting of proteins, which impart a resistance to phosphinothricin, glyphosate, bromoxynil, dalapon, 2-deoxyglucose 6-phosphate, tetracyclines, ampicillin, kanamycin, G 418, neomycin, paromomycin, bleomycin, zeocin, hygromycin, chloramphenicol, sulfonyl urea herbicides, imidazolinone herbicides.

8. The transgenic expression cassette according to any of claims 6 to 8, wherein the selection marker is selected from the group consisting of phosphinothricin acetyltransferases, 5-enolpyruvylshikimate 3-phosphate synthases, glyphosate oxidoreductases, dehalogenases, nitrilases, neomycin phosphotransferases, DOG^{R}1 genes, acetolactate synthases, hygromycin phosphotransferases, chloramphenicol acetyltransferases, streptomycin adenylyltransferases, β-lactamases, tetA genes, tetR genes, isopentenyl transferases, thymidine kinases, diphtheria toxin A, cytosine deaminase (codA), cytochrome P450, haloalkane dehalogenases, iaaH gene, tms2 gene, β-glucuronidases, mannose 6-phosphate isomerases, UDP-galactose 4-epimerases.

9. The transgenic expression cassette according to any of claims 6 to 9, wherein the selection marker is encoded by nucleic acid sequences
i) described by SEQ ID NO: 5 or 6.

10. A vector comprising an expression cassette according to any of claims 1 to 10.

11. A method for transgenic ubiquitous or development-independent expression of nucleic acids, wherein a nucleic acid sequence which is functionally linked to
a) a promoter according to SEQ ID NO : 3 or
b) a functional equivalent selected from the group consisting of
i) *Arabidopsis thaliana* triose phosphate translocator promoter, amplifiable with primers of SEQ ID NO: 17 and 18 or SEQ ID NO: 18 and 26;
ii) sequences which hybridize with the nucleic acid sequence coding for a promoter according to SEQ ID NO: 3 or the nucleic acid sequences complementary thereto under conditions including 0.2x SSC at 50°C, and
iii) the equivalent fragment of a), specified by SEQ ID NO: 27,
is expressed transgenically;
a) or b) being functionally linked to a nucleic acid sequence to be expressed transgenically, and mediating ubiquitous and development-independent expression thereof.

12. A method for preparing a cell, tissue or part of a dicotyledonous organism or propagation material derived from dicotyledonous organisms or a dicotyledonous plant organism, said plant or said part thereof being transformed with a nucleic acid molecule comprising the expression cassette according to any of claims 1 to 11,

13. The method according to any of claims 10 to 12, wherein
a) the nucleic acid sequence to be expressed, for ubiquitous and development-independent expression, is functionally linked to further genetic control sequences selected from the group consisting of 5'-untranslated regions, introns, noncoding 3' region of genes, expression-increasing enhancer sequences, recombinant sequences, polyadenylation signals, terminators, or
b) the expression cassette, for ubiquitous and development-independent expression, comprises additional functional elements selected from the group consisting of reporter genes, origins of replication, border sequences and multiple cloning regions.

14. The method according to any of claims 10 to 13, wherein the nucleic acid sequence to be expressed transgenically comprises
a) a nucleic acid sequence encoding a protein, or
b) a nucleic acid sequence encoding a sense or antisense RNA.

15. The method according to any of claims 10 to 14, wherein the nucleic acid sequence to be expressed transgenically is selected from nucleic acids coding for selection markers, reporter genes, cellulases, chitinases, glucanases, ribosome-inactivating proteins, lysozymes, *Bacillus thuringiensis* endotoxin, α-amylase inhibitor, protease inhibitors, lectins, RNAases, ribozymes, acetyl-CoA carboxylases, phytases, the 2S albumin from *Bertholletia excelsa*, antifreeze proteins, trehalose phosphate synthase, trehalose phosphate phosphatase, trehalase, DREB1A factor, farnesyl transferases, ferritin, oxalate oxidase, calcium-dependent protein kinases, calcineurins, glutamate dehydrogenases, the N-hydroxylating multifunctional cytochrome P-450, the transcriptional activator CBF1, phytoene desaturases, polygalacturonases, flavonoid 3'-hydroxylases, dihydroflavanol 4-reductases, chalcone isomerases, chalcone synthases, flavanone 3-beta-hydroxylases, flavone synthase II, branching enzyme Q, starch branching enzyme.

16. A method for selecting transformed plant organisms, wherein a nucleic acid sequence coding for a selection marker, which is functionally and transgenically linked to
a) a promoter according to SEQ ID NO: 3, or
b) a functional equivalent selected from the group consisting of
i) *Arabidopsis thaliana* triose phosphate translocator promoter, amplifiable with primers of SEQ ID NO: 17 and 18 or SEQ ID NO: 18 and 26;
ii) sequences which hybridize with the nucleic acid sequence coding for a promoter according to SEQ ID NO: 3 or the nucleic acid sequences complementary thereto under conditions including 0.2x SSC at 50°C, and
iii) the equivalent fragment of a), specified by SEQ ID NO:27,
a) or b) being functionally linked to a nucleic acid sequence to be expressed transgenically, and mediating ubiquitous and development-independent expression thereof,
is introduced into an organism, the selection marker is expressed and a selection is carried out.

17. The method according to claim 16, wherein the selection marker is selected from the groups of selection markers which are mentioned in claims 5 to 8.

18. A cell, cell culture, tissue or part of a dicotyledonous organism or propagation material derived from a dicotyledonous organism or a dicotyledonous of claims 12 to 17.

19. A cell, cell culture, tissue or part of a dicotyledonous organism or propagation material derived from a dicotyledonous organism or a dicotyledonous plant organism transformed with or comprising and expression cassette according to claims 1 to 9 or a vector according to claim 10.

20. A cell or tissue according to claim 18 or 19, selected from the group consisting of bacteria, yeasts, fungi and derived from a dicotyledonous plant organism.

21. The plant according to any of claims 18 to 20 selected from the group consisting of *Arabidopsis*, tomato, tobacoo, potatoes, oilseed rape, sunflowers, beet, sugarbeet, bean plants and soyabean.

22. A cell culture, part or transgenic propagation material derived from a transgenic organism according to any of claims 18 to 21, comprising the expression cassette according to any of claims 1 to 9 or the vector according to claim 10.

23. The use of a transgenic organism according to any of claims 18 to 21 or of cell cultures, parts or transgenic propagation material derived therefrom according to claim 24 for the production of food- and feedstuffs, seed, pharmaceuticals or fine chemicals.

## Revendications

1. Cassette d'expression dans le but de réaliser l'expression transgénique d'acides nucléiques, contenant :
a) un promoteur représenté par la SEQ ID n° 3, ou
b) des équivalents fonctionnels choisis dans le groupe constitué:
i) d'un promoteur du gène de translocation du triose-phosphate de l'espèce Arabidopsis thaliana, qui peut être amplifié avec des amorces représentées par les SEQ ID n° 17 et 18 ou par les SEQ ID n° 18 et 26 ;
ii) de séquences qui s'hybrident, dans des conditions de SSC 0,2x à 50°C, avec la séquence d'acide nucléique codant pour un promoteur représenté par la SEQ ID n° 3 ou avec des séquences d'acide nucléique qui lui sont complémentaires, et
iii) du fragment équivalent de a) et représenté par la SEQ ID n° 27,
qui présentent sensiblement les mêmes activités de promoteur que celui décrit en a),
a) ou b) étant relié de manière fonctionnelle à une séquence d'acide nucléique que l'on souhaits faire exprimer par voie transgénique, et conférant son expression ubiquitaire et indépendante du développement.

2. Cassette d'expression selon la revendication 1, **caractérisée en ce que** :
a) la séquence d'acide nucléique que l'on souhaite faire exprimer dans le but de réaliser une expression ubiquitaire et indépendante du développement, est reliée de manière fonctionnelle à d'autres séquences de régulation génétiques choisies dans le groupe constitué des régions non traduites en 5', des introns, de la région 3' non codante de gènes, des séquences d'activation renforçant l'expression, des séquences de recombinaison, des signaux de polyadénylation, des terminateurs, ou
b) la cassette d'expression en vue de réaliser l'expression ubiquitaire et indépendante du développement, contient d'autres élements fonctionnels choisis dans le groupe constitué des gènes rapporteurs, des origines de réplication, des séquences de bordure et des sites de clonage multiples, ou
c) on dispose de a) et de b).

3. Cassette d'expression selon l'une quelconque des revendication 1 ou 2, dans laquelle la séquence d'acide nucléique que l'on souhaite faire exprimer par voie transgénique comprend:
a) une séquence d'acide nucléique codant pour une protéine, ou
b) une séquence d'acide nucléique codant pour un ARN sens ou antisens.

4. Cassette d'expression selon l'une quelconque des revendications 1 à 3, dans laquelle la séquence d'acide nucléique que l'on souhaite faire exprimer par voie transgénique est choisie parmi des acides nucléiques codant pour des marqueurs de sélection, des gènes rapporteurs, des cellulases, des chitinases, des glucanases, des protéines inactivatrices du ribosome,des lysozynes, une endotoxine de l'espèce Bacillus thuringiensis, un inhibiteur de la α-amylase, des inhibiteurs de protéases, des lectines, des ARNases, des ribozymes, des acécyl-CoA carboxylases, des phytases, l'albumine 26 de l'espèce Bertholletia excelsa, des protéines "antigel", une tréhalose-phosphate synthase, une tréhalose-phosphate phosphatase, une tréhalase, le facteur DREB1A, des farnésyle-transférases, la ferritine, une oxalatoxydase, des protéine-kinases dépendantes du calcium, des calcineurines, des glutamate-déshydrogénases, le cytochrome P-450 multifonctionnel à activité d'hydroxylation de l'azote, l'activateur transcriptionnel CBF1, des phytoène-désaturases, des polygalacturonases, des flavonoïde-3'-hydroxylases, des dihydroflavanol-4-réductases, des chalcone-isomérases, des chalcone-synthases, des flavanone-3-bêta-hydroxylases, la flavone-synthase II, l'enzyme de ramification Q et des enzymes de "ramification de l'amidon".

5. Cassette d'expression transgénique selon l'une quelconque des revendications 1 à 4, dans laquelle la séquence d'acide nucléique que l'on souhaite faire exprimer par voie transgénique est choisie dans le groupe constitué des marqueurs de sélection positifs et des marqueurs de sélection négatifs.

6. Cassette d'expression transgénique selon la revendication 6, **caractérisée en ce que** le marqueur de sélection est choisi dans le groupe constitué des protéines qui confèrent une résistance à des antibiotiques, à des inhibiteurs de métabolisme, à des herbicides ou à des biocides.

7. Cassette d'expression transgénique selon les revendications 6 ou 7, **caractérisée en ce que** le marqueur de sélection est choisi dans le groupe constitué des protéines qui confèrent une résistance à la phosphinothricine, au glyphosate, au bromoxynile, au dalapon, au 2-désoxyglucose-6-phosphate, à la tétracycline, à l'ampicilline, à la kanamycine, au composé G418, à la néomycine, à la paromomycine, à la bléomycine, à la zéocine, à l'hygromycine, au chloramphénicol, à des herbicides à base de sulfonylurée et à des herbicides à base d'imidazolinone.

8. Cassette d'expression transgénique selon l'une quelconque des revendications 6 à 8, **caractérisée en ce que** le marqueur de sélection est choisi dans le groupe constitué des phosphilotricine-acétyltransférases, des 5-énolpyruvylshikimate-3-phosphate-synthases, des glyphosate-oxydoréductases, des déshalogénases, des nitrilases, des néomycine-phosphotransférases, des gènes DOG^{B}1, des acécolactate-synthases, des hygromycine-phosphotransférases, des chloramphénicol-acétyltransférases, des streptomycine-adénylyltransférases, des β-lactamases, des gènes tetA, des gènes tetR, des isopentényle-transtérases, des thymidine-kinases, de la toxine diphtérique A, de la cytosine-désaminase (codA); du cytochrome P450, des halogénoalcane-déshalogénases, des gènes iaaH, des gènes tme2, des β-glucuronidases, des mannose-6-phosphate-isomérases, des UDP-galactose-4-épimérases.

9. Cassette d'expression transgénique selon l'une quelconque des revendications 6 à 9, **caractérisée en ce que** le marqueur de sélection est codé par des séquences d'acide nucléique qui :
i) sont décrites par la SEQ ID n° 5 ou 6.

10. Vecteurs contenant une cassette d'expression selon l'une quelconque des revendications 1 à 10.

11. Procédé dans le but de réaliser l'expression transgénique ubiquitaire ou indépendante du développement d'acides nucléiques, **caractérisé en ce que** l'on fait exprimer par voie transgénique une séquence d'acide nucléique reliée de manière fonctionnelle avec :
a) un promoteur représenté par la SEQ ID n° 3, ou
b) un équivalent fonctionnel choisi dans le groupe constitué :
i) du promoteur du gène de translocation du triose-phosphate de l'espèce Arabidopsis thaliana, qui peut être amplifié avec des amorces représentées par les SEQ ID n° 17 et 18 ou par les SEQ ID n° 18 et 26,
ii) de séquences qui s'hybrident dans des conditions de SSC 0,2x à 50 °C avec la séquence d'acide nucléique codant pour un promoteur représentés par la SEQ ID n° 3 ou avec les séquences d'acide nucléique qui lui sont complémentaires, et
iii) du fragment équivalent de a) et représenté par la SEQ ID n° 27;
a) ou b) étant relié de manière fonctionnelle à une séquence d'acide nucléique que l'on souhaite faire exprimer par voie transgénique et conférant son expression ubiquitaire et indépendante du développement.

12. Procédé pour la production d'une cellule, d'un tissu ou d'une partie d'un organisme du type dicotylédone ou d'un produit d'amplification dérivé d'organismes du type dicotylédone ou d'un organisme végétal de type dicotylédone, dans lequel on transforme la plante, ou la partie de celle-ci, avec une molécule d'acide nucléique comprenant la cassette d'expression selon l'une quelconque des revendications 1 à 11.

13. Procédé selon l'une quelconque des revendications 10 à 12, **caractérisé en ce que**;
a) la séquence d'acide nucléique que l'on souhaite faire exprimer pour réaliser l'expression ubiquitaire et indépendante du développement, est reliée de manière fonctionnelle à d'autres séquences de régulation génétiques choisies dans le groupe constitué des régions non traduites en 5', des introns, de la région 3' non codante de gènes, des séquences d'activation renforçant l'expression, des séquences de recombinaison, des signaux de polyadénylation, des terminateurs, ou
b) la cassette d'expression en vue de réaliser l'expression ubiquitaire et indépendante du développement, contient d'autres éléments fonctionnels choisis dans le groupe constitué des gènes rapporteurs, des origines de réplication, des séquences de bordure et des sites de clonage multiples.

14. Procédé selon l'une quelconque des revendications 10 à 13, **caractérisé en ce que** la séquence d'acide nucléique l'on souhaite faire exprimer par voie transgénique comprend:
a) une séquence d'acide nucléique codant pour une protéine, ou
b) une séquence d'acide nucléique codant pour un ARN sens ou antisens.

15. Procédé selon l'une quelconque des revendications 10 à 14, dans lequel la séquence d'acide nucléique que l'on souhaite faire exprimer par voie transgénique est choisis parmi des acides nucléiques codant pour des marqueurs de sélection, des gènes rapporteurs, des cellulases, des chitinases, des glucanases, des protéines inactivatrices du ribosome, des lysozymes, l'endotoxine de l'espèce Bacillus thuringiensis, un inhibiteur de la α-amylase, des inhibiteurs de protéases, des lectines, des ARNases, des, ribozymes des acétyl-CoA carboxylases, des phytases, l'albumine 2S de l'espèce Bertholletia excelsa, des protéines "antigel", une tréhalose-phosphatase, une tréhalase, le facteur DREB1A des farnésyle-transférases, la ferritine, une oxalatoxydase, des protéine-kinases dépendantes du calcium, des calcineurines des glutamate-déshydrogénases, le cytochrome P-450 multifonctionnel à activité d'hydroxylation de l'azote, l'activateur transcriptionnel CBF1, des phytoène-désaturases, des polygalacturonases, des flavonoïde -3'-hydroxylases, des dihydroflavanol-4-réductases, des chalcone-isomérases, des chalcone-synthases, des flavanone-3-bêta-hydroxylases, la flavone synthase II, l'enzyme de ramification Q et des enzymes de "ramification de l'amidon".

16. Procédé pour sélectionner des organismes végétaux transformés, **caractérisé en ce que** l'on incorpore une séquence d'acide nucléique codant pour un marqueur de sélection reliée de manière fonctionnelle et transgénique avec :
a) un promoteur représenté par la SEQ ID n° 3, ou
b) un équivalent fonctionnel choisi dans le groupe constitué:
i) du promoteur du gène de translocation du triose-phosphate de l'espèce Arabidopsis thaliana, qui peut être amplifié avec des amorces représentées par les SBQ ID n° 17 et 18 on par les SEQ ID n° 18 et 26 ;
ii) des séquences qui s'hybrident dans des conditions de SSC 0.2x à 50 °C avec la séquence d'acide nucléique codant pour un promoteur selon la SEQ ID n° 3 ou avec les séquences d'acide nucléique qui lui sont complémentaires, et
iii) du fragment équivalent de a) et représenté par la SEQ ID n° 27 ;
a) ou b) étant relié de manière fonctionnelle à une séquence d'acide nucléique que l'on souhaite faire exprimer par voie transgénique, et conférant son expression ubiquitaire et indépendante du développement, à un organisme végétal, **en ce que** l'expression du marqueur de sélection a lieu et **en ce qu'**une sélection s'exerce.

17. Procédé selon la revendication 16, **caractérisé en ce que** le marqueur de sélection est choisi dans les groupes de marqueurs de sélection cités dans les revendications 5 à 8.

18. Cellule, culture cellulaire, tissu ou partie d'un organisme du type dicotylédone, ou produit d'amplification dérivé d'un organisme du type dicotylédone ou organisme végétal du type dicotylédone, que l'on produit en utilisant le procédé selon l'une quelconque des revendications 12 à 17.

19. Cellule, culture cellulaire, tissu ou partie d'un organisme du type dicotylédone, ou produit d'amplification dérivé d'un organisme du type dicotylédone ou organisme végétal du type dicotylédone, que l'on a transformé avec une cassette d'expression selon les revendications 1 à 9 ou avec un vecteur selon la revendication 10, ou qui contient celle-ci/celui-ci.

20. Cellule ou tissu selon la revendication 18 ou 19, choisi(e) dans le groupe constitué des bactéries, des levures et des champignons, et dérivé(e) d'un organisme végétal du type dicotylédone.

21. Plante selon l'une quelconque des revendications 18 à 20, choisie dans le groupe constitué de la variété arabidopsis, de la tomate, du tabac, de la pomme de terre, du colza, du tournesol, de la betterave, de la betterave sucrière, de plants de haricots et du soja.

22. Cultures cellulaires, parties ou produit d'amplification transgénique dérivé d'un organisme transgénique selon l'une quelconque des revendications 18 à 21, contenant la cassette d'expression selon l'une quelconque des revendications 1 à 9 ou contenant le vecteur selon la revendication 10.

23. Utilisation d'un organisme transgénique selon l'une quelconque des revendications 18 à 21, ou de cultures cellulaires, de parties, ou d'un produit d'amplification transgénique qui dérivent de celui-ci, selon la revendication 24, dans le but de fabriquer des produits pour l'alimentation humaine, des produits pour l'alimentation animale, des semences, des produits pharmaceutiques ou des produits chimiques fins.


### SEQUENZPROTOKOLL

<110> SunGene GmbH & Co KGaA
<120> Expressionskassetten zur transgenen Expression von Nukleinsäuren
<130> NAE3614/2001
<140>
   <141>
<160> 29
<170> PatentIn Ver. 2.1
<210> 1
   <211> 836
   <212> DNA
   <213> Arabidopsis thaliana
<220>
   <221> promoter
   <222> (1)..(836)
   <223> pFD promoter
<400> 1
   gattcaagct tcactgctta aattcacaaa aagagaaaag taagaccaaa ggaataaatc 60
   atcctcaaac caaaaacaca tcatacaaaa tcatcaaaca taaatctcca gatgtatgag 120
   caccaatcca gttatacaac actcttaaca ccaaatcaac agatttaaca gcgaaataag 180
   cttaagccca tacaattatc cgatccaaac aaatataatc gaaaccggca gaggaataag 240
   caagtgaatc aaaaagtatg ggacgaggaa gaagatgata cctgaatgag aaagtcaata 300
   accttgaccc gaatcgtttt gaagaaaatg gagaaaatcg gttgtatgga ataaaatctt 360
   cgaatgatga gatatatgat ctctttggtg tcagtcacat ggcacacgct atcaatttag 420
   aaaaacgcgg tggttggtca ccagaattac tacttctcgg tctgatttgg tcatatccgt 480
   attaagtccg gttaatattt tccataactg gggtttgaac attcggtttc tttttttcag 540
   ttagtccgat ttggagtttt gagtatggaa aaataatact gaatttattt gttcaaactg 600
   ttttggaaaa aatatttccc ttaattacga atataattaa aattttaaaa ttcattttat 660
   tagatcttgg ttaattcggt ttaatgcatt aatgaatttc ggtttaagtc ggttttcggt 720
   ttttatgtcc caccactatc tacaaccgat gatcaacctt atctccgtat tcaccacaaa 780
   cagtcatcac tctcacttga cacaaaaact cttttgtctc cgtctctctg tctctc     836
<210> 2
   <211> 635
   <212> DNA
   <213> Arabidopsis thaliana
<220>
   <221> promoter
   <222> (1)..(635)
   <223> FNR promoter
<400> 2
   ttcattattt cgattttgat ttcgtgacca gcgaacgcag aataccttgt tgtgtaatac 60
   tttacccgtg taaatcaaaa acaaaaaggc ttttgagctt tttgtagttg aatttctctg 120
   gctgatcttt tctgtacaga ttcatatatc tgcagagacg atatcattga ttatttgagc 180
   ttcttttgaa ctatttcgtg taatttggga tgagagctct atgtatgtgt gtaaactttg 240
   aagacaacaa gaaaggtaac aagtgaggga gggatgactc catgtcaaaa tagatgtcat 300
   aagaggccca tcaataagtg cttgagccca ttagctagcc cagtaactac cagattgtga 360
   gatggatgtg tgaacagttt tttttttgat gtaggactga aatgtgaaca acaggcgcat 420
   gaaaggctaa attaggacaa tgataagcag aaataactta tcctctctaa cacttggcct 480
   cacattgccc ttcacacaat ccacacacat ccaatcacaa cctcatcata tatctcccgc 540
   taatcttttt ttctttgatc tttttttttt tgcttattat ttttttgact ttgatctccc 600
   atcagttcat cttcttcttc ttcttctgat caacc                            635
<210> 3
   <211> 2038
   <212> DNA
   <213> Arabidopsis thaliana
<220>
   <221> promoter
   <222> (1)..(2038)
   <223> TPT promoter
<400> 3
   ataaccaaaa gaactctgat catgtacgta cccatttgcg tattccgccg ttgcggaatc 60
   aaaaactgcc atagacttct ccacatcttt ttcagctcgc atcagtttga taacctgtga 120
   aggcgtgatg ttctttgacc acttgaacat catcactttg ttacccatta ctaaacaatg 180
   ataccttcca acaatagcaa agaatacacc tttttataga gaagaatctc agctacgcac 240
   tacgtcgaac aggttgtgtg cataaacgat ttcgataggc ccaaaccaaa tgaaagaaac 300
   acagaccaga aaaatcattt gatcttcaaa acatacgagt tccaaaagtg aaggaagcaa 360
   caatgaaact cgttacgaac tagaaggtta atcaaattgc cggagaagaa tcgctcacca 420
   gttttggcta gggtttatga aatgggagac tttagctgca aagagaagag tctctggacg 480
   atttagaggg tgtctctcta ataggcaaca aagtacatat tattacagta ttaaccaaat 540
   ttaaacgaat taagtgtcaa caaaagctta tataaaaaat ttaaagttta aaaattataa 600
   aatatgtcaa caatatttta gtacttaaaa ttattatgcg aaatatttag atcaatggac 660
   tactcatcta atatatttgc acctaatttt aaagtataaa ttcaaccaat aattagaaaa 720
   tgatagctta tactcaaatt caacaagtta tatataaatg tatagatact acaatatcat 780
   taacaaaagt caccttaaat aaatacacat atcttttatg ttctctattg ttttgcgtac 840
   gctaacacaa tttctcatat gcaaaaggat gaatgagtaa caaattacct cataagaaca 900
   atcatctttg cttacatact aatacaataa tcactcaatc aaccaataac atcaatcaca 960
   taggtttaca tacaataatc actcaatcaa cttcataaga agaatcatgt ttacttaatt 1020
   catcaattat ccccaaaaac accactatta agtataaact ataacatatt tgtagtgatg 1080
   ggtcaacatt tttatcatat ttaaactcgg gttccctcaa atcgagaaat agtgaacatg 1140
   taatattaat tttaaatcgc aattacagaa attaattgaa tttggtcaaa tggacagaat 1200
   tttatagatt gggtggaact agaaaaaaaa aaaaaaagag tatagggtga attgagtaca 1260
   tgaaagtaca tggtaatcct agttaaacgc ataatacatg tgggttcatt tgtatttttt 1320
   tgtaacttac gagtaaactg gctacaacaa aaaaaattag aagatttttt tgttttgtag 1380
   aaaaccctaa ttttagttat agttgtataa ctttgataaa attataaaat tgtattacga 1440
   aaaaagtaat aagatattca aaaaagccta gaataacgta tatgactatg agcatgaaac 1500
   tgcaagtcaa atgctgacag acaaccataa acaaaagaaa ttaaatagag atacctttaa 1560
   aataagtaaa atttcattta taaaaaatct actttcttgt gaatctgtca cgttcaataa 1620
   tttgaagacc actcaacata caaggtaaat aatgaaaaat aaaatctacc aaaatttcaa 1680
   tcattattat cttccaaaaa aacaaaatta tacagatgat gatggtgata tggaacttcg 1740
   attggctaat attcactgtg tctctaaaaa ccatccactt atcaagataa gatggaccct 1800
   acactcatcc aatctaaacc agtatctcaa gattcttatc taattacatc attctctacc 1860
   gttagatgaa attgaccatt aaccctacca taactccata caccgcgaga tactggatta 1920
   accaaatcga gatcatcgta gccgtccgat caacaagtac catctcttga aatactcgaa 1980
   atcctcataa gtccgtccct ctttgctctc actatcaaaa ctctgaattt cgatttca 2038
<210> 4
   <211> 699
   <212> DNA
   <213> Arabidopsis thaliana
<220>
   <221> promoter
   <222> (1)..(699)
   <223> FDS promoter
<400> 4
   aacactctta acaccaaatc aacagattta acagcgaaat aagcttaagc ccatacaatt 60
   atccgatcca aacaaatata atcgaaaccg gcagaggaat aagcaagtga atcaaaaagt 120
   atgggacgag gaagaagatg atacctgaat gagaaagtca ataaccttga cccgaatcgt 180
   tttgaagaaa atggagaaaa tcggttgtat ggaataaaat cttcgaatga tgagatatat 240
   gatctctttg gtgtcagtca catggcacac gctatcaatt tagaaaaacg cggtggttgg 300
   tcaccagaat tactacttct cggtctgatt tggtcatatc cgtattaagt ccggttaata 360
   ttttccataa ctggggtttg aacattcggt ttcttttttt cagttagtcc gatttggagt 420
   tttgagtatg gaaaaataat actgaattta tttgttcaaa ctgttttgga aaaaatattt 480
   cccttaatta cgaatataat taaaatttta aaattcattt tattagatct tggttaattc 540
   ggtttaatgc attaatgaat ttcggtttaa gtcggttttc ggtttttatg tcccaccact 600
   atctacaacc gatgatcaac cttatctccg tattcaccac aaacagtcat cactctcact 660
   tgacacaaaa actcttttgt ctccgtctct ctgtctctc                        699
<210> 5
   <211> 552
   <212> DNA
   <213> Künstliche Sequenz
<220>
   <223> Beschreibung der künstlichen Sequenz: codon adapted sequence for phosphinotricin-N-acetyltransferase
<220>
   <221> CDS
   <222> (1)..(549)
   <223> phosphinotricin-N-acetyltransferase (PAT)
<400> 5
   atg tct ccg gag agg aga cca gtt gag att agg cca gct aca gca gcc    48
   Met Ser Pro Glu Arg Arg Pro Val Glu Ile Arg Pro Ala Thr Ala Ala
    1               5                  10                  15
   gat atg gcc gcg gtt tgt gac atc gtt aac cat tac att gag acg tct    96
   Asp Met Ala Ala Val Cys Asp Ile Val Asn His Tyr Ile Glu Thr Ser
               20                  25                  30
   aca gtg aac ttt agg aca gag cca caa aca cca caa gag tgg att gat   144
   Thr Val Asn Phe Arg Thr Glu Pro Gln Thr Pro Gln Glu Trp Ile Asp
           35                  40                  45
   gac cta gag agg ttg caa gat aga tac cct tgg ttg gtt gct gag gtt   192
   Asp Leu Glu Arg Leu Gln Asp Arg Tyr Pro Trp Leu Val Ala Glu Val
       50                  55                  60
   gag ggt gtt gtg gct ggt att gct tac gct ggg ccc tgg aag gct agg   240
   Glu Gly Val Val Ala Gly Ile Ala Tyr Ala Gly Pro Trp Lys Ala Arg
   65                  70                  75                  80
   aac gct tac gat tgg aca gtt gag agt act gtt tac gtg tca cat agg   288
   Asn Ala Tyr Asp Trp Thr Val Glu Ser Thr Val Tyr Val Ser His Arg
                   85                  90                  95
   cat caa agg ttg ggc cta gga tct aca ttg tac aca cat ttg ctt aag   336
   His Gln Arg Leu Gly Leu Gly Ser Thr Leu Tyr Thr His Leu Leu Lys
               100                 105                 110
   tct atg gag gcg caa ggt ttt aag tct gtg gtt gct gtt ata ggc ctt   384
   Ser Met Glu Ala Gln Gly Phe Lys Ser Val Val Ala Val Ile Gly Leu
           115                 120                 125
   cca aac gat cca tct gtt agg ttg cat gag gct ttg gga tac aca gcg   432
   Pro Asn Asp Pro Ser Val Arg Leu His Glu Ala Leu Gly Tyr Thr Ala
       130                 135                 140
   cgg ggt aca ttg cgc gcg gct gga tac aag cat ggt gga tgg cat gat   480
   Arg Gly Thr Leu Arg Ala Ala Gly Tyr Lys His Gly Gly Trp His Asp
   145                 150                 155                 160
   gtt ggt ttt tgg caa agg gat ttt gag ttg cca gct cct cca agg cca   528
   Val Gly Phe Trp Gln Arg Asp Phe Glu Leu Pro Ala Pro Pro Arg Pro
                   165                 170                 175
   gtt agg cca gtt acc cag atc tga                                   552
   Val Arg Pro Val Thr Gln Ile
               180
<210> 6
   <211> 183
   <212> PRT
   <213> Künstliche Sequenz
   <223> Beschreibung der künstlichen Sequenz: codon adapted sequence for phosphinotricin-N-acetyltransferase
<400> 6
   Met Ser Pro Glu Arg Arg Pro Val Glu Ile Arg Pro Ala Thr Ala Ala
   1               5                   10                  15
   Asp Met Ala Ala Val Cys Asp Ile Val Asn His Tyr Ile Glu Thr Ser
               20                  25                  30
   Thr Val Asn Phe Arg Thr Glu Pro Gln Thr Pro Gln Glu Trp Ile Asp
           35                  40                  45
   Asp Leu Glu Arg Leu Gln Asp Arg Tyr Pro Trp Leu Val Ala Glu Val
       50                  55                  60
   Glu Gly Val Val Ala Gly Ile Ala Tyr Ala Gly Pro Trp Lys Ala Arg
   65                  70                  75                  80
   Asn Ala Tyr Asp Trp Thr Val Glu Ser Thr Val Tyr Val Ser His Arg
                   85                  90                  95
   His Gln Arg Leu Gly Leu Gly Ser Thr Leu Tyr Thr His Leu Leu Lys
               100                 105                 110
   Ser Met Glu Ala Gln Gly Phe Lys Ser Val Val Ala Val Ile Gly Leu
           115                 120                 125
   Pro Asn Asp Pro Ser Val Arg Leu His Glu Ala Leu Gly Tyr Thr Ala
       130                 135                 140
   Arg Gly Thr Leu Arg Ala Ala Gly Tyr Lys His Gly Gly Trp His Asp
   145                 150                 155                 160
   Val Gly Phe Trp Gln Arg Asp Phe Glu Leu Pro Ala Pro Pro Arg Pro
                   165                 170                 175
   Val Arg Pro Val Thr Gln Ile
               180
<210> 7
   <211> 2013
   <212> DNA
   <213> Arabidopsis thaliana
<220>
   <221> CDS
   <222> (1)..(2010)
   <223> acetolactate synthase
<400> 7
   atg gcg gcg gca aca aca aca aca aca aca tct tct tcg atc tcc ttc    48
   Met Ala Ala Ala Thr Thr Thr Thr Thr Thr Ser Ser Ser Ile Ser Phe
    1               5                  10                  15
   tcc acc aaa cca tct cct tcc tcc tcc aaa tca cca tta cca atc tcc      96
   Ser Thr Lys Pro Ser Pro Ser Ser Ser Lys Ser Pro Leu Pro Ile Ser
               20                  25                  30
   aga ttc tcc ctc cca ttc tcc cta aac ccc aac aaa tca tcc tcc tcc     144
   Arg Phe Ser Leu Pro Phe Ser Leu Asn Pro Asn Lys Ser Ser Ser Ser
           35                  40                  45
   tcc cgc cgc cgc ggt atc aaa tcc agc tct ccc tcc tcc atc tcc gcc     192
   Ser Arg Arg Arg Gly Ile Lys Ser Ser Ser Pro Ser Ser Ile Ser Ala
       50                  55                  60
   gtg ctc aac aca acc acc aat gtc aca acc act ccc tct cca acc aaa     240
   Val Leu Asn Thr Thr Thr Asn Val Thr Thr Thr Pro Ser Pro Thr Lys
   65                  70                  75                  80
   cct acc aaa ccc gaa aca ttc atc tcc cga ttc gct cca gat caa ccc     288
   Pro Thr Lys Pro Glu Thr Phe Ile Ser Arg Phe Ala Pro Asp Gln Pro
                   85                  90                  95
   cgc aaa ggc gct gat atc ctc gtc gaa gct tta gaa cgt caa ggc gta     336
   Arg Lys Gly Ala Asp Ile Leu Val Glu Ala Leu Glu Arg Gln Gly Val
               100                 105                 110
   gaa acc gta ttc gct tac cct gga ggt gca tca atg gag att cac caa     384
   Glu Thr Val Phe Ala Tyr Pro Gly Gly Ala Ser Met Glu Ile His Gln
           115                 120                 125
   gcc tta acc cgc tct tcc tca atc cgt aac gtc ctt cct cgt cac gaa     432
   Ala Leu Thr Arg Ser Ser Ser Ile Arg Asn Val Leu Pro Arg His Glu
       130                 135                 140
   caa gga ggt gta ttc gca gca gaa gga tac gct cga tcc tca ggt aaa     480
   Gln Gly Gly Val Phe Ala Ala Glu Gly Tyr Ala Arg Ser Ser Gly Lys
   145                 150                 155                 160
   cca ggt atc tgt ata gcc act tca ggt ccc gga gct aca aat ctc gtt     528
   Pro Gly Ile Cys Ile Ala Thr Ser Gly Pro Gly Ala Thr Asn Leu Val
                   165                 170                 175
   agc gga tta gcc gat gcg ttg tta gat agt gtt cct ctt gta gca atc     576
   Ser Gly Leu Ala Asp Ala Leu Leu Asp Ser Val Pro Leu Val Ala Ile
               180                 185                 190
   aca gga caa gtc cct cgt cgt atg att ggt aca gat gcg ttt caa gag     624
   Thr Gly Gln Val Pro Arg Arg Met Ile Gly Thr Asp Ala Phe Gln Glu
           195                 200                 205
   act ccg att gtt gag gta acg cgt tcg att acg aag cat aac tat ctt     672
   Thr Pro Ile Val Glu Val Thr Arg Ser Ile Thr Lys His Asn Tyr Leu
       210                 215                 220
   gtg atg gat gtt gaa gat atc cct agg att att gag gaa gct ttc ttt     720
   Val Met Asp Val Glu Asp Ile Pro Arg Ile Ile Glu Glu Ala Phe Phe
   225                 230                 235                 240
   tta gct act tct ggt aga cct gga cct gtt ttg gtt gat gtt cct aaa     768
   Leu Ala Thr Ser Gly Arg Pro Gly Pro Val Leu Val Asp Val Pro Lys
                   245                 250                 255
   gat att caa caa cag ctt gcg att cct aat tgg gaa cag gct atg aga     816
   Asp Ile Gln Gln Gln Leu Ala Ile Pro Asn Trp Glu Gln Ala Met Arg
               260                 265                 270
   tta cct ggt tat atg tct agg atg cct aaa cct ccg gaa gat tct cat    864
   Leu Pro Gly Tyr Met Ser Arg Met Pro Lys Pro Pro Glu Asp Ser His
           275                 280                 285
   ttg gag cag att gtt agg ttg att tct gag tct aag aag cct gtg ttg    912
   Leu Glu Gln Ile Val Arg Leu Ile Ser Glu Ser Lys Lys Pro Val Leu
       290                 295                 300
   tat gtt ggt ggt ggt tgt ttg aat tct agc gat gaa ttg ggt agg ttt    960
   Tyr Val Gly Gly Gly Cys Leu Asn Ser Ser Asp Glu Leu Gly Arg Phe
   305                 310                 315                 320
   gtt gag ctt acg ggg atc cct gtt gcg agt acg ttg atg ggg ctg gga    1008
   Val Glu Leu Thr Gly Ile Pro Val Ala Ser Thr Leu Met Gly Leu Gly
                   325                 330                 335
   tct tat cct tgt gat gat gag ttg tcg tta cat atg ctt gga atg cat    1056
   Ser Tyr Pro Cys Asp Asp Glu Leu Ser Leu His Met Leu Gly Met His
               340                 345                 350
   ggg act gtg tat gca aat tac gct gtg gag cat agt gat ttg ttg ttg    1104
   Gly Thr Val Tyr Ala Asn Tyr Ala Val Glu His Ser Asp Leu Leu Leu
           355                 360                 365
   gcg ttt ggg gta agg ttt gat gat cgt gtc acg ggt aag ctt gag gct    1152
   Ala Phe Gly Val Arg Phe Asp Asp Arg Val Thr Gly Lys Leu Glu Ala
       370                 375                 380
   ttt gct agt agg gct aag att gtt cat att gat att gac tcg gct gag    1200
   Phe Ala Ser Arg Ala Lys Ile Val His Ile Asp Ile Asp Ser Ala Glu
   385                 390                 395                 400
   att ggg aag aat aag act cct cat gtg tct gtg tgt ggt gat gtt aag    1248
   Ile Gly Lys Asn Lys Thr Pro His Val Ser Val Cys Gly Asp Val Lys
                   405                 410                 415
   ctg gct ttg caa ggg atg aat aag gtt ctt gag aac cga gcg gag gag    1296
   Leu Ala Leu Gln Gly Met Asn Lys Val Leu Glu Asn Arg Ala Glu Glu
               420                 425                 430
   ctt aag ctt gat ttt gga gtt tgg agg aat gag ttg aac gta cag aaa    1344
   Leu Lys Leu Asp Phe Gly Val Trp Arg Asn Glu Leu Asn Val Gln Lys
           435                 440                 445
   cag aag ttt ccg ttg agc ttt aag acg ttt ggg gaa gct att cct cca    1392
   Gln Lys Phe Pro Leu Ser Phe Lys Thr Phe Gly Glu Ala Ile Pro Pro
       450                 455                 460
   cag tat gcg att aag gtc ctt gat gag ttg act gat gga aaa gcc ata    1440
   Gln Tyr Ala Ile Lys Val Leu Asp Glu Leu Thr Asp Gly Lys Ala Ile
   465                 470                 475                 480
   ata agt act ggt gtc ggg caa cat caa atg tgg gcg gcg cag ttc tac    1488
   Ile Ser Thr Gly Val Gly Gln His Gln Met Trp Ala Ala Gln Phe Tyr
                   485                 490                 495
   aat tac aag aaa cca agg cag tgg cta tca tca gga ggc ctt gga gct    1536
   Asn Tyr Lys Lys Pro Arg Gln Trp Leu Ser Ser Gly Gly Leu Gly Ala
               500                 505                 510
   atg gga ttt gga ctt cct gct gcg att gga gcg tct gtt gct aac cct    1584
   Met Gly Phe Gly Leu Pro Ala Ala Ile Gly Ala Ser Val Ala Asn Pro
           515                 520                 525
   gat gcg ata gtt gtg gat att gac gga gat gga agc ttt ata atg aat    1632
   Asp Ala Ile Val Val Asp Ile Asp Gly Asp Gly Ser Phe Ile Met Asn
       530                 535                 540
   gtg caa gag cta gcc act att cgt gta gag aat ctt cca gtg aag gta    1680
   Val Gln Glu Leu Ala Thr Ile Arg Val Glu Asn Leu Pro Val Lys Val
   545                 550                 555                 560
   ctt tta tta aac aac cag cat ctt ggc atg gtt atg caa tgg gaa gat    1728
   Leu Leu Leu Asn Asn Gln His Leu Gly Met Val Met Gln Trp Glu Asp
                   565                 570                 575
   cgg ttc tac aaa gct aac cga gct cac aca ttt ctc ggg gat ccg gct    1776
   Arg Phe Tyr Lys Ala Asn Arg Ala His Thr Phe Leu Gly Asp Pro Ala
               580                 585                 590
   cag gag gac gag ata ttc ccg aac atg ttg ctg ttt gca gca gct tgc    1824
   Gln Glu Asp Glu Ile Phe Pro Asn Met Leu Leu Phe Ala Ala Ala Cys
           595                 600                 605
   ggg att cca gcg gcg agg gtg aca aag aaa gca gat ctc cga gaa gct    1872
   Gly Ile Pro Ala Ala Arg Val Thr Lys Lys Ala Asp Leu Arg Glu Ala
       610                 615                 620
   att cag aca atg ctg gat aca cca gga cct tac ctg ttg gat gtg att    1920
   Ile Gln Thr Met Leu Asp Thr Pro Gly Pro Tyr Leu Leu Asp Val Ile
   625                 630                 635                 640
   tgt ccg cac caa gaa cat gtg ttg ccg atg atc ccg aat ggt ggc act    1968
   Cys Pro His Gln Glu His Val Leu Pro Met Ile Pro Asn Gly Gly Thr
                   645                 650                 655
   ttc aac gat gtc ata acg gaa gga gat ggc cgg att aaa tac tga        2013
   Phe Asn Asp Val Ile Thr Glu Gly Asp Gly Arg Ile Lys Tyr
               660                 665                 670
<210> 8
   <211> 670
   <212> PRT
   <213> Arabidopsis thaliana
<400> 8
   Met Ala Ala Ala Thr Thr Thr Thr Thr Thr Ser Ser Ser Ile Ser Phe
    1               5                  10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
   165 170 175
   Ser Gly Leu Ala Asp Ala Leu Leu Asp Ser Val Pro Leu Val Ala Ile
   180 185 190
   Thr Gly Gln Val Pro Arg Arg Met Ile Gly Thr Asp Ala Phe Gln Glu
   195 200 205
   Thr Pro Ile Val Glu Val Thr Arg Ser Ile Thr Lys His Asn Tyr Leu
   210 215 220
   Val Met Asp Val Glu Asp Ile Pro Arg Ile Ile Glu Glu Ala Phe Phe
   225 230 235 240
   Leu Ala Thr Ser Gly Arg Pro Gly Pro Val Leu Val Asp Val Pro Lys
   245 250 255
   Asp Ile Gln Gln Gln Leu Ala Ile Pro Asn Trp Glu Gln Ala Met Arg
   260 265 270
   Leu Pro Gly Tyr Met Ser Arg Met Pro Lys Pro Pro Glu Asp Ser His
   275 280 285
   Leu Glu Gln Ile Val Arg Leu Ile Ser Glu Ser Lys Lys Pro Val Leu
   290 295 300
   Tyr Val Gly Gly Gly Cys Leu Asn Ser Ser Asp Glu Leu Gly Arg Phe
   305 310 315 320
   Val Glu Leu Thr Gly Ile Pro Val Ala Ser Thr Leu Met Gly Leu Gly
   325 330 335
   Ser Tyr Pro Cys Asp Asp Glu Leu Ser Leu His Met Leu Gly Met His
   340 345 350
   Gly Thr Val Tyr Ala Asn Tyr Ala Val Glu His Ser Asp Leu Leu Leu
   355 360 365
   Ala Phe Gly Val Arg Phe Asp Asp Arg Val Thr Gly Lys Leu Glu Ala
   370 375 380
   Phe Ala Ser Arg Ala Lys Ile Val His Ile Asp Ile Asp Ser Ala Glu
   385 390 395 400
   Ile Gly Lys Asn Lys Thr Pro His Val Ser Val Cys Gly Asp Val Lys
   405 410 415
   Leu Ala Leu Gln Gly Met Asn Lys Val Leu Glu Asn Arg Ala Glu Glu
   420 425 430
   Leu Lys Leu Asp Phe Gly Val Trp Arg Asn Glu Leu Asn Val Gln Lys
   435 440 445
   Gln Lys Phe Pro Leu Ser Phe Lys Thr Phe Gly Glu Ala Ile Pro Pro
   450 455 460
   Gln Tyr Ala Ile Lys Val Leu Asp Glu Leu Thr Asp Gly Lys Ala Ile
   465 470 475 480
   Ile Ser Thr Gly Val Gly Gln His Gln Met Trp Ala Ala Gln Phe Tyr
   485 490 495
   Asn Tyr Lys Lys Pro Arg Gln Trp Leu Ser Ser Gly Gly Leu Gly Ala
   500 505 510
   Met Gly Phe Gly Leu Pro Ala Ala Ile Gly Ala Ser Val Ala Asn Pro
   515 520 525
   Asp Ala Ile Val Val Asp Ile Asp Gly Asp Gly Ser Phe Ile Met Asn
   530 535 540
   Val Gln Glu Leu Ala Thr Ile Arg Val Glu Asn Leu Pro Val Lys Val
   545                 550                 555                 560
   Leu Leu Leu Asn Asn Gln His Leu Gly Met Val Met Gln Trp Glu Asp
                   565                 570                 575
   Arg Phe Tyr Lys Ala Asn Arg Ala His Thr Phe Leu Gly Asp Pro Ala
               580                 585                 590
   Gln Glu Asp Glu Ile Phe Pro Asn Met Leu Leu Phe Ala Ala Ala Cys
           595                 600                 605
   Gly Ile Pro Ala Ala Arg Val Thr Lys Lys Ala Asp Leu Arg Glu Ala
       610                 615                 620
   Ile Gln Thr Met Leu Asp Thr Pro Gly Pro Tyr Leu Leu Asp Val Ile
   625                 630                 635                 640
   Cys Pro His Gln Glu His Val Leu Pro Met Ile Pro Asn Gly Gly Thr
                   645                 650                 655
   Phe Asn Asp Val Ile Thr Glu Gly Asp Gly Arg Ile Lys Tyr
               660                 665                 670
<210> 9
   <211> 29
   <212> DNA
   <213> Künstliche Sequenz
<220>
   <223> Beschreibung der künstlichen Sequenz: oligonucleotide primer
<400> 9
   gtcgacgaat tcgagagaca gagagacgg 29
<210> 10
   <211> 29
   <212> DNA
   <213> Künstliche Sequenz
<220>
   <223> Beschreibung der künstlichen Sequenz: oligonucleotide primer
<400> 10
   gtcgacggta ccgattcaag cttcactgc 29
<210> 11
   <211> 25
   <212> DNA
   <213> Künstliche Sequenz
<220>
   <223> Beschreibung der künstlichen Sequenz: oligonucleotide primer
<400> 11
   gagaattcga ttcaagcttc actgc 25
<210> 12
   <211> 22
   <212> DNA
   <213> Künstliche Sequenz
<220>
   <223> Beschreibung der künstlichen Sequenz: oligonucleotide primer
<400> 12
   ccatgggaga gacagagaga cg 22
<210> 13
   <211> 33
   <212> DNA
   <213> Künstliche Sequenz
<220>
   <223> Beschreibung der künstlichen Sequenz: oligonucleotide primer
<400> 13
   acggatccga gagacagaga gacggagaca aaa 33
<210> 14
   <211> 32
   <212> DNA
   <213> Künstliche Sequenz
<220>
   <223> Beschreibung der künstlichen Sequenz: oligonucleotide primer
<400> 14
   gcggatccaa cactcttaac accaaatcaa ca 32
<210> 15
   <211> 44
   <212> DNA
   <213> Künstliche Sequenz
<220>
   <223> Beschreibung der künstlichen Sequenz: oligonucleotide primer
<400> 15
   gtcgacggat ccggttgatc agaagaagaa gaagaagatg aact 44
<210> 16
   <211> 41
   <212> DNA
   <213> Künstliche Sequenz
<220>
   <223> Beschreibung der künstlichen Sequenz: oligonucleotide primer
<400> 16
   gtcgactcta gattcattat ttcgattttg atttcgtgac c 41
<210> 17
   <211> 50
   <212> DNA
   <213> Künstliche Sequenz
<220>
   <223> Beschreibung der künstlichen Sequenz: oligonucleotide primer
<400> 17
   aagtcgacgg atccataacc aaaagaactc tgatcatgta cgtacccatt 50
<210> 18
   <211> 50
   <212> DNA
   <213> Künstliche Sequenz
<220>
   <223> Beschreibung der künstlichen Sequenz: oligonucleotide primer
<400> 18
   agacgtcgac tctagatgaa atcgaaattc agagttttga tagtgagagc 50
<210> 19
   <211> 30
   <212> DNA
   <213> Künstliche Sequenz
<220>
   <223> Beschreibung der künstlichen Sequenz: oligonucleotide primer
<400> 19
   ccaaaccatg gtaagtttgt ctaaagctta 30
<210> 20
   <211> 31
   <212> DNA
   <213> Künstliche Sequenz
<220>
   <223> Beschreibung der künstlichen Sequenz: oligonucleotide primer
<400> 20
   cggatccttt tgtgtttcgt cttctctcac g 31
<210> 21
   <211> 25
   <212> DNA
   <213> Künstliche Sequenz
<220>
   <223> Beschreibung der künstlichen Sequenz: oligonucleotide primer
<400> 21
   gtctagaggc aaaccaccga gtgtt 25
<210> 22
   <211> 31
   <212> DNA
   <213> Künstliche Sequenz
<220>
   <223> Beschreibung der künstlichen Sequenz: oligonucleotide primer
<400> 22
   cggtacctgt ttccagaaaa ttttgattca g 31
<210> 23
   <211> 7554
   <212> DNA
   <213> Künstliche Sequenz
<220>
   <223> Beschreibung der künstlichen Sequenz: binary plant expression vector
<400> 23
   ttccatggac atacaaatgg acgaacggat aaaccttttc acgccctttt aaatatccga 60
   ttattctaat aaacgctctt ttctcttagg tttacccgcc aatatatcct gtcaaacact 120
   gatagtttaa actgaaggcg ggaaacgaca atcagatcta gtaggaaaca gctatgacca 180
   tgattacgcc aagcttgcat gcctgcaggt cgactctaga ctagtggatc cgatatcgcc 240
   cgggctcgag gtaccgagct cgaattcact ggccgtcgtt ttacaacgac tcagagcttg 300
   acaggaggcc cgatctagta acatagatga caccgcgcgc gataatttat cctagtttgc 360
   gcgctatatt ttgttttcta tcgcgtatta aatgtataat tgcgggactc taatcataaa 420
   aacccatctc ataaataacg tcatgcatta catgttaatt attacatgct taacgtaatt 480
   caacagaaat tatatgataa tcatcgcaag accggcaaca ggattcaatc ttaagaaact 540
   ttattgccaa atgtttgaac gatcggggat catccgggtc tgtggcggga actccacgaa 600
   aatatccgaa cgcagcaaga tctaagcttg ggtcccgctc agaagaactc gtcaagaagg 660
   cgatagaagg cgatgcgctg cgaatcggga gcggcgatac cgtaaagcac gaggaagcgg 720
   tcagcccatt cgccgccaag ctcttcagca atatcacggg tagccaacgc tatgtcctga 780
   tagcggtccg ccacacccag ccggccacag tcgatgaatc cagaaaagcg gccattttcc 840
   accatgatat tcggcaagca ggcatcgcca tgggtcacga cgagatcctc gccgtcgggc 900
   atgcgcgcct tgagcctggc gaacagttcg gctggcgcga gcccctgatg ctcttcgtcc 960
   agatcatcct gatcgacaag accggcttcc atccgagtac gtgctcgctc gatgcgatgt 1020
   ttcgcttggt ggtcgaatgg gcaggtagcc ggatcaagcg tatgcagccg ccgcattgca 1080
   tcagccatga tggatacttt ctcggcagga gcaaggtgag atgacaggag atcctgcccc 1140
   ggcacttcgc ccaatagcag ccagtccctt cccgcttcag tgacaacgtc gagcacagct 1200
   gcgcaaggaa cgcccgtcgt ggccagccac gatagccgcg ctgcctcgtc ctgcagttca 1260
   ttcagggcac cggacaggtc ggtcttgaca aaaagaaccg ggcgcccctg cgctgacagc 1320
   cggaacacgg cggcatcaga gcagccgatt gtctgttgtg cccagtcata gccgaatagc 1380
   ctctccaccc aagcggccgg agaacctgcg tgcaatccat cttgttcaat catgcgaaac 1440
   gatccagatc cggtgcagat tatttggatt gagagtgaat atgagactct aattggatac 1500
   cgaggggaat ttatggaacg tcagtggagc atttttgaca agaaatattt gctagctgat 1560
   agtgacctta ggcgactttt gaacgcgcaa taatggtttc tgacgtatgt gcttagctca 1620
   ttaaactcca gaaacccgcg gctgagtggc tccttcaacg ttgcggttct gtcagttcca 1680
   aacgtaaaac ggcttgtccc gcgtcatcgg cgggggtcat aacgtgactc ccttaattct 1740
   ccgctcatga tcagattgtc gtttcccgcc ttcagtttaa actatcagtg tttgacagga 1800
   tcctgcttgg taataattgt cattagattg tttttatgca tagatgcact cgaaatcagc 1860
   caattttaga caagtatcaa acggatgtta attcagtaca ttaaagacgt ccgcaatgtg 1920
   ttattaagtt gtctaagcgt caatttgttt acaccacaat atatcctgcc accagccagc 1980
   caacagctcc ccgaccggca gctcggcaca aaatcaccac gcgttaccac cacgccggcc 2040
   ggccgcatgg tgttgaccgt gttcgccggc attgccgagt tcgagcgttc cctaatcatc 2100
   gaccgcaccc ggagcgggcg cgaggccgcc aaggcccgag gcgtgaagtt tggcccccgc 2160
   cctaccctca ccccggcaca gatcgcgcac gcccgcgagc tgatcgacca ggaaggccgc 2220
   accgtgaaag aggcggctgc actgcttggc gtgcatcgct cgaccctgta ccgcgcactt 2280
   gagcgcagcg aggaagtgac gcccaccgag gccaggcggc gcggtgcctt ccgtgaggac 2340
   gcattgaccg aggccgacgc cctggcggcc gccgagaatg aacgccaaga ggaacaagca 2400
   tgaaaccgca ccaggacggc caggacgaac cgtttttcat taccgaagag atcgaggcgg 2460
   agatgatcgc ggccgggtac gtgttcgagc cgcccgcgca cgtctcaacc gtgcggctgc 2520
   atgaaatcct ggccggtttg tctgatgcca agctggcggc ctggccggcc agcttggccg 2580
   ctgaagaaac cgagcgccgc cgtctaaaaa ggtgatgtgt atttgagtaa aacagcttgc 2640
   gtcatgcggt cgctgcgtat atgatgcgat gagtaaataa acaaatacgc aaggggaacg 2700
   catgaaggtt atcgctgtac ttaaccagaa aggcgggtca ggcaagacga ccatcgcaac 2760
   ccatctagcc cgcgccctgc aactcgccgg ggccgatgtt ctgttagtcg attccgatcc 2820
   ccagggcagt gcccgcgatt gggcggccgt gcgggaagat caaccgctaa ccgttgtcgg 2880
   catcgaccgc ccgacgattg accgcgacgt gaaggccatc ggccggcgcg acttcgtagt 2940
   gatcgacgga gcgccccagg cggcggactt ggctgtgtcc gcgatcaagg cagccgactt 3000
   cgtgctgatt ccggtgcagc caagccctta cgacatatgg gccaccgccg acctggtgga 3060
   gctggttaag cagcgcattg aggtcacgga tggaaggcta caagcggcct ttgtcgtgtc 3120
   gcgggcgatc aaaggcacgc gcatcggcgg tgaggttgcc gaggcgctgg ccgggtacga 3180
   gctgcccatt cttgagtccc gtatcacgca gcgcgtgagc tacccaggca ctgccgccgc 3240
   cggcacaacc gttcttgaat cagaacccga gggcgacgct gcccgcgagg tccaggcgct 3300
   ggccgctgaa attaaatcaa aactcatttg agttaatgag gtaaagagaa aatgagcaaa 3360
   agcacaaaca cgctaagtgc cggccgtccg agcgcacgca gcagcaaggc tgcaacgttg 3420
   gccagcctgg cagacacgcc agccatgaag cgggtcaact ttcagttgcc ggcggaggat 3480
   cacaccaagc tgaagatgta cgcggtacgc caaggcaaga ccattaccga gctgctatct 3540
   gaatacatcg cgcagctacc agagtaaatg agcaaatgaa taaatgagta gatgaatttt 3600
   agcggctaaa ggaggcggca tggaaaatca agaacaacca ggcaccgacg ccgtggaatg 3660
   ccccatgtgt ggaggaacgg gcggttggcc aggcgtaagc ggctgggttg tctgccggcc 3720
   ctgcaatggc actggaaccc ccaagcccga ggaatcggcg tgagcggtcg caaaccatcc 3780
   ggcccggtac aaatcggcgc ggcgctgggt gatgacctgg tggagaagtt gaaggccgcg 3840
   caggccgccc agcggcaacg catcgaggca gaagcacgcc ccggtgaatc gtggcaagcg 3900
   gccgctgatc gaatccgcaa agaatcccgg caaccgccgg cagccggtgc gccgtcgatt 3960
   aggaagccgc ccaagggcga cgagcaacca gattttttcg ttccgatgct ctatgacgtg 4020
   ggcacccgcg atagtcgcag catcatggac gtggccgttt tccgtctgtc gaagcgtgac 4080
   cgacgagctg gcgaggtgat ccgctacgag cttccagacg ggcacgtaga ggtttccgca 4140
   gggccggccg gcatggccag tgtgtgggat tacgacctgg tactgatggc ggtttcccat 4200
   ctaaccgaat ccatgaaccg ataccgggaa gggaagggag acaagcccgg ccgcgtgttc 4260
   cgtccacacg ttgcggacgt actcaagttc tgccggcgag ccgatggcgg aaagcagaaa 4320
   gacgacctgg tagaaacctg cattcggtta aacaccacgc acgttgccat gcagcgtacg 4380
   aagaaggcca agaacggccg cctggtgacg gtatccgagg gtgaagcctt gattagccgc 4440
   tacaagatcg taaagagcga aaccgggcgg ccggagtaca tcgagatcga gctagctgat 4500
   tggatgtacc gcgagatcac agaaggcaag aacccggacg tgctgacggt tcaccccgat 4560
   tactttttga tcgatcccgg catcggccgt tttctctacc gcctggcacg ccgcgccgca 4620
   ggcaaggcag aagccagatg gttgttcaag acgatctacg aacgcagtgg cagcgccgga 4680
   gagttcaaga agttctgttt caccgtgcgc aagctgatcg ggtcaaatga cctgccggag 4740
   tacgatttga aggaggaggc ggggcaggct ggcccgatcc tagtcatgcg ctaccgcaac 4800
   ctgatcgagg gcgaagcatc cgccggttcc taatgtacgg agcagatgct agggcaaatt 4860
   gccctagcag gggaaaaagg tcgaaaaggt ctctttcctg tggatagcac gtacattggg 4920
   aacccaaagc cgtacattgg gaaccggaac ccgtacattg ggaacccaaa gccgtacatt 4980
   gggaaccggt cacacatgta agtgactgat ataaaagaga aaaaaggcga tttttccgcc 5040
   taaaactctt taaaacttat taaaactctt aaaacccgcc tggcctgtgc ataactgtct 5100
   ggccagcgca cagccgaaga gctgcaaaaa gcgcctaccc ttcggtcgct gcgctcccta 5160
   cgccccgccg cttcgcgtcg gcctatcgcg gccgctggcc gctcaaaaat ggctggccta 5220
   cggccaggca atctaccagg gcgcggacaa gccgcgccgt cgccactcga ccgccggcgc 5280
   ccacatcaag gcaccctgcc tcgcgcgttt cggtgatgac ggtgaaaacc tctgacacat 5340
   gcagctcccg gagacggtca cagcttgtct gtaagcggat gccgggagca gacaagcccg 5400
   tcagggcgcg tcagcgggtg ttggcgggtg tcggggcgca gccatgaccc agtcacgtag 5460
   cgatagcgga gtgtatactg gcttaactat gcggcatcag agcagattgt actgagagtg 5520
   caccatatgc ggtgtgaaat accgcacaga tgcgtaagga gaaaataccg catcaggcgc 5580
   tcttccgctt cctcgctcac tgactcgctg cgctcggtcg ttcggctgcg gcgagcggta 5640
   tcagctcact caaaggcggt aatacggtta tccacagaat caggggataa cgcaggaaag 5700
   aacatgtgag caaaaggcca gcaaaaggcc aggaaccgta aaaaggccgc gttgctggcg 5760
   tttttccata ggctccgccc ccctgacgag catcacaaaa atcgacgctc aagtcagagg 5820
   tggcgaaacc cgacaggact ataaagatac caggcgtttc cccctggaag ctccctcgtg 5880
   cgctctcctg ttccgaccct gccgcttacc ggatacctgt ccgcctttct cccttcggga 5940
   agcgtggcgc tttctcatag ctcacgctgt aggtatctca gttcggtgta ggtcgttcgc 6000
   tccaagctgg gctgtgtgca cgaacccccc gttcagcccg accgctgcgc cttatccggt 6060
   aactatcgtc ttgagtccaa cccggtaaga cacgacttat cgccactggc agcagccact 6120
   ggtaacagga ttagcagagc gaggtatgta ggcggtgcta cagagttctt gaagtggtgg 6180
   cctaactacg gctacactag aaggacagta tttggtatct gcgctctgct gaagccagtt 6240
   accttcggaa aaagagttgg tagctcttga tccggcaaac aaaccaccgc tggtagcggt 6300
   ggtttttttg tttgcaagca gcagattacg cgcagaaaaa aaggatctca agaagatcct 6360
   ttgatctttt ctacggggtc tgacgctcag tggaacgaaa actcacgtta agggattttg 6420
   gtcatgcatg atatatctcc caatttgtgt agggcttatt atgcacgctt aaaaataata 6480
   aaagcagact tgacctgata gtttggctgt gagcaattat gtgcttagtg catctaacgc 6540
   ttgagttaag ccgcgccgcg aagcggcgtc ggcttgaacg aatttctagc tagacattat 6600
   ttgccgacta ccttggtgat ctcgcctttc acgtagtgga caaattcttc caactgatct 6660
   gcgcgcgagg ccaagcgatc ttcttcttgt ccaagataag cctgtctagc ttcaagtatg 6720
   acgggctgat actgggccgg caggcgctcc attgcccagt cggcagcgac atccttcggc 6780
   gcgattttgc cggttactgc gctgtaccaa atgcgggaca acgtaagcac tacatttcgc 6840
   tcatcgccag cccagtcggg cggcgagttc catagcgtta aggtttcatt tagcgcctca 6900
   aatagatcct gttcaggaac cggatcaaag agttcctccg ccgctggacc taccaaggca 6960
   acgctatgtt ctcttgcttt tgtcagcaag atagccagat caatgtcgat cgtggctggc 7020
   tcgaagatac ctgcaagaat gtcattgcgc tgccattctc caaattgcag ttcgcgctta 7080
   gctggataac gccacggaat gatgtcgtcg tgcacaacaa tggtgacttc tacagcgcgg 7140
   agaatctcgc tctctccagg ggaagccgaa gtttccaaaa ggtcgttgat caaagctcgc 7200
   cgcgttgttt catcaagcct tacggtcacc gtaaccagca aatcaatatc actgtgtggc 7260
   ttcaggccgc catccactgc ggagccgtac aaatgtacgg ccagcaacgt cggttcgaga 7320
   tggcgctcga tgacgccaac tacctctgat agttgagtcg atacttcggc gatcaccgct 7380
   tcccccatga tgtttaactt tgttttaggg cgactgccct gctgcgtaac atcgttgctg 7440
   ctccataaca tcaaacatcg acccacggcg taacgcgctt gctgcttgga tgcccgaggc 7500
   atagactgta ccccaaaaaa acagtcataa caagccatga aaaccgccac tgcg       7554
<210> 24
   <211> 8327
   <212> DNA
   <213> Künstliche Sequenz
<220>
   <223> Beschreibung der künstlichen Sequenz: binary plant expression vector
<400> 24
   ttccatggac atacaaatgg acgaacggat aaaccttttc acgccctttt aaatatccga 60
   ttattctaat aaacgctctt ttctcttagg tttacccgcc aatatatcct gtcaaacact 120
   gatagtttaa actgaaggcg ggaaacgaca atcagatcta gtaggaaaca gctatgacca 180
   tgattacgcc aagcttgcat gccgatcccc cctgcagata gactatacta tgttttagcc 240
   tgcctgctgg ctagctacta tgttatgtta tgttgtaaaa taaacacctg ctaaggtata 300
   tctatctata ttttagcatg gctttctcaa taaattgtct ttccttatcg tttactatct 360
   tatacctaat aatgaaataa taatatcaca tatgaggaac ggggcaggtt taggcatata 420
   tatacgagtg tagggcggag tgggggggta cggtcgactc tagactagtg gatccnnntc 480
   aagaaggcga tagaaggcga tgcgctgcga atcgggagcg gcgataccgt aaagcacgag 540
   gaagcggtca gcccattcgc cgccaagctc ttcagcaata tcacgggtag ccaacgctat 600
   gtcctgatag cggtccgcca cacccagccg gccacagtcg atgaatccag aaaagcggcc 660
   attttccacc atgatattcg gcaagcaggc atcgccatgg gtcacgacga gatcctcgcc 720
   gtcgggcatg cgcgccttga gcctggcgaa cagttcggct ggcgcgagcc cctgatgctc 780
   ttcgtccaga tcatcctgat cgacaagacc ggcttccatc cgagtacgtg ctcgctcgat 840
   gcgatgtttc gcttggtggt cgaatgggca ggtagccgga tcaagcgtat gcagccgccg 900
   cattgcatca gccatgatgg atactttctc ggcaggagca aggtgagatg acaggagatc 960
   ctgccccggc acttcgccca atagcagcca gtcccttccc gcttcagtga caacgtcgag 1020
   cacagctgcg caaggaacgc ccgtcgtggc cagccacgat agccgcgctg cctcgtcctg 1080
   cagttcattc agggcaccgg acaggtcggt cttgacaaaa agaaccgggc gcccctgcgc 1140
   tgacagccgg aacacggcgg catcagagca gccgattgtc tgttgtgccc agtcatagcc 1200
   gaatagcctc tccacccaag cggccggaga acctgcgtgc aatccatctt gttcaatcat 1260
   nnnagatccg atatcgcccg ggctcgaggt accgagctcg aattcactgg ccgtcgtttt 1320
   acaacgactc agccagcttg acaggaggcc cgatctagta acatagatga caccgcgcgc 1380
   gataatttat cctagtttgc gcgctatatt ttgttttcta tcgcgtatta aatgtataat 1440
   tgcgggactc taatcataaa aacccatctc ataaataacg tcatgcatta catgttaatt 1500
   attacatgct taacgtaatt caacagaaat tatatgataa tcatcgcaag accggcaaca 1560
   ggattcaatc ttaagaaact ttattgccaa atgtttgaac gatcggggat catccgggtc 1620
   tgtggcggga actccacgaa aatatccgaa cgcagcaaga tcggtcgatc gactcagatc 1680
   tgggtaactg gcctaactgg ccttggagga gctggcaact caaaatccct ttgccaaaaa 1740
   ccaacatcat gccatccacc atgcttgtat ccagccgcgc gcaatgtacc ccgcgctgtg 1800
   tatcccaaag cctcatgcaa cctaacagat ggatcgtttg gaaggcctat aacagcaacc 1860
   acagacttaa aaccttgcgc ctccatagac ttaagcaaat gtgtgtacaa tgtagatcct 1920
   aggcccaacc tttgatgcct atgtgacacg taaacagtac tctcaactgt ccaatcgtaa 1980
   gcgttcctag ccttccaggg cccagcgtaa gcaataccag ccacaacacc ctcaacctca 2040
   gcaaccaacc aagggtatct atcttgcaac ctctctaggt catcaatcca ctcttgtggt 2100
   gtttgtggct ctgtcctaaa gttcactgta gacgtctcaa tgtaatggtt aacgatgtca 2160
   caaaccgcgg ccatatcggc tgctgtagct ggcctaatct caactggtct cctctccgga 2220
   gacatgtcga gattatttgg attgagagtg aatatgagac tctaattgga taccgagggg 2280
   aatttatgga acgtcagtgg agcatttttg acaagaaata tttgctagct gatagtgacc 2340
   ttaggcgact tttgaacgcg caataatggt ttctgacgta tgtgcttagc tcattaaact 2400
   ccagaaaccc gcggctgagt ggctccttca acgttgcggt tctgtcagtt ccaaacgtaa 2460
   aacggcttgt cccgcgtcat cggcgggggt cataacgtga ctcccttaat tctccgctca 2520
   tgatcagatt gtcgtttccc gccttcagtt taaactatca gtgtttgaca ggatcctgct 2580
   tggtaataat tgtcattaga ttgtttttat gcatagatgc actcgaaatc agccaatttt 2640
   agacaagtat caaacggatg ttaattcagt acattaaaga cgtccgcaat gtgttattaa 2700
   gttgtctaag cgtcaatttg tttacaccac aatatatcct gccaccagcc agccaacagc 2760
   tccccgaccg gcagctcggc acaaaatcac cacgcgttac caccacgccg gccggccgca 2820
   tggtgttgac cgtgttcgcc ggcattgccg agttcgagcg ttccctaatc atcgaccgca 2880
   cccggagcgg gcgcgaggcc gccaaggccc gaggcgtgaa gtttggcccc cgccctaccc 2940
   tcaccccggc acagatcgcg cacgcccgcg agctgatcga ccaggaaggc cgcaccgtga 3000
   aagaggcggc tgcactgctt ggcgtgcatc gctcgaccct gtaccgcgca cttgagcgca 3060
   gcgaggaagt gacgcccacc gaggccaggc ggcgcggtgc cttccgtgag gacgcattga 3120
   ccgaggccga cgccctggcg gccgccgaga atgaacgcca agaggaacaa gcatgaaacc 3180
   gcaccaggac ggccaggacg aaccgttttt cattaccgaa gagatcgagg cggagatgat 3240
   cgcggccggg tacgtgttcg agccgcccgc gcacgtctca accgtgcggc tgcatgaaat 3300
   cctggccggt ttgtctgatg ccaagctggc ggcctggccg gccagcttgg ccgctgaaga 3360
   aaccgagcgc cgccgtctaa aaaggtgatg tgtatttgag taaaacagct tgcgtcatgc 3420
   ggtcgctgcg tatatgatgc gatgagtaaa taaacaaata cgcaagggga acgcatgaag 3480
   gttatcgctg tacttaacca gaaaggcggg tcaggcaaga cgaccatcgc aacccatcta 3540
   gcccgcgccc tgcaactcgc cggggccgat gttctgttag tcgattccga tccccagggc 3600
   agtgcccgcg attgggcggc cgtgcgggaa gatcaaccgc taaccgttgt cggcatcgac 3660
   cgcccgacga ttgaccgcga cgtgaaggcc atcggccggc gcgacttcgt agtgatcgac 3720
   ggagcgcccc aggcggcgga cttggctgtg tccgcgatca aggcagccga cttcgtgctg 3780
   attccggtgc agccaagccc ttacgacata tgggccaccg ccgacctggt ggagctggtt 3840
   aagcagcgca ttgaggtcac ggatggaagg ctacaagcgg cctttgtcgt gtcgcgggcg 3900
   atcaaaggca cgcgcatcgg cggtgaggtt gccgaggcgc tggccgggta cgagctgccc 3960
   attcttgagt cccgtatcac gcagcgcgtg agctacccag gcactgccgc cgccggcaca 4020
   accgttcttg aatcagaacc cgagggcgac gctgcccgcg aggtccaggc gctggccgct 4080
   gaaattaaat caaaactcat ttgagttaat gaggtaaaga gaaaatgagc aaaagcacaa 4140
   acacgctaag tgccggccgt ccgagcgcac gcagcagcaa ggctgcaacg ttggccagcc 4200
   tggcagacac gccagccatg aagcgggtca actttcagtt gccggcggag gatcacacca 4260
   agctgaagat gtacgcggta cgccaaggca agaccattac cgagctgcta tctgaataca 4320
   tcgcgcagct accagagtaa atgagcaaat gaataaatga gtagatgaat tttagcggct 4380
   aaaggaggcg gcatggaaaa tcaagaacaa ccaggcaccg acgccgtgga atgccccatg 4440
   tgtggaggaa cgggcggttg gccaggcgta agcggctggg ttgtctgccg gccctgcaat 4500
   ggcactggaa cccccaagcc cgaggaatcg gcgtgagcgg tcgcaaacca tccggcccgg 4560
   tacaaatcgg cgcggcgctg ggtgatgacc tggtggagaa gttgaaggcc gcgcaggccg 4620
   cccagcggca acgcatcgag gcagaagcac gccccggtga atcgtggcaa gcggccgctg 4680
   atcgaatccg caaagaatcc cggcaaccgc cggcagccgg tgcgccgtcg attaggaagc 4740
   cgcccaaggg cgacgagcaa ccagattttt tcgttccgat gctctatgac gtgggcaccc 4800
   gcgatagtcg cagcatcatg gacgtggccg ttttccgtct gtcgaagcgt gaccgacgag 4860
   ctggcgaggt gatccgctac gagcttccag acgggcacgt agaggtttcc gcagggccgg 4920
   ccggcatggc cagtgtgtgg gattacgacc tggtactgat ggcggtttcc catctaaccg 4980
   aatccatgaa ccgataccgg gaagggaagg gagacaagcc cggccgcgtg ttccgtccac 5040
   acgttgcgga cgtactcaag ttctgccggc gagccgatgg cggaaagcag aaagacgacc 5100
   tggtagaaac ctgcattcgg ttaaacacca cgcacgttgc catgcagcgt acgaagaagg 5160
   ccaagaacgg ccgcctggtg acggtatccg agggtgaagc cttgattagc cgctacaaga 5220
   tcgtaaagag cgaaaccggg cggccggagt acatcgagat cgagctagct gattggatgt 5280
   accgcgagat cacagaaggc aagaacccgg acgtgctgac ggttcacccc gattactttt 5340
   tgatcgatcc cggcatcggc cgttttctct accgcctggc acgccgcgcc gcaggcaagg 5400
   cagaagccag atggttgttc aagacgatct acgaacgcag tggcagcgcc ggagagttca 5460
   agaagttctg tttcaccgtg cgcaagctga tcgggtcaaa tgacctgccg gagtacgatt 5520
   tgaaggagga ggcggggcag gctggcccga tcctagtcat gcgctaccgc aacctgatcg 5580
   agggcgaagc atccgccggt tcctaatgta cggagcagat gctagggcaa attgccctag 5640
   caggggaaaa aggtcgaaaa ggtctctttc ctgtggatag cacgtacatt gggaacccaa 5700
   agccgtacat tgggaaccgg aacccgtaca ttgggaaccc aaagccgtac attgggaacc 5760
   ggtcacacat gtaagtgact gatataaaag agaaaaaagg cgatttttcc gcctaaaact 5820
   ctttaaaact tattaaaact cttaaaaccc gcctggcctg tgcataactg tctggccagc 5880
   gcacagccga agagctgcaa aaagcgccta cccttcggtc gctgcgctcc ctacgccccg 5940
   ccgcttcgcg tcggcctatc gcggccgctg gccgctcaaa aatggctggc ctacggccag 6000
   gcaatctacc agggcgcgga caagccgcgc cgtcgccact cgaccgccgg cgcccacatc 6060
   aaggcaccct gcctcgcgcg tttcggtgat gacggtgaaa acctctgaca catgcagctc 6120
   ccggagacgg tcacagcttg tctgtaagcg gatgccggga gcagacaagc ccgtcagggc 6180
   gcgtcagcgg gtgttggcgg gtgtcggggc gcagccatga cccagtcacg tagcgatagc 6240
   ggagtgtata ctggcttaac tatgcggcat cagagcagat tgtactgaga gtgcaccata 6300
   tgcggtgtga aataccgcac agatgcgtaa ggagaaaata ccgcatcagg cgctcttccg 6360
   cttcctcgct cactgactcg ctgcgctcgg tcgttcggct gcggcgagcg gtatcagctc 6420
   actcaaaggc ggtaatacgg ttatccacag aatcagggga taacgcagga aagaacatgt 6480
   gagcaaaagg ccagcaaaag gccaggaacc gtaaaaaggc cgcgttgctg gcgtttttcc 6540
   ataggctccg cccccctgac gagcatcaca aaaatcgacg ctcaagtcag aggtggcgaa 6600
   acccgacagg actataaaga taccaggcgt ttccccctgg aagctccctc gtgcgctctc 6660
   ctgttccgac cctgccgctt accggatacc tgtccgcctt tctcccttcg ggaagcgtgg 6720
   cgctttctca tagctcacgc tgtaggtatc tcagttcggt gtaggtcgtt cgctccaagc 6780
   tgggctgtgt gcacgaaccc cccgttcagc ccgaccgctg cgccttatcc ggtaactatc 6840
   gtcttgagtc caacccggta agacacgact tatcgccact ggcagcagcc actggtaaca 6900
   ggattagcag agcgaggtat gtaggcggtg ctacagagtt cttgaagtgg tggcctaact 6960
   acggctacac tagaaggaca gtatttggta tctgcgctct gctgaagcca gttaccttcg 7020
   gaaaaagagt tggtagctct tgatccggca aacaaaccac cgctggtagc ggtggttttt 7080
   ttgtttgcaa gcagcagatt acgcgcagaa aaaaaggatc tcaagaagat cctttgatct 7140
   tttctacggg gtctgacgct cagtggaacg aaaactcacg ttaagggatt ttggtcatgc 7200
   atgatatatc tcccaatttg tgtagggctt attatgcacg cttaaaaata ataaaagcag 7260
   acttgacctg atagtttggc tgtgagcaat tatgtgctta gtgcatctaa cgcttgagtt 7320
   aagccgcgcc gcgaagcggc gtcggcttga acgaatttct agctagacat tatttgccga 7380
   ctaccttggt gatctcgcct ttcacgtagt ggacaaattc ttccaactga tctgcgcgcg 7440
   aggccaagcg atcttcttct tgtccaagat aagcctgtct agcttcaagt atgacgggct 7500
   gatactgggc cggcaggcgc tccattgccc agtcggcagc gacatccttc ggcgcgattt 7560
   tgccggttac tgcgctgtac caaatgcggg acaacgtaag cactacattt cgctcatcgc 7620
   cagcccagtc gggcggcgag ttccatagcg ttaaggtttc atttagcgcc tcaaatagat 7680
   cctgttcagg aaccggatca aagagttcct ccgccgctgg acctaccaag gcaacgctat 7740
   gttctcttgc ttttgtcagc aagatagcca gatcaatgtc gatcgtggct ggctcgaaga 7800
   tacctgcaag aatgtcattg cgctgccatt ctccaaattg cagttcgcgc ttagctggat 7860
   aacgccacgg aatgatgtcg tcgtgcacaa caatggtgac ttctacagcg cggagaatct 7920
   cgctctctcc aggggaagcc gaagtttcca aaaggtcgtt gatcaaagct cgccgcgttg 7980
   tttcatcaag ccttacggtc accgtaacca gcaaatcaat atcactgtgt ggcttcaggc 8040
   cgccatccac tgcggagccg tacaaatgta cggccagcaa cgtcggttcg agatggcgct 8100
   cgatgacgcc aactacctct gatagttgag tcgatacttc ggcgatcacc gcttccccca 8160
   tgatgtttaa ctttgtttta gggcgactgc cctgctgcgt aacatcgttg ctgctccata 8220
   acatcaaaca tcgacccacg gcgtaacgcg cttgctgctt ggatgcccga ggcatagact 8280
   gtaccccaaa aaaacagtca taacaagcca tgaaaaccgc cactgcg              8327
<210> 25
   <211> 8441
   <212> DNA
   <213> Künstliche Sequenz
<220>
   <223> Beschreibung der künstlichen Sequenz: binary plant expression vector
<400> 25
   ttccatggac atacaaatgg acgaacggat aaaccttttc acgccctttt aaatatccga 60
   ttattctaat aaacgctctt ttctcttagg tttacccgcc aatatatcct gtcaaacact 120
   gatagtttaa actgaaggcg ggaaacgaca atcagatcta gtaggaaaca gctatgacca 180
   tgattacgcc aagcttgcat gccagcttga caggaggccc gatctagtaa catagatgac 240
   accgcgcgcg ataatttatc ctagtttgcg cgctatattt tgttttctat cgcgtattaa 300
   atgtataatt gcgggactct aatcataaaa acccatctca taaataacgt catgcattac 360
   atgttaatta ttacatgctt aacgtaattc aacagaaatt atatgataat catcgcaaga 420
   ccggcaacag gattcaatct taagaaactt tattgccaaa tgtttgaacg atcggggatc 480
   atccgggtct gtggcgggaa ctccacgaaa atatccgaac gcagcaagat cggtcgatcg 540
   actcagatct gggtaactgg cctaactggc cttggaggag ctggcaactc aaaatccctt 600
   tgccaaaaac caacatcatg ccatccacca tgcttgtatc cagccgcgcg caatgtaccc 660
   cgcgctgtgt atcccaaagc ctcatgcaac ctaacagatg gatcgtttgg aaggcctata 720
   acagcaacca cagacttaaa accttgcgcc tccatagact taagcaaatg tgtgtacaat 780
   gtagatccta ggcccaacct ttgatgccta tgtgacacgt aaacagtact ctcaactgtc 840
   caatcgtaag cgttcctagc cttccagggc ccagcgtaag caataccagc cacaacaccc 900
   tcaacctcag caaccaacca agggtatcta tcttgcaacc tctctaggtc atcaatccac 960
   tcttgtggtg tttgtggctc tgtcctaaag ttcactgtag acgtctcaat gtaatggtta 1020
   acgatgtcac aaaccgcggc catatcggct gctgtagctg gcctaatctc aactggtctc 1080
   ctctccggag acatgtcgac tctagactag tggatccgat atcgcccggg ctcgaggtac 1140
   cgagctcgaa ttcactggcc gtcgttttac aacgactcag agcttgacag gaggcccgat 1200
   ctagtaacat agatgacacc gcgcgcgata atttatccta gtttgcgcgc tatattttgt 1260
   tttctatcgc gtattaaatg tataattgcg ggactctaat cataaaaacc catctcataa 1320
   ataacgtcat gcattacatg ttaattatta catgcttaac gtaattcaac agaaattata 1380
   tgataatcat cgcaagaccg gcaacaggat tcaatcttaa gaaactttat tgccaaatgt 1440
   ttgaacgatc ggggatcatc cgggtctgtg gcgggaactc cacgaaaata tccgaacgca 1500
   gcaagatcta gagcttgggt cccgctcaga agaactcgtc aagaaggcga tagaaggcga 1560
   tgcgctgcga atcgggagcg gcgataccgt aaagcacgag gaagcggtca gcccattcgc 1620
   cgccaagctc ttcagcaata tcacgggtag ccaacgctat gtcctgatag cggtccgcca 1680
   cacccagccg gccacagtcg atgaatccag aaaagcggcc attttccacc atgatattcg 1740
   gcaagcaggc atcgccatgg gtcacgacga gatcctcgcc gtcgggcatg cgcgccttga 1800
   gcctggcgaa cagttcggct ggcgcgagcc cctgatgctc ttcgtccaga tcatcctgat 1860
   cgacaagacc ggcttccatc cgagtacgtg ctcgctcgat gcgatgtttc gcttggtggt 1920
   cgaatgggca ggtagccgga tcaagcgtat gcagccgccg cattgcatca gccatgatgg 1980
   atactttctc ggcaggagca aggtgagatg acaggagatc ctgccccggc acttcgccca 2040
   atagcagcca gtcccttccc gcttcagtga caacgtcgag cacagctgcg caaggaacgc 2100
   ccgtcgtggc cagccacgat agccgcgctg cctcgtcctg cagttcattc agggcaccgg 2160
   acaggtcggt cttgacaaaa agaaccgggc gcccctgcgc tgacagccgg aacacggcgg 2220
   catcagagca gccgattgtc tgttgtgccc agtcatagcc gaatagcctc tccacccaag 2280
   cggccggaga acctgcgtgc aatccatctt gttcaatcat gcgaaacgat ccagatccgg 2340
   tgcagattat ttggattgag agtgaatatg agactctaat tggataccga ggggaattta 2400
   tggaacgtca gtggagcatt tttgacaaga aatatttgct agctgatagt gaccttaggc 2460
   gacttttgaa cgcgcaataa tggtttctga cgtatgtgct tagctcatta aactccagaa 2520
   acccgcggct gagtggctcc ttcaacgttg cggttctgtc agttccaaac gtaaaacggc 2580
   ttgtcccgcg tcatcggcgg gggtcataac gtgactccct taattctccg ctcatgatca 2640
   gattgtcgtt tcccgccttc agtttaaact atcagtgttt gacaggatcc tgcttggtaa 2700
   taattgtcat tagattgttt ttatgcatag atgcactcga aatcagccaa ttttagacaa 2760
   gtatcaaacg gatgttaatt cagtacatta aagacgtccg caatgtgtta ttaagttgtc 2820
   taagcgtcaa tttgtttaca ccacaatata tcctgccacc agccagccaa cagctccccg 2880
   accggcagct cggcacaaaa tcaccacgcg ttaccaccac gccggccggc cgcatggtgt 2940
   tgaccgtgtt cgccggcatt gccgagttcg agcgttccct aatcatcgac cgcacccgga 3000
   gcgggcgcga ggccgccaag gcccgaggcg tgaagtttgg cccccgccct accctcaccc 3060
   cggcacagat cgcgcacgcc cgcgagctga tcgaccagga aggccgcacc gtgaaagagg 3120
   cggctgcact gcttggcgtg catcgctcga ccctgtaccg cgcacttgag cgcagcgagg 3180
   aagtgacgcc caccgaggcc aggcggcgcg gtgccttccg tgaggacgca ttgaccgagg 3240
   ccgacgccct ggcggccgcc gagaatgaac gccaagagga acaagcatga aaccgcacca 3300
   ggacggccag gacgaaccgt ttttcattac cgaagagatc gaggcggaga tgatcgcggc 3360
   cgggtacgtg ttcgagccgc ccgcgcacgt ctcaaccgtg cggctgcatg aaatcctggc 3420
   cggtttgtct gatgccaagc tggcggcctg gccggccagc ttggccgctg aagaaaccga 3480
   gcgccgccgt ctaaaaaggt gatgtgtatt tgagtaaaac agcttgcgtc atgcggtcgc 3540
   tgcgtatatg atgcgatgag taaataaaca aatacgcaag gggaacgcat gaaggttatc 3600
   gctgtactta accagaaagg cgggtcaggc aagacgacca tcgcaaccca tctagcccgc 3660
   gccctgcaac tcgccggggc cgatgttctg ttagtcgatt ccgatcccca gggcagtgcc 3720
   cgcgattggg cggccgtgcg ggaagatcaa ccgctaaccg ttgtcggcat cgaccgcccg 3780
   acgattgacc gcgacgtgaa ggccatcggc cggcgcgact tcgtagtgat cgacggagcg 3840
   ccccaggcgg cggacttggc tgtgtccgcg atcaaggcag ccgacttcgt gctgattccg 3900
   gtgcagccaa gcccttacga catatgggcc accgccgacc tggtggagct ggttaagcag 3960
   cgcattgagg tcacggatgg aaggctacaa gcggcctttg tcgtgtcgcg ggcgatcaaa 4020
   ggcacgcgca tcggcggtga ggttgccgag gcgctggccg ggtacgagct gcccattctt 4080
   gagtcccgta tcacgcagcg cgtgagctac ccaggcactg ccgccgccgg cacaaccgtt 4140
   cttgaatcag aacccgaggg cgacgctgcc cgcgaggtcc aggcgctggc cgctgaaatt 4200
   aaatcaaaac tcatttgagt taatgaggta aagagaaaat gagcaaaagc acaaacacgc 4260
   taagtgccgg ccgtccgagc gcacgcagca gcaaggctgc aacgttggcc agcctggcag 4320
   acacgccagc catgaagcgg gtcaactttc agttgccggc ggaggatcac accaagctga 4380
   agatgtacgc ggtacgccaa ggcaagacca ttaccgagct gctatctgaa tacatcgcgc 4440
   agctaccaga gtaaatgagc aaatgaataa atgagtagat gaattttagc ggctaaagga 4500
   ggcggcatgg aaaatcaaga acaaccaggc accgacgccg tggaatgccc catgtgtgga 4560
   ggaacgggcg gttggccagg cgtaagcggc tgggttgtct gccggccctg caatggcact 4620
   ggaaccccca agcccgagga atcggcgtga gcggtcgcaa accatccggc ccggtacaaa 4680
   tcggcgcggc gctgggtgat gacctggtgg agaagttgaa ggccgcgcag gccgcccagc 4740
   ggcaacgcat cgaggcagaa gcacgccccg gtgaatcgtg gcaagcggcc gctgatcgaa 4800
   tccgcaaaga atcccggcaa ccgccggcag ccggtgcgcc gtcgattagg aagccgccca 4860
   agggcgacga gcaaccagat tttttcgttc cgatgctcta tgacgtgggc acccgcgata 4920
   gtcgcagcat catggacgtg gccgttttcc gtctgtcgaa gcgtgaccga cgagctggcg 4980
   aggtgatccg ctacgagctt ccagacgggc acgtagaggt ttccgcaggg ccggccggca 5040
   tggccagtgt gtgggattac gacctggtac tgatggcggt ttcccatcta accgaatcca 5100
   tgaaccgata ccgggaaggg aagggagaca agcccggccg cgtgttccgt ccacacgttg 5160
   cggacgtact caagttctgc cggcgagccg atggcggaaa gcagaaagac gacctggtag 5220
   aaacctgcat tcggttaaac accacgcacg ttgccatgca gcgtacgaag aaggccaaga 5280
   acggccgcct ggtgacggta tccgagggtg aagccttgat tagccgctac aagatcgtaa 5340
   agagcgaaac cgggcggccg gagtacatcg agatcgagct agctgattgg atgtaccgcg 5400
   agatcacaga aggcaagaac ccggacgtgc tgacggttca ccccgattac tttttgatcg 5460
   atcccggcat cggccgtttt ctctaccgcc tggcacgccg cgccgcaggc aaggcagaag 5520
   ccagatggtt gttcaagacg atctacgaac gcagtggcag cgccggagag ttcaagaagt 5580
   tctgtttcac cgtgcgcaag ctgatcgggt caaatgacct gccggagtac gatttgaagg 5640
   aggaggcggg gcaggctggc ccgatcctag tcatgcgcta ccgcaacctg atcgagggcg 5700
   aagcatccgc cggttcctaa tgtacggagc agatgctagg gcaaattgcc ctagcagggg 5760
   aaaaaggtcg aaaaggtctc tttcctgtgg atagcacgta cattgggaac ccaaagccgt 5820
   acattgggaa ccggaacccg tacattggga acccaaagcc gtacattggg aaccggtcac 5880
   acatgtaagt gactgatata aaagagaaaa aaggcgattt ttccgcctaa aactctttaa 5940
   aacttattaa aactcttaaa acccgcctgg cctgtgcata actgtctggc cagcgcacag 6000
   ccgaagagct gcaaaaagcg cctacccttc ggtcgctgcg ctccctacgc cccgccgctt 6060
   cgcgtcggcc tatcgcggcc gctggccgct caaaaatggc tggcctacgg ccaggcaatc 6120
   taccagggcg cggacaagcc gcgccgtcgc cactcgaccg ccggcgccca catcaaggca 6180
   ccctgcctcg cgcgtttcgg tgatgacggt gaaaacctct gacacatgca gctcccggag 6240
   acggtcacag cttgtctgta agcggatgcc gggagcagac aagcccgtca gggcgcgtca 6300
   gcgggtgttg gcgggtgtcg gggcgcagcc atgacccagt cacgtagcga tagcggagtg 6360
   tatactggct taactatgcg gcatcagagc agattgtact gagagtgcac catatgcggt 6420
   gtgaaatacc gcacagatgc gtaaggagaa aataccgcat caggcgctct tccgcttcct 6480
   cgctcactga ctcgctgcgc tcggtcgttc ggctgcggcg agcggtatca gctcactcaa 6540
   aggcggtaat acggttatcc acagaatcag gggataacgc aggaaagaac atgtgagcaa 6600
   aaggccagca aaaggccagg aaccgtaaaa aggccgcgtt gctggcgttt ttccataggc 6660
   tccgcccccc tgacgagcat cacaaaaatc gacgctcaag tcagaggtgg cgaaacccga 6720
   caggactata aagataccag gcgtttcccc ctggaagctc cctcgtgcgc tctcctgttc 6780
   cgaccctgcc gcttaccgga tacctgtccg cctttctccc ttcgggaagc gtggcgcttt 6840
   ctcatagctc acgctgtagg tatctcagtt cggtgtaggt cgttcgctcc aagctgggct 6900
   gtgtgcacga accccccgtt cagcccgacc gctgcgcctt atccggtaac tatcgtcttg 6960
   agtccaaccc ggtaagacac gacttatcgc cactggcagc agccactggt aacaggatta 7020
   gcagagcgag gtatgtaggc ggtgctacag agttcttgaa gtggtggcct aactacggct 7080
   acactagaag gacagtattt ggtatctgcg ctctgctgaa gccagttacc ttcggaaaaa 7140
   gagttggtag ctcttgatcc ggcaaacaaa ccaccgctgg tagcggtggt ttttttgttt 7200
   gcaagcagca gattacgcgc agaaaaaaag gatctcaaga agatcctttg atcttttcta 7260
   cggggtctga cgctcagtgg aacgaaaact cacgttaagg gattttggtc atgcatgata 7320
   tatctcccaa tttgtgtagg gcttattatg cacgcttaaa aataataaaa gcagacttga 7380
   cctgatagtt tggctgtgag caattatgtg cttagtgcat ctaacgcttg agttaagccg 7440
   cgccgcgaag cggcgtcggc ttgaacgaat ttctagctag acattatttg ccgactacct 7500
   tggtgatctc gcctttcacg tagtggacaa attcttccaa ctgatctgcg cgcgaggcca 7560
   agcgatcttc ttcttgtcca agataagcct gtctagcttc aagtatgacg ggctgatact 7620
   gggccggcag gcgctccatt gcccagtcgg cagcgacatc cttcggcgcg attttgccgg 7680
   ttactgcgct gtaccaaatg cgggacaacg taagcactac atttcgctca tcgccagccc 7740
   agtcgggcgg cgagttccat agcgttaagg tttcatttag cgcctcaaat agatcctgtt 7800
   caggaaccgg atcaaagagt tcctccgccg ctggacctac caaggcaacg ctatgttctc 7860
   ttgcttttgt cagcaagata gccagatcaa tgtcgatcgt ggctggctcg aagatacctg 7920
   caagaatgtc attgcgctgc cattctccaa attgcagttc gcgcttagct ggataacgcc 7980
   acggaatgat gtcgtcgtgc acaacaatgg tgacttctac agcgcggaga atctcgctct 8040
   ctccagggga agccgaagtt tccaaaaggt cgttgatcaa agctcgccgc gttgtttcat 8100
   caagccttac ggtcaccgta accagcaaat caatatcact gtgtggcttc aggccgccat 8160
   ccactgcgga gccgtacaaa tgtacggcca gcaacgtcgg ttcgagatgg cgctcgatga 8220
   cgccaactac ctctgatagt tgagtcgata cttcggcgat caccgcttcc cccatgatgt 8280
   ttaactttgt tttagggcga ctgccctgct gcgtaacatc gttgctgctc cataacatca 8340
   aacatcgacc cacggcgtaa cgcgcttgct gcttggatgc ccgaggcata gactgtaccc 8400
   caaaaaaaca gtcataacaa gccatgaaaa ccgccactgc g 8441
<210> 26
   <211> 45
   <212> DNA
   <213> Künstliche Sequenz
<220>
   <223> Beschreibung der künstlichen Sequenz: oligonucleotid primer
<400> 26
   aagtcgacgg atcctgatag cttatactca aattcaacaa gttat 45
<210> 27
   <211> 1318
   <212> DNA
   <213> Arabidopsis thaliana
<220>
   <221> promoter
   <222> (1)..(1318)
   <223> TPT truncated promoter
<400> 27
   tgatagctta tactcaaatt caacaagtta tatataaatg tatagatact acaatatcat 60
   taacaaaagt caccttaaat aaatacacat atcttttatg ttctctattg ttttgcgtac 120
   gctaacacaa tttctcatat gcaaaaggat gaatgagtaa caaattacct cataagaaca 180
   atcatctttg cttacatact aatacaataa tcactcaatc aaccaataac atcaatcaca 240
   taggtttaca tacaataatc actcaatcaa cttcataaga agaatcatgt ttacttaatt 300
   catcaattat ccccaaaaac accactatta agtataaact ataacatatt tgtagtgatg 360
   ggtcaacatt tttatcatat ttaaactcgg gttccctcaa atcgagaaat agtgaacatg 420
   taatattaat tttaaatcgc aattacagaa attaattgaa tttggtcaaa tggacagaat 480
   tttatagatt gggtggaact agaaaaaaaa aaaaaaagag tatagggtga attgagtaca 540
   tgaaagtaca tggtaatcct agttaaacgc ataatacatg tgggttcatt tgtatttttt 600
   tgtaacttac gagtaaactg gctacaacaa aaaaaattag aagatttttt tgttttgtag 660
   aaaaccctaa ttttagttat agttgtataa ctttgataaa attataaaat tgtattacga 720
   aaaaagtaat aagatattca aaaaagccta gaataacgta tatgactatg agcatgaaac 780
   tgcaagtcaa atgctgacag acaaccataa acaaaagaaa ttaaatagag atacctttaa 840
   aataagtaaa atttcattta taaaaaatct actttcttgt gaatctgtca cgttcaataa 900
   tttgaagacc actcaacata caaggtaaat aatgaaaaat aaaatctacc aaaatttcaa 960
   tcattattat cttccaaaaa aacaaaatta tacagatgat gatggtgata tggaacttcg 1020
   attggctaat attcactgtg tctctaaaaa ccatccactt atcaagataa gatggaccct 1080
   acactcatcc aatctaaacc agtatctcaa gattcttatc taattacatc attctctacc 1140
   gttagatgaa attgaccatt aaccctacca taactccata caccgcgaga tactggatta 1200
   accaaatcga gatcatcgta gccgtccgat caacaagtac catctcttga aatactcgaa 1260
   atcctcataa gtccgtccct ctttgctctc actatcaaaa ctctgaattt cgatttca   1318
<210> 28
   <211> 234
   <212> DNA
   <213> Solanum tuberosum
<220>
   <221> terminator
   <222> (1)..(234)
   <223> terminators sequence of the Cathepsin D Inhibitor gene from potato
<400> 28
   cctgcagata gactatacta tgttttagcc tgcctgctgg ctagctacta tgttatgtta 60
   tgttgtaaaa taaacacctg ctaaggtata tctatctata ttttagcatg gctttctcaa 120
   taaattgtct ttccttatcg tttactatct tatacctaat aatgaaataa taatatcaca 180
   tatgaggaac ggggcaggtt taggcatata tatacgagtg tagggcggag tggg       234
<210> 29
   <211> 298
   <212> DNA
   <213> Vicia faba
<220>
   <221> terminator
   <222> (1) .. (298)
   <223> terminator of storage protein gene VflE1B3 from Vicia faba
<400> 29
   gatcctgcaa tagaatgttg aggtgaccac tttctgtaat aaaataatta taaaataaat 60
   ttagaattgc tgtagtcaag aacatcagtt ctaaaatatt aataaagtta tggccttttg 120
   acatatgtgt ttcgataaaa aaatcaaaat aaattgagat ttattcgaaa tacaatgaaa 180
   gtttgcagat atgagatatg tttctacaaa ataataactt aaaactcaac tatatgctaa 240
   tgtttttctt ggtgtgtttc atagaaaatt gtatccgttt cttagaaaat gctcgtaa   298